(19) Europäisches Patentamt
European Patent Office
Office européen des brevets

(11) EP 0 655 065 B1

(12) **EUROPEAN PATENT SPECIFICATION**

(45) Date of publication and mention of the grant of the patent:
**11.12.1996 Bulletin 1996/50**

(21) Application number: **93919994.9**

(22) Date of filing: **12.08.1993**

(51) Int Cl.[6]: **C07D 498/18**, C07F 7/18, A61K 31/435 // (C07D498/18, 311:00, 273:00, 221:00)

(86) International application number:
**PCT/US93/07581**

(87) International publication number:
**WO 94/04540 (03.03.1994 Gazette 1994/06)**

(54) **27-HYDROXYRAPAMYCIN AND DERIVATIVES THEREOF**

27 HYDROXYRAPAMYCIN UND DERIVATE DAVON

27-HYDROXYRAPAMYCINE ET SES DERIVES

(84) Designated Contracting States:
**AT BE CH DE DK ES FR GB GR IE IT LI LU NL PT SE**

(30) Priority: **13.08.1992 US 930124**
**27.01.1993 US 9605**

(43) Date of publication of application:
**31.05.1995 Bulletin 1995/22**

(73) Proprietor: **AMERICAN HOME PRODUCTS CORPORATION**
**Madison, New Jersey 07940-0874 (US)**

(72) Inventor: **NELSON, Frances, Christy**
**Yardley, PA 19067 (US)**

(74) Representative: **Wileman, David Francis et al**
**c/o John Wyeth and Brother Limited**
**Huntercombe Lane South**
**Taplow Maidenhead Berkshire SL6 OPH (GB)**

(56) References cited:
**EP-A- 0 467 606** **US-A- 5 102 876**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### BACKGROUND OF THE INVENTION

This invention relates to a compound of formula I, which is named 27-hydroxyrapamycin, and derivatives thereof and a method for using them for inducing immunosuppression, and in the treatment of transplantation rejection, host vs. graft disease, autoimmune diseases, diseases of inflammation, solid tumors, fungal infections, and hyperproliferative vascular disorders.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R. Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [Fifth Int. Conf. Inflamm. Res. Assoc. 121 (Abstract), (1990)], and smooth muscle cell proliferation and intimal thickening following vascular injury [Morris, R. J. Heart Lung Transplant 11 (pt. 2): 197 (1992)].

Mono- and diacylated derivatives of rapamycin (esterified at the 28 and 43 positions) have been shown to be useful as antifungal agents (U.S. Patent 4,316,885) and used to make water soluble prodrugs of rapamycin (U.S. Patent 4,650,803). Recently, the numbering convention for rapamycin has been changed; therefore according to Chemical Abstracts nomenclature, the esters described above would be at the 31- and 42- positions. Under the older numbering convention, 27-hydroxyrapamycin would be named as 24-hydroxyrapamycin.

U.S. Patent 5,102,876 discloses 15-hydroxyrapamycin and 15,27-bis-hydroxyrapamycin, which were prepared by the reduction of rapamycin with diisobutylaluminum hydride, and a method of using them as immunosuppressive, antiinflammatory, and antifungal agents. 27-hydroxyrapamycin cannot be produced via the synthetic methodology disclosed in U.S. Patent 5,102,876.

U.S. Patents 5,138,051 and 5,169,851 disclose 33-hydroxyrapamycin which were prepared by the reduction of rapamycin using sodium triacetoxyborohydride, and a method of using them as immunosuppressive, antiinflammatory, and antifungal agents. 27-hydroxyrapamycin cannot be produced via the synthetic methodology disclosed in U.S. Patents 5,138,051 and 5,169,851.

### DESCRIPTION OF THE INVENTION

This invention provides a compound of formula I,

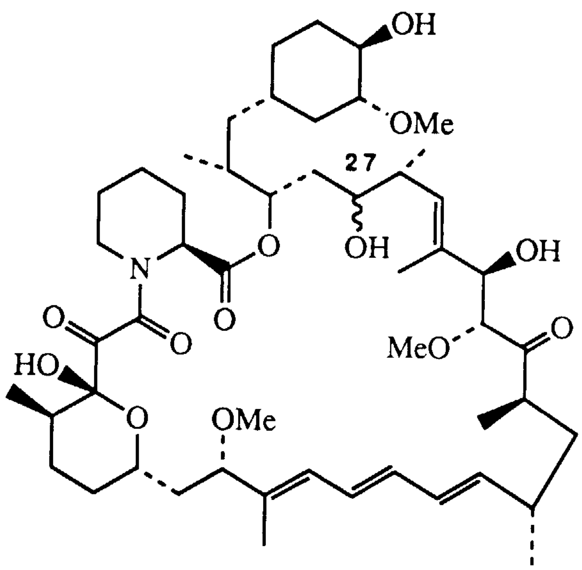

which is useful as an immunosuppressive, antiinflammatory, antifungal, antitumor, and antiproliferative agent. The compound depicted by formula I is named 27-hydroxyrapamycin, and may also be referred to as 27-deoxo-27-hydroxyrapamycin. 27-Hydroxyrapamycin may be administered orally, parenterally, intranasally, intrabronchially, transdermally, or rectally when administered in accordance with this disclosure.

This invention also provides derivatives of 27-hydroxyrapamycin which are useful as immunosuppressive, antiinflammatory, antifungal, antitumor, and antiproliferative agents having the formula II:

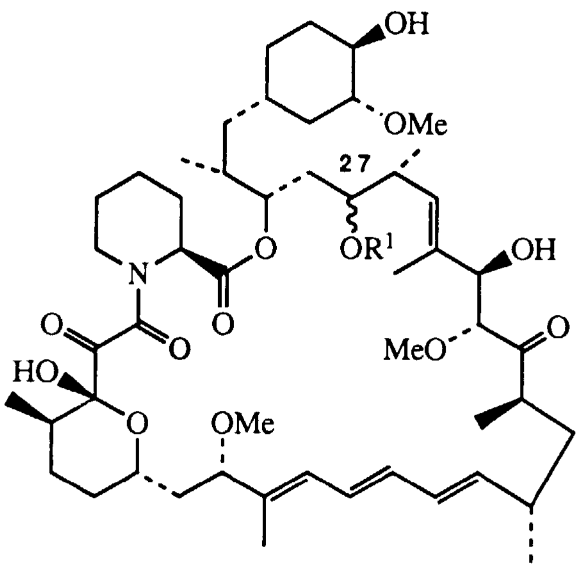

II

wherein

$R^1$ is

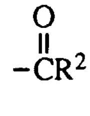

and

$R^2$ is alkyl of 1-10 carbon atoms, arylalkyl of 7-10 carbon atoms, or aryl wherein the aryl group may be optionally mono-, di-, or tri- substituted with a group selected from alkyl of 1-6 carbon atoms, arylalkyl of 7-10 carbon

atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof;
or having the formula III:

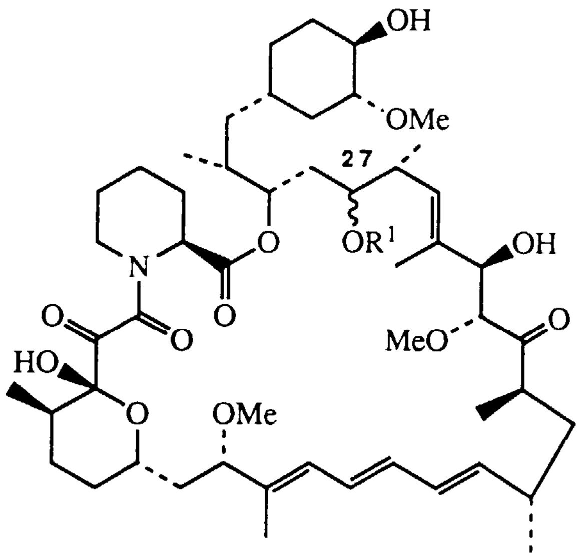

III

wherein

$R^1$ is

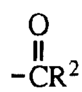

and
$R^2$ is a mono-, di-, poly-, or per-fluorinated alkyl group of 1-10 carbon atoms;

or having the formula IV:

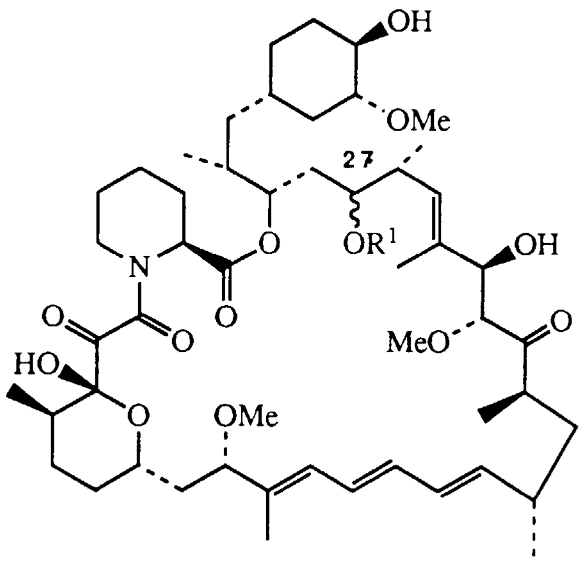

IV

wherein

R[1] is

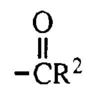

;

R[2] is

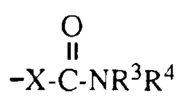

;

X is $-(CH_2)_m-$ or $-Ar^1-$ ; where $-AR^1-$ is an optionally mono- or di-substituted group selected from:

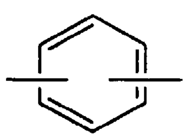

,

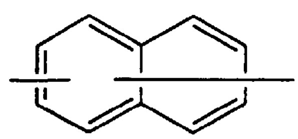

,

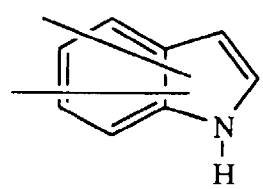

,

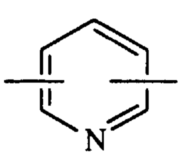

,

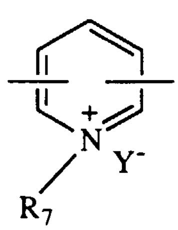

,

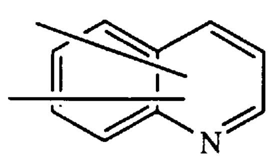

or

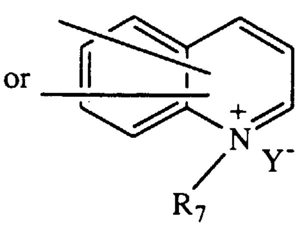

R[3] and R[4] are each, independently, hydrogen, alkyl of 1-12 carbon atoms, $-(CH_2)_n-Ar$, $-(CH_2)_p-NR^5R^6$, or $-(CH_2)_p-N^+R^5R^6R^7Y^-$;

R[5] and R[6] are each, independently, hydrogen, alkyl of 1-12 carbon atoms, or $-(CH_2)_n-Ar$;

Ar is an optionally mono- or di- substituted group selected from

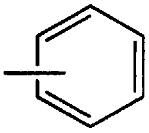 , 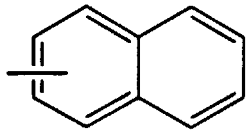 , 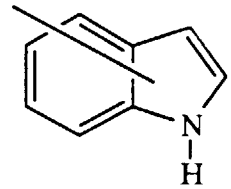 ,

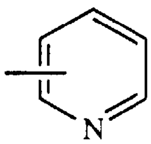 , 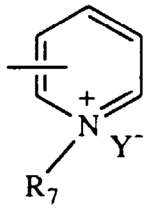 , 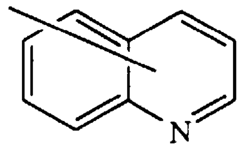 or 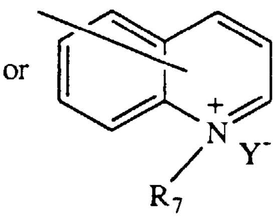

in which the optional substituents are selected from the group consisting of alkyl of 1-6 carbon atoms, arylalkyl of 7-10 carbon atoms, alkoxy of 1-6 carbon atoms, cyano, halo, nitro, carbalkoxy of 2-7 carbon atoms, or perfluoroalkyl of 1-6 carbon atoms;

$R^7$ is alkyl of 1-6 carbon atoms;

Y is a halide, sulfate, phosphate, or p-toluenesulfonate anion;

m = 1-6;
n = 1-6;
p = 1-6;
or a pharmaceutically acceptable salt thereof;
or having the formula V:

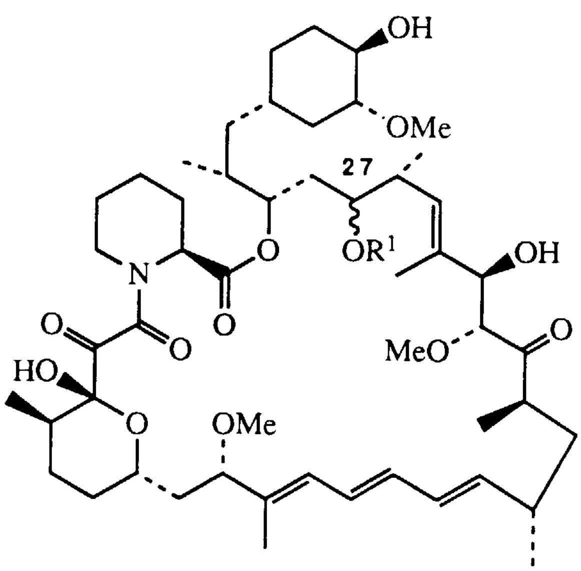

V

wherein

$R^1$ is

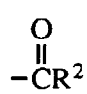 ;

$R^2$ is $-NH(CR^3R^4)_n-X$ ;

$R^3$ and $R^4$ are each, independently, hydrogen, alkyl of 1-6 carbon atoms, arylalkyl of 7-10 carbon atoms, cycloalkyl

of 3-8 carbon atoms, halogen, or trifluoromethyl;

| | |
|---|---|
| X is | hydrogen, lower alkyl of 1-6 carbon atoms, cycloalkyl of 3-8 carbon atoms, trifluoromethyl, nitro, alkoxy of 1-6 carbon atoms, carboalkoxy of 2-7 carbon atoms, arylalkyl of 7-10 carbon atoms, halo, dialkylamino of 1-6 carbon atoms per alkyl group, thioalkyl of 1-6 carbon atoms, or Y; |
| Y is | a phenyl group which may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, dialkylamino of 1-6 carbon atoms per alkyl group, or alkylthio of 1-6 carbon atoms, $-SO_3H$, $-PO_3H$, and $-CO_2H$; |

n = 0-5;
with the proviso that when n = 0, X is lower alkyl of 1-6 carbon atoms, cycloalkyl of 3-8 carbon atoms, arylalkyl of 7-10 carbon atoms, or Y;
or a pharmaceutically acceptable salt thereof:
or having the formula VI:

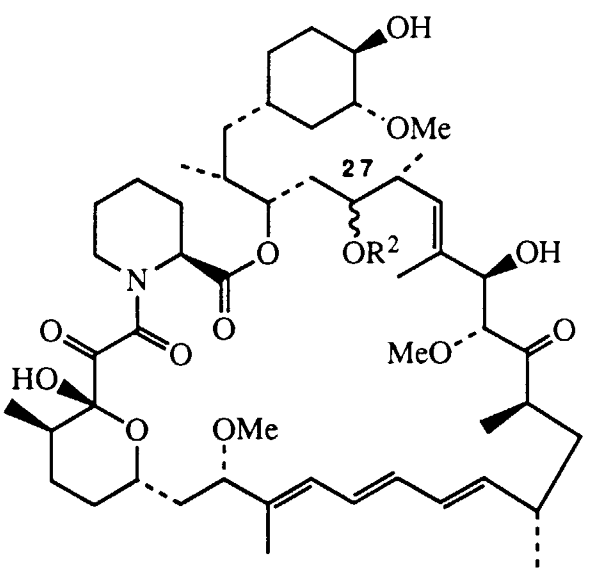

VI

wherein

$R^2$ is

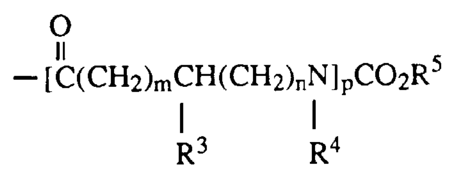
;

| | |
|---|---|
| $R^3$ is | hydrogen, alkyl of 1-6 carbon atoms, arylalkyl of 7-10 carbon atoms, $-(CH_2)_qCO_2R^6$, $-(CH_2)_rNR^7CO_2R^8$, carbamylalkyl of 2-3 carbon atoms, aminoalkyl of 1-4 carbon atoms, hydroxyalkyl of 1-4 carbon atoms, guanylalkyl of 2-4 carbon atoms, mercaptoalkyl of 1-4 carbon atoms, alkylthioalkyl of 2-6 carbon atoms, indolylmethyl, hydroxyphenylmethyl, imidazoylmethyl or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| $R^4$ and $R^7$ are | each, independently, hydrogen, alkyl of 1-6 carbon atoms, or arylalkyl of 7-10 carbon atoms; |
| $R^5$, $R^6$, and $R^8$ are | each, independently, alkyl of 1-6 carbon atoms, arylalkyl of 7-10 carbon atoms, fluorenylmethyl, or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| m is | 0-4; |
| n is | 0-4; |

p is 1-2;
q is 0-4;
r is 0-4;

wherein $R^3$, $R^4$, m, and n are independent in each of the

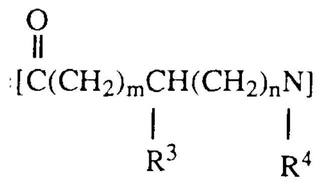

subunits when p = 2;
or a pharmaceutically acceptable salt thereof;
or having the formula VII:

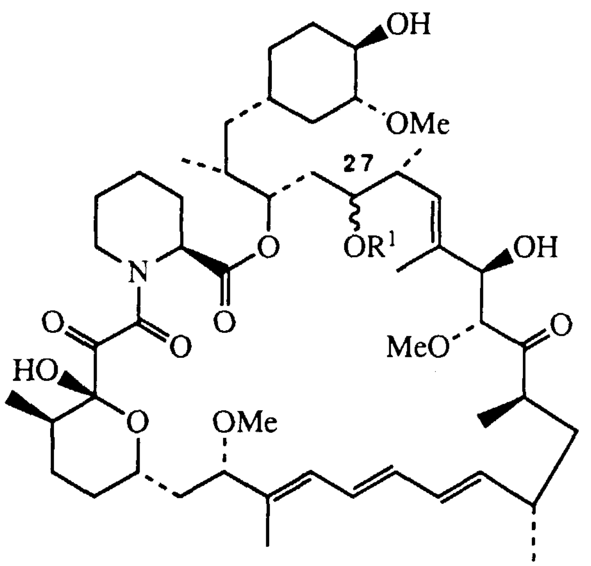

VII

wherein

$R^1$ is alkyl of 1-6 carbon atoms, arylalkyl of 7-10 carbon atoms, $-CH_2YX$, $-C(CH_3)_2YX$, $-CH(CH_3)YX$, or L ;
Y is O or S;
X is $-CH_3$, $-(CH_2)_nCH_3$ , $-CH_2Ar$ , $-(CH_2)_2OCH_3$, $-CH_2CCl_3$ , $-CH(CH_3)_2$, or $-CH_2CH_2SiMe_3$;
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri-substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;
L is tetrahydrofuran-2-yl, tetrahydrothiophen-2-yl, tetrahydrothiopyran-2-yl, tetrahydropyran-2-yl, 4-methoxytetrahydropyran-2-yl, 4-methoxytetrahydrothiopyran-2-yl, or 4-methoxytetrahydrothiopyran-2-yl S,S dioxide: and

n = 1-5;
or having the formula VIII:

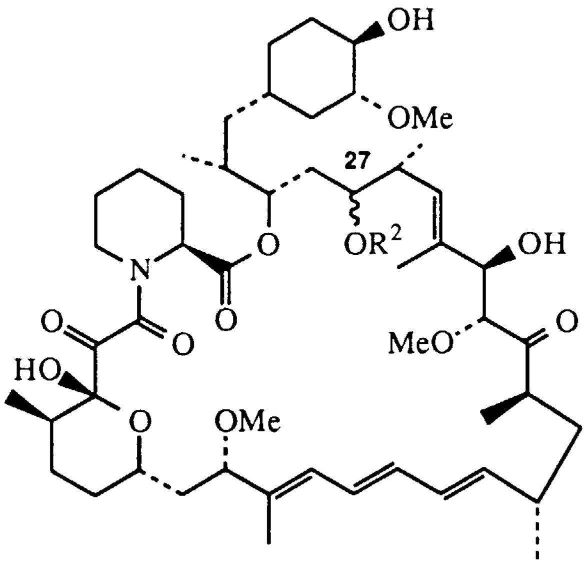

VIII

wherein

$R^2$ is

$$-\overset{O}{\overset{\|}{C}}(CH_2)_mNRR^1\ ;$$

R and $R^1$ are each hydrogen or alkyl of 1-3 carbon atoms or R and $R^1$ together with the nitrogen to which they are attached form a saturated heterocyclic ring having 4-5 carbon atoms; and

m = 1-3; or a pharmaceutically acceptable salt thereof;
or having the formula IX:

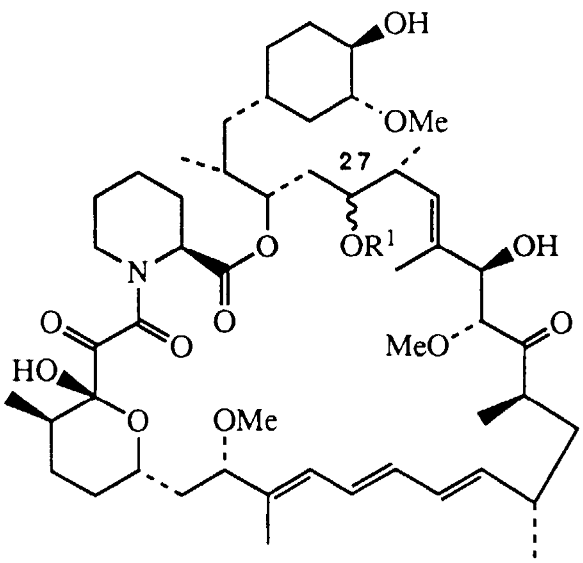

IX

wherein

$R^1$ is -$CONHSO_2$-Ar; and
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri-

substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof when the Ar group contains a basic nitrogen or when the Ar group is substituted by dialklyamino of 1-6 carbon atoms per alkyl group, $-SO_3H$, $-PO_3H$, or $-CO_2H$;
or having formula X:

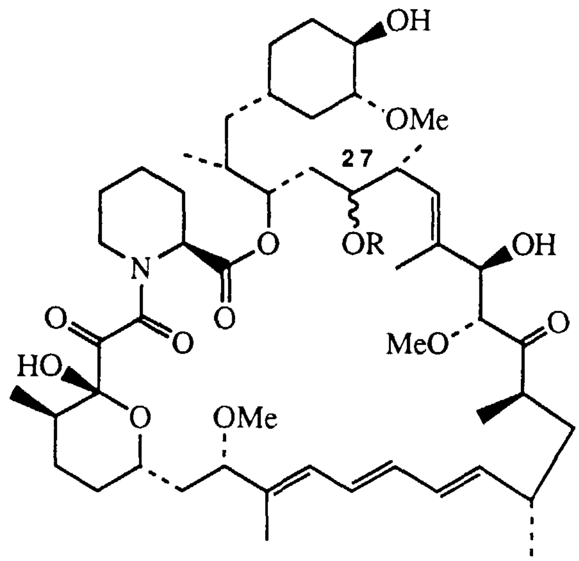

X

wherein

R is $-SO_2R^1$;
$R^1$ is alkyl, alkenyl, alkynyl containing 1 to 6 carbon atoms; or an aromatic moiety selected from the group consisting of phenyl and naphthyl or a heterocyclic moiety selected from the group consisting of thiophenyl and quinolinyl; or $-NHCOR^2$; and
$R^2$ is lower alkyl containing 1 to 6 carbon atoms;

or a pharmaceutically acceptable salt thereof.

Pharmaceutically acceptable salts may be formed from the compounds of formulas II, IV - VI, and VII - X from organic and inorganic acids and inorganic cations. Preferred organic and inorganic acids are those such as acetic, lactic, citric, tartaric, succinic, maleic, malonic, gluconic, hydrochloric, hydrobromic, phosphoric, nitric, sulfuric, methanesulfonic, and the like. Preferred inorganic cations are those such as sodium, potassium, and the like. Based on this disclosure, other pharmaceutically acceptable salts that can be formed will be readily apparent to one skilled in the art.

When any of the compounds of formulas II - X contain an aryl or arylalkyl moiety, it is preferred that the aryl portion is a phenyl, naphthyl, pyridyl, quinolyl, isoquinolyl, quinoxalyl, thienyl, thioraphthyl, furyl, benzofuryl, benzodioxyl, benzoxazolyl, benzoisoxazolyl, or benzodioxolyl group that may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$. It is more preferred that the aryl moiety is a phenyl group that is optionally mono-, di-, or tri-substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$.

For the compounds of formula IV, preferred members are those in which X is $-(CH_2)_m-$ and $R^3$ and $R^4$ are alkyl of 1-6 carbon atoms; and those in which X is $-(CH_2)_m-$, $R^3$ is hydrogen, and $R^4$ is $-(CH_2)_n-Ar$.

For the compounds of formula V, preferred members are those in which n is 0 and X is Y.

For the compounds of formula VI, preferred members are those in which m = 0, n = 0, and p = 1; m = 0, n = 0, and p = 2; n = 0, and $R^3$ is $-(CH_2)_qCO_2R^6$; m = 0, n = 0, and $R^3$ is $-(CH_2)_rNR^7CO_2R^8$; and m = 0, n = 0, and $R^3$ is hydrogen.

For the compounds of formula VII, preferred members include those in which Y is O and those in which $R^1$ is alkyl of 1-6 carbon atoms.

The compounds of formulas II - X may be administered orally, parenterally, intranasally, intrabronchially, transdermally, or rectally when administered in accordance with this disclosure.

This invention also provides pharmaceutical compositions comprising an effective amount of 27-hydroxyrapamycin or any of the compounds of formulas II - X, and a pharmaceutical carrier.

It is contemplated that when the compounds of this invention are used as an immunosuppressive or antiinflammatory agent, they can be administered in conjunction with one or more other immunoregulatory agents. Such other antirejection chemotherapeutic agents include, but are not limited to azathioprine, corticosteroids, such as prednisone and methylprednisolone, cyclophosphamide, rapamycin, cyclosporin A, FK-506, OKT-3, and ATG. By combining 27-hydroxyrapamycin or a derivative thereof with such other drugs or agents for inducing immunosuppression or treating inflammatory conditions, the lesser amounts of each of the agents are required to achieve the desired effect. The basis for such combination therapy was established by Stepkowski whose results showed that the use of a combination of rapamycin and cyclosporin A at subtherapeutic doses significantly prolonged heart allograft survival time. [Transplantation Proc. 23: 507 (1991)].

The preparation of 27-hydroxyrapamycin can be accomplished by the sequence of reactions shown below, beginning with rapamycin.

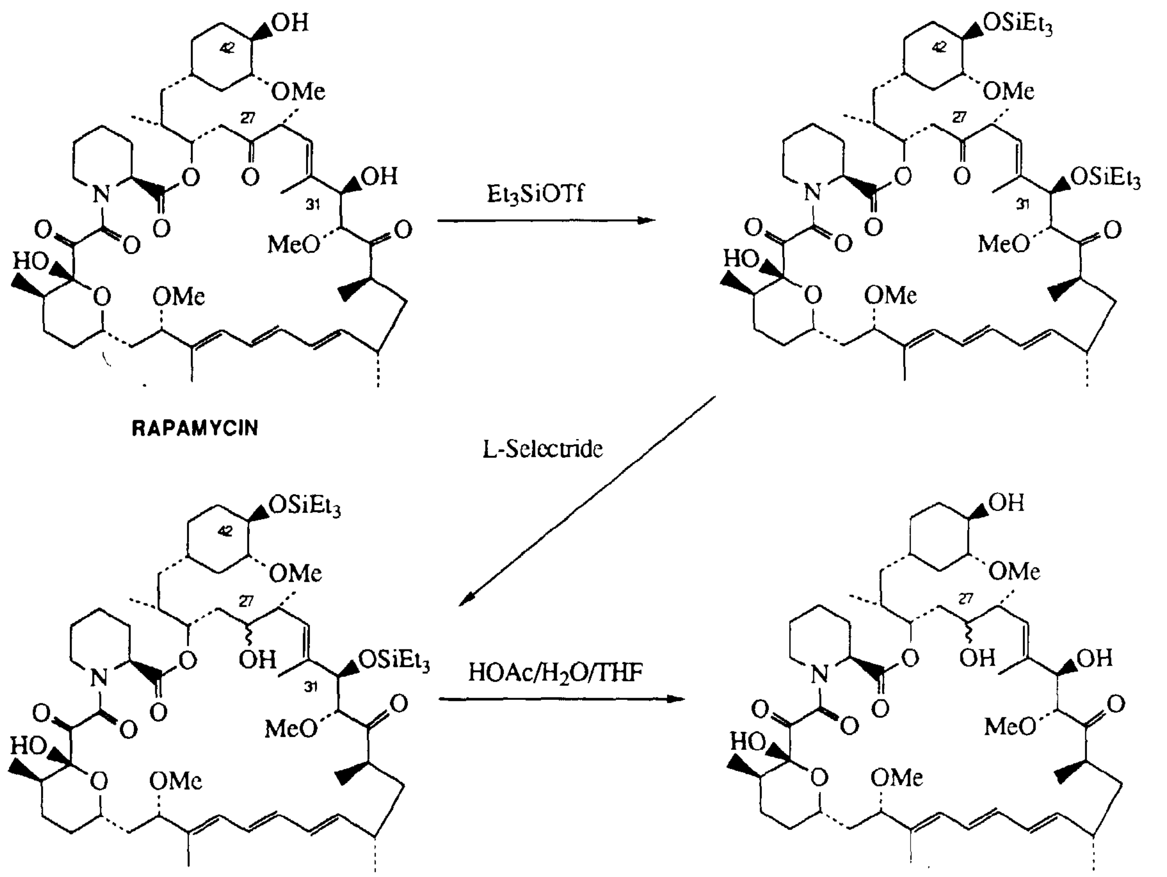


RAPAMYCIN

I

The 31- and 42- hydroxyl groups of rapamycin are first protected with a suitable protecting group, such as the triethylsilyl ether protecting group. Protection of the hydroxyl groups prior to reduction appears to be necessary to prevent overreduction and ring degradation. Reduction of the 27-ketone was selectively accomplished with L-Selectride (lithium tri-sec-butylborohydride) to provide a compound which was named 31,42-bis-triethylsilyl ether of 27-hydroxyrapamycin. Attempted reduction with DIBAL, as disclosed in U.S. Patent 5,102,876, failed to provide any products in which the 27-ketone was the only keto-group that was reduced. Removal of the silyl ether protecting groups was accomplished under mildly acidic conditions, such as with a mixture of acetic acid, water, and THF. Removal of the silyl ether protecting groups can also be accomplished using fluoride ion generating reagents, such as hydrogen fluoride/pyridine. It is also contemplated that the 31- and 42-hydroxyl groups can be protected with other silylating reagants, such as triisopropylsilyl chloride or t-butyldimethylsilyl chloride, to allow selective reduction of the 27-ketone of

rapamycin.

The derivatives of 27-hydroxyrapamycin that are claimed as part of this invention can be prepared by reacting the intermediate 31,42-bis-triethylsilyl ether of 27-hydroxyrapamycin with suitable electrophilic agents, e.g. by

(1) acylating the intermediate using

(a) an acid of formula $HOC(O)R^2$ or a reactive derivative thereof where $R^2$ is as defined in connection with formulae II, III or IV; or

(b) an acid of formula $HOR^2$ where $R^2$ is as defined in connection with formula VI or VIII, said reactive derivatives including acyl halides, e.g., the chloride, bromide or iodide or acid anhydrides including mixed anhydrides, the reaction being carried out in the presence of a suitable coupling agent when the acid of formula $HOC(O)R^2$ is used; or

(2) carbamylating the intermediate with an isocyanate of formula O=C=N-Q where -Q is $(CR^3R^4)_nX$ as defined in connection with formula V or $-SO_2$-Ar as defined in connection with formula IX or

(3) acetalising or etherifying the intermediate using a compound of formula hal-$R^1$ or $CH_2$=CHYX where $R^1$, Y and X are as defined in connection with formula VII and hal is a halogen, e.g., chlorine or bromine or

(4) sulfonating the intermediate using a sulfonating agent of formula

$$R^1SO_2hal$$

$$(R^1SO_2)_2O$$

$$R^2OCONSO_2N(Alkyl)_3$$

in which formula $R^1$ and $R^2$ are as defined in connection with formula X, hal is a halogen such as chlorine or bromine, alkyl is an alkyl group, e.g., alkyl of 1-6 carbon atoms such as methyl or ethyl, followed by deprotection to remove protecting groups from the 31 and 42 positions.

The 27-acyl derivatives of formula II can be prepared by the method used in Examples 4 and 5. The 27-acyl derivative of formula II can also be prepared by reacting the 27-hydroxyl group of 31,42-bis-triethylsilyl ether of 27-hydroxyrapamycin with an acylating agent according the method described in U.S. Patent 4,316,885 and EP-A-46661, the disclosures of which are hereby incorporated by reference, followed by deprotection according to Examples 3 or 5.

The 27-fluorinated esters of formula III can be prepared by reacting the 27-hydroxyl group of 31,42-bis-triethylsilyl ether of 27-hydroxyrapamycin with a suitable fluorinated acylating agent as described in U.S. Patent 5,100,883, the disclosure of which is hereby incorporated by reference, followed by deprotection according to Examples 3 or 5.

The 27-amide esters of formula IV can be prepared by acylating the 27-hydroxyl group of 31,42-bis-triethylsilyl ether of 27-hydroxyrapamycin with a suitable acylating agent as described in U.S. Patent 5,118,677and EP-A-515140, the disclosures of which are hereby incorporated by reference, followed by deprotection according to Examples 3 or 5.

The 27-carbamates of formula V can be prepared by carbamylating the 27-hydroxyl group of 31,42-bis-triethylsilyl ether of 27-hydroxyrapamycin with a suitable carbamylating agent as described in U.S. Patent 5,118,678 and EP-A-509795, the disclosures of which are hereby incorporated by reference, followed by deprotection according to Examples 3 or 5.

The 27-aminoesters of formula VI can be prepared by acylating the 27-hydroxyl group of 31,42-bis-triethylsilyl ether of 27-hydroxyrapamycin with a suitable acylating agent as described in U.S. Patent 5,130,307 and WO 92/05179, the disclosures of which are hereby incorporated by reference, followed by deprotection according to Examples 3 or 5.

The 27-ethers of formula VII can be prepared by reacting the 27-hydroxyl group of 31,42-bis-triethylsilyl ether of 27-hydroxyrapamycin with a suitable acetal forming reagent as described in U.S. Patent 5,151,413, the disclosure of which is hereby incorporated by reference, followed by deprotection using hydrogen fluoride/ pyridine according to standard literature procedures. The alkyl or arylalkyl ethers of formula VII can be formed by alkylating the 27-hydroxyl group of 31,42-bis-triethylsilyl ether of 27-hydroxyrapamycin with a suitable alkylating agent, such as with an alkyl halide in pyridine followed by deprotection.

The 27-aminoacyl compounds of formula VIII can be prepared by acylating the 27-hydroxyl group of 31,42-bis-triethylsilyl ether of 27-hydroxyrapamycin with a suitable acylating agent as described in U.S. Patent 4,650,803 or EP227355, the disclosures of which are hereby incorporated by reference, followed by deprotection according to Examples 3 or 5.

The 27-sulfonylcarbamates of formula IX can be prepared by carbamylating the 27-hydroxyl group of 31,42-bis-triethylsilyl ether of 27-hydroxyrapamycin with a suitable carbamylating agent as described in U.S. Patent Application Serial Number 07/837,048, filed February 18, 1992, and EP-A-509795, the disclosures of which are incorporated by reference, followed by deprotection according to Examples 3 or 5.

The 27-sulfonates and sulfamates of formula X can be prepared by reacting the 27-hydroxyl group of 31,42-bis-triethylsilyl ether of 27-hydroxyrapamycin with a suitable sulfonyl halide or (carboxysulfamoyl)triethylammonium hydroxide inner salt as described in U.S. Patent 5,177,203, the disclosure of which is hereby incorporated by reference, followed by deprotection according to Examples 3 or 5.

Based on this disclosure, other derivatives of 27-hydroxyrapamycin will be apparent to one skilled in the art. For example, it is contemplated that other esters of the 27-hydroxyl group can be prepared. These include both organic esters and inorganic esters, such as phosphate, nitrate, sulfinate, sulfonate esters, and the like, and organic esters of these inorganic acids. These compounds are also expected to have a similar activity profile to the compounds of this invention. Additionally, the 27-hydroxyl group may be protected with suitable protecting groups, such as a silyl ether, to provide a 27,31,42-tris-silyl ether of 27-hydroxyrapamycin.

Immunosuppressive activity for representative compounds of this invention was evaluated in an in vitro standard pharmacological test procedure to measure lymphocyte proliferation (LAF) and in an in vivo standard pharmacological test procedure which evaluated the survival time of a pinch skin graft.

The comitogen-induced thymocyte proliferation procedure (LAF) was used as an in vitro measure of the immunosuppressive effects of representative compounds. Briefly, cells from the thymus of normal BALB/c mice are cultured for 72 hours with PHA and IL-1 and pulsed with tritiated thymidine during the last six hours. Cells are cultured with and without various concentrations of rapamycin, cyclosporin A, or test compound. Cells are harvested and incorporated radioactivity is determined. Inhibition of lymphoproliferation is assessed as percent change in counts per minute from non-drug treated controls. The results are expressed as an $IC_{50}$.

A representative compound of this invention was also evaluated in an in vivo test procedure designed to determine the survival time of pinch skin graft from male DBA/2 donors transplanted to male BALB/c recipients. The method is adapted from Billingham R.E. and Medawar P.B., J. Exp. Biol. 28:385-402, (1951). Briefly, a pinch skin graft from the donor is grafted on the dorsum of the recipient as a homograft, and an autograft is used as control in the same region. The recipients are treated with either varying concentrations of cyclosporin A as test control or the test compound, intraperitoneally. Untreated recipients serve as rejection control. The graft is monitored daily and observations are recorded until the graft becomes dry and forms a blackened scab. This is considered as the rejection day. The mean graft survival time (number of days ± S.D.) of the drug treatment group is compared with the control group.

The following table summarizes the results of representative compounds of this invention in these three standard test procedures.

TABLE 1

| Compound | L A F ($IC_{50}$) | Skin Graft (days + SD) |
|---|---|---|
| 27-Hydroxyrapamycin | 3.7 nM | 8.5 ± 1.2* |
| | | 8.17 ± 0.75* |
| | | 8.00 ± 0.63* |
| | | 9.17 ± 0.98+ |
| | | 9.17 ± 0.75+ |
| Example 5 | 99 nM# | |
| Rapamycin | 4.8 nM | 12.0 ± 1.7* |
| No Treatment | | 7.2 ± 0.45 |

* Evaluated in the skin graft procedure at a dose of 4 mg/kg.

+ Evaluated in the skin graft procedure at a dose of 16 mg/kg.

# 94% inhibition of T-cell proliferation at 1 µM and 69% inhibition at 0.1 µM.

The results of these standard pharmacological test procedures demonstrate immunosuppressive activity both in vitro and in vivo for the compounds of this invention. The results obtained in the LAF test procedure indicates suppression of T-cell proliferation. As a transplanted pinch skin grafts are typically rejected within 6-7 days without the use of an immunosuppressive agent, the increased survival time of the skin graft when treated with the compounds of this invention further demonstrates their utility as immunosuppressive agents.

Because the compounds of this invention are structurally similar to rapamycin and have a similar activity profile to rapamycin, the compounds of this invention also are considered to have antitumor, antifungal, and antiproliferative activities.

As such, the compounds of this invention are useful in the treatment of transplantation rejection such as, heart, kidney, liver, bone marrow, and skin transplants; autoimmune diseases such as lupus, rheumatoid arthritis, diabetes mellitus, myasthenia gravis, and multiple sclerosis; diseases of inflammation such as psoriasis, dermatitis, eczema, seborrhea, inflammatory bowel disease, and eye uveitis; solid tumors; fungal infections; and hyperproliferative vascular diseases such as restenosis.

Additionally, 27-Hydroxyrapamycin was found to have a half life of 17.5 hours in 0.1 M phosphate buffer (pH 7.4, 37° C) whereas rapamycin was found to have a half life of 11.8 hours under the same conditions. Therefore, by virtue of the reduced ketone at the 27-position, 27-hydroxyrapamycin provides an advantage over rapamycin by preventing degredative ring opening reactions, thus resulting in a more stable compound. The 27-hydroxyrapamycin derivatives of formulas II - X are also expected to resist ring degredative reactions better than rapamycin and 31- and/or 42-substituted rapamycin derivatives of rapamycin. The half life of 27-hydroxyrapamycin-27-ester with acetic acid in 0.1 M phosphate buffer (pH 7.4, 37° C) is 34 hours.

As 27-hydroxyrapamycin and the compound of Example V was prepared via its 31,42-silylated intermediate (Example 2), the compound of Example 2 is therefore useful as an intermediate of these two compounds. Additionally, 31,42-Bis-triethylsilyl ether of 27-hydroxyrapamycin-27-ester with acetic acid is also a useful intermediate in the preparation of the compound of Example 5.

The compounds of this invention can be formulated neat or with a pharmaceutical carrier to a mammal in need thereof. The pharmaceutical carrier may be solid or liquid.

A solid carrier can include one or more substances which may also act as flavoring agents, lubricants, solubilizers, suspending agents, fillers, glidants, compression aids, binders or tablet-disintegrating agents; it can also be an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers are used in preparing solutions, suspensions, emulsions, syrups, elixirs and pressurized compositions. The active ingredient can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the carrier can also be an oily ester such as ethyl oleate and isopropyl myristate. Sterile liquid carriers are useful in sterile liquid form compositions for parenteral administration. The liquid carrier for pressurized compositions can be halogenated hydrocarbon or other pharmaceutically acceptable propellant.

Liquid pharmaceutical compositions which are sterile solutions or suspensions can be utilized by, for example, intramuscular, intraperitoneal or subcutaneous injection. Sterile solutions can also be administered intravenously. The compounds of this invention can also be administered orally either in liquid or solid composition form.

The compounds of this invention may be administered rectally in the form of a conventional suppository.

For administration by intranasal or intrabronchial inhalation or insufflation, the compounds of this invention may be formulated into an aqueous or partially aqueous solution, which can then be utilized in the form of an aerosol. The compounds of this invention may also be administered transdermally through the use of a transdermal patch containing the active compound and a carrier that is inert to the active compound, is non toxic to the skin, and allows delivery of the agent for systemic absorption into the blood stream via the skin. The carrier may take any number of forms such as creams and ointments, pastes, gels, and occlusive devices. The creams and ointments may be viscous liquid or semisolid emulsions of either the oil-in-water or water-in-oil type. Pastes comprised of absorptive powders dispersed in petroleum or hydrophilic petroleum containing the active ingredient may also be suitable. A variety of occlusive devices may be used to release the active ingredient into the blood stream such as a semipermiable membrane covering a reservoir containing the active ingredient with or without a carrier, or a matrix containing the active ingredient. Other occlusive devices are known in the literature.

In addition, the compounds of this invention may be employed as a solution, cream, or lotion by formulation with pharmaceutically acceptable vehicles containing 0.1 - 5 percent, preferably 2%, of active compound which may be administered to a fungally affected area.

The dosage requirements vary with the particular compositions employed, the route of administration, the severity of the symptoms presented and the particular subject being treated. Based on the results obtained in the standard pharmacological test procedures, projected daily intravenous dosages of the compounds of this invention would be

0.001 - 25 mg/kg, preferably between 0.005 - 5 mg/kg, and more preferably between 0.01 - 0.5 mg/kg. Projected daily oral dosages of the compounds of this invention would be 0.005 - 75 mg/kg, preferably between 0.01 - 50 mg/kg, and more preferably between 0.05 - 10 mg/kg.

Treatment will generally be initiated with small dosages less than the optimum dose of the compound. Thereafter the dosage is increased until the optimum effect under the circumstances is reached; precise dosages for oral, parenteral, intranasal, intrabronchial, transdermal, or rectal administration will be determined by the administering physician based on experience with the individual subject treated. In general, the compounds of this invention, are most desirably administered at a concentration that will generally afford effective results without causing any harmful or deleterious side effects.

The following examples illustrate the preparation of representative compounds of this invention.

**Example 1**

31,42-Bis-triethylsilyl ether of rapamycin

To a solution of rapamycin (2 g, 2.18 mmol) in $CH_2Cl_2$ (10.9 mL) at 0 °C was added 2,6-lutidine (1.17 mL, 10.1 mmol) and triethylsilyl trifluoromethanesulfonate (1.13 mL, 5.03 mmol) dropwise. The reaction was stirred at 0 °C for an additional 45 min, allowed to warm to room temperature, and stirred overnight. The reaction was then quenched with $NaHCO_3$ and diluted with ethyl acetate. The organic layer was separated and washed with 2 N HO, $NaHCO_3$, brine, dried over $Na_2SO_4$, and concentrated *in vacuo.* The residue was chromatographed using hexane-ethyl acetate (4:1) as eluant to provide 1.04 g (42%) of 31,42-bis-triethylsilyl ether of rapamycin.

IR (KBr) 3500 (m, br), 2925 (s), 2875 (s), 1720 (s), 1640 (s), 1450 (s), 1370 (w), 1235 (w), 1185 (w), 1100 (s), 980 (m), 815 (m), 745 (m); $^1$H NMR (400 MHz, $CDCl_3$) δ 0.44-0.50 (comp m, 6 H), 0.52-0.60 (comp m, 6 H), 0.67 (m, 1 H), 0.82-0.96 (comp m, 24 H), 1.00-1.04 (comp m, 9 H), 1.06-1.25 (comp m, 4 H), 1.30-1.60 (comp m, 12 H), 1.61, 1.64 (d, rotamers, *J* = 3.74, 0.80 Hz, 3 H), 1.68-1.83 (comp m, 5 H), 1.72, 1.74 (d, rotamers, *J* = 1.04 Hz, 3 H), 1.96 (m, 1 H), 2.25 (m, 2 H), 2.32 (dd, *J* = 3.00, 15.88 Hz, 1 H), 2.58 (dd, *J* = 8.09, 16.00 Hz, 1 H), 2.68 (m, 1 H), 2.87 (m, 1 H), 3.10, 3.11 (s, rotamers, 3 H), 3.24 (s, 3 H), 3.33 (m, 3 H), 3.37, 3.39 (s, rotamers, 3 H), 3.68 (m, 2 H), 3.75 (m, 1 H), 3.82 (d, J = 6.23 Hz, 1 H), 4.10 (d, *J* = 5.60 Hz, 1 H), 4.68 (d, *J* = 1.66 Hz, 1 H), 5.00 (m, 1 H), 5.20 (d, *J* = 10.17 Hz, 1 H), 5.28 (d, *J* = 4.57 Hz, 1 H), 5.53 (dd, *J* = 8.20, 15.05 Hz, 1 H), 6.02 (dd, *J* = 1.04, 10.79 Hz, 1 H), 6.14 (m, 1 H), 6.34 (qd, *J* = 10.48, 28.94 Hz, 2 H); $^{13}$C NMR (100 MHz, $CDCl_3$) δ 4.63, 4.72, 5.01, 6.68, 6.72, 6.79, 10.14, 12.33, 13.72, 14.94, 15.42, 16.06, 21.46, 25.14, 26.86, 27.31, 31.29, 31.82, 32.97, 33.88, 33.98, 34.08, 35.24, 36.15, 38.60, 38.67, 39.87, 41.70, 42.44, 44.03, 47.03, 51.25, 55.78, 58.07, 58.15, 66.92, 75.61, 79.26, 84.05, 84.11, 84.80, 98.67, 126.81, 127.12, 129.36, 130.68, 132.85, 135.84, 138.16, 139.18, 166.29, 169.61, 193.41, 208.34, 211.46; high resolution mass spectrum (negative ion FAB) *m/z* 1141.7 [(M-H); calcd for $C_{63}H_{106}NO_{13}Si_2$: 1141.6].

**Example 2**

31,42-Bis-triethylsilyl ether of 27-hydroxyrapamycin

To a solution of 31,42-bis-triethylsilyl ether of rapamycin (400 mg, 0.35 mmol) in THF (3.5 mL) at -78 °C was added L-Selectride (0.4 mL, 0.4 mmol, 1 M in THF) dropwise. After 2h, the reaction was quenched with $H_2O$ and EtOAc and allowed to warm to room temperature. The aqueous layer was separated and extracted with EtOAc. The organic layers were combined, washed with brine, dried over $Na_2SO_4$ and concentrated *in vacuo.* The residue was purified via flash column chromatography using hexane-ethyl acetate (3:1) as eluant to provide 140 mg (35%) of 31,42-bis-triethylsilyl ether of 27-hydroxyrapamycin.

IR (KBr) 3300 (s, br), 2950 (s), 2880 (s), 1720 (s), 1640 (s), 1450 (s), 1190 (w), 1100 (s), 1010 (m), 800 (m), 749 (m); $^1$H NMR (400 MHz, $CDCl_3$) δ 0.47 (m, 6 H), 0.49 (m, 6 H), 0.57 (m, 1 H), 0.81-1.00 (comp m, 27 H), 1.01-1.04 (comp m, 6 H), 1.14-1.58 (comp m, 16 H), 1.60 (d,*J*=0.83 Hz, 3 H), 1.63 (d, *J* = 0.83 Hz, 3 H), 1.64-1.82 (comp m, 8 H), 2.00 (m, 2 H), 2.31 (m, 2 H), 2.43 (m, 1 H), 2.78 (m, 1 H), 2.88 (m, 1 H), 3.11 (s, 3 H), 3.21, 3.23 (s, rotamers. 3 H), 3.37 (m, 3 H), 3.40, 3.41 (s, rotamers, 3 H), 3.54 (m, 1 H), 3.70 (m, 1 H), 3.73 (d, *J* = 7.26 Hz, 1 H), 3.78 (m, 1 H), 4.06 (d, *J* = 7.06 Hz, 1 H), 4.81 (s, 1 H), 5.02 (m, 1 H), 5.23 (d, *J* = 8.72 Hz, 1 H), 5.33 (dd, *J* = 0.42, 4.78 Hz, 1 H), 5.66 (dd, *J* = 7.15, 15.04 Hz, 1 H), 6.00 (d, *J* = 9.75 Hz, 1 H), 6.13 (m, 1 H), 6.30 (m, 2 H); $^{13}$C NMR (100 MHz, $CDCl_3$) δ 4.69, 4.99, 5.03, 6.74, 6.82, 10.03, 12.12, 13.78, 14.14, 15.42, 16.16, 20.89, 21.38, 25.37, 27.06, 27.36, 29.69, 31.25, 31.86, 33.20, 33.86, 34.07, 34.15, 34.70, 36.17, 36.37, 38.70, 38.74, 39.71, 42.61, 44.21, 51.17, 55.79, 58.15, 58.22, 67.07, 71.59, 75.70, 79.23, 84.23, 84.85, 98.44, 126.78, 129.51, 130.11, 131.12, 133.31, 135.40, 136.02, 139.27, 167.00, 169.73, 192.86, 212.62; high resolution mass spectrum (negative ion FAB) *m/z* 1143.7 [(M-H); calcd for $C_{63}H_{108}NO_{13}Si_2$: 1143.6].

**Example 3**

27-Hydroxyrapamycin

31,42-Bis-triethylsilyl ether of 27-hydroxyrapamycin (101 mg, 0.088 mmol) was dissolved in 1.5 mL of HOAc:THF:$H_2O$ (3:1:1). Additional THF (0.1 mL) was added to effect solution. The reaction was stirred overnight and then diluted with ethyl acetate and washed with $NaHCO_3$. The aqueous layer was back extracted with ethyl acetate. The organic layers were combined, washed with brine, dried over $Na_2SO_4$, and concentrated *in vacuo.* The residue was purified via flash column chromatography using $CHCl_3$:MeOH (95:5) as eluant to provide 57 mg (70%) of 27-Hydroxyrapamycin.

IR (KBr) 3440 (s, br), 2920 (s), 1740 (s), 1650 (s), 1440 (s), 1370 (w), 1190 (w), 1085 (m), 985 (m); $^1$H NMR (400 MHz, $CDCl_3$) δ 0.68 (m, 1 H), 0.83-1.08 (comp m, 15 H), 1.16-1.62 (comp m, 12 H), 1.66 (s, 3 H), 1.68 (s, 3 H), 1.71-1.88 (comp m, 8 H), 1.98 (m, 2 H), 2.14 (m, 3 H), 2.28 (m, 2 H), 2.39 (m, 1 H), 2.66 (s, 1 H), 2.84 (m, 1 H), 2.94 (m, 1 H), 3.13 (s, 3 H), 3.28 (d, *J* = 1.18 Hz, 1 H), 3.34 (s, 3 H), 3.42 (s, 3 H), 3.47-3.58 (comp m, 3 H), 3.58 (d, *J* = 7.24 Hz, 1 H), 3.65 (m, 1 H), 3.81 (m, 1 H), 4.13 (m, 1 H), 4.84 (s, 1 H), 4.99 (m, 1 H), 5.31 (m, 2 H), 5.55 (dd, *J* = 9.0, 24.0 Hz, 1 H), 5.94 (d, *J* = 10.5 Hz, 1 H), 6.15 (m, 1H), 6.35 (m, 2 H); $^{13}$C NMR (100 MHz, $CDCl_3$) δ 10.09, 12.52, 14.03, 15.67, 16.16, 16.22, 20.56, 21.93, 25.27, 26.94, 27.22, 31.22, 31.27, 31.89, 33.22, 33.31, 33.62, 34.00, 35.37, 35.46, 37.99, 38.77, 38.82, 39.03, 40.09, 40.92, 44.22, 51.33, 55.82, 56.62, 60.03, 67.17, 73.65, 73.92, 78.06, 78.89, 84.46, 85.17, 98.42, 126.25, 129.80, 130.31, 131.01, 133.20, 133.73, 135.16, 140.43, 167.06, 170.14, 192.39, 217.19; high resolution mass spectrum (negative ion FAB) *m/z* 915.3 [(M-H); calcd for $C_{57}H_{80}NO_{13}$: 915.2].

**Example 4**

31,42-Bis-triethylsilyl ether of 27-hydroxyrapamycin-27-ester with acetic acid

To a solution of 31,42-bis-triethylsilyl ether of 27-hydroxyrapamycin (0.74 g, 0.64 mmol) in $CH_2Cl_2$ (3.2 mL) at 0 °C was added pyridine (0.2 mL, 2.58 mmol) and acetyl chloride (0.092 mL, 1.29 mmol) dropwise. The reaction was held at 0 °C for 30 min, allowed to warm to room temperature, and stirred for 3 h. Additional equivalents of pyridine (0.050 mL, 0.61 mmol) and acetyl chloride (0.023 mL, 0.32 mmol) were added at 0 °C. The reaction was again allowed to warm to room temperature and was quenched after an additional 1.5 h with $NaHCO_3$ and diluted with ethyl acetate. The organic layer was separated and washed with 1 N HCl, $NaHCO_3$, brine, dried over $Na_2SO_4$, and concentrated *in vacuo.* The residue was chromatographed using hexane-ethyl acetate (4:1) as eluant to provide 0.237 g (31%) of 31,42-bis-triethylsilyl ether-27-hydroxyrapamycin-27-ester with acetic acid along with 0.154 g (20%) of recovered starting material.

IR (KBr) 3400 (w, br), 2940 (s), 1740 (s), 1650 (m), 1460 (m), 1240 (s), 1105 (s), 1005 (w), 740 (m); $^1$H NMR (400 MHz, $CDCl_3$) δ 0.46-0.54 (comp m, 6 H), 0.57-0.63 (comp m, 6 H), 0.75 (m, 1 H), 0.81-0.99 (comp m, 27 H), 1.05 (m, 6 H), 1.54 (s, 3 H), 1.22-1.63 (comp m, 16 H), 1.64 (d, *J* = 1.8 Hz, 3 H), 1.66-1.98 (comp m, 8 H), 1.99 (s, 3 H), 2.06 (m, 1 H), 2.32 (m, 2 H), 2.62 (m, 1 H), 2.78 (m, 1 H), 2.89 (m, 1 H), 3.14 (s, 3 H), 3.23 (s, 3 H), 3.42 (m, 2 H), 3.43 (s, 3 H), 3.54 (m, 1 H), 3.75 (d, superimposed on m, *J* = 7.2 Hz, 1 H), 3.76 (m, 2 H), 4.08 (d, *J* = 6.7 Hz, 1 H), 4.87 (dd, *J* = 0.41, 4.98 Hz, 1 H), 4.99 (m, 1 H), 5.03 (m, 1 H), 5.20 (d, *J* = 0.4 Hz, 1 H), 5.41 (m, 1 H), 5.78 (m, 1 H), 6.00 (m, 1 H), 6.13 (m, 1 H), 6.37 (m, 2 H); $^{13}$C NMR (100 MHz, $CDCl_3$) δ 4.6, 4.9, 6.7, 6.8, 9.9, 14.0, 14.9, 15.3, 16.2, 20.6, 20.8, 20.9, 25.4, 27.3, 27.4, 30.1, 31.3, 31.7, 33.1, 33.3, 33.5, 33.9, 34.0, 34.2, 36.2, 38.2, 38.6, 39.9, 42.6, 44.0, 50.8, 55.6, 58.0, 58.3, 66.9, 73.7, 75.6, 75.9, 76.4, 79.1, 84.1, 84.4, 98.2, 126.6, 129.6, 129.9, 130.0, 133.6, 134.5, 135.9, 139.0, 167.1, 169.3, 170.5, 191.6, 212.0; high resolution mass spectrum (negative ion FAB) *m/z* 1185.7 [(M-H); calcd for $C_{65}H_{110}NO_{14}Si_2$: 1185.6].

**Example 5**

27-Hydroxyrapamycin-27-ester with acetic acid

31,42-bis-triethylsilyl ether-27-hydroxyrapamycin-27-ester with acetic acid (0.16 g, 0.13 mmol) was dissolved in 2.5 mL of a 3:1:1 solution of HOAc:THF:$H_2O$. The reaction was stirred overnight and was then quenched with $NaHCO_3$ and diluted with ethyl acetate. The organic layer was separated and washed with $NaHCO_3$, brine, dried over $Na_2SO_4$, and concentrated *in vacuo.* The residue was chromatographed using $CH_2Cl_2$:MeOH (20:1) as eluant followed by HPLC (70:30 hexane:ethyl acetate gradient over 60 min to 100% ethyl acetate) to provide 0.050 g (40%) of 27-hydroxyrapamycin-27-ester with acetic acid.

$^1$H NMR (400 MHz, $CDCl_3$) δ 0.68 (m, 1 H), 0.95-1.04 (comp m, 15 H), 1.13-1.69 (comp m, 18 H), 1.59 (s, 3 H), 1.66 (d, *J* = 5.6 Hz, 3 H), 1.78-1.98 (comp m, 9 H), 2.02 (s, 3 H), 2.30 (m, 2 H), 2.68 (m, 1 H), 2.85 (m, 1 H), 2.95 (m, 1 H), 3.13 (s, 3 H), 3.37 (s, 3 H), 3.43 (s, superimposed on m, 3 H), 3.43 (m, 2 H), 3.59-3.70 (comp m, 3 H), 3.79 (m,

1 H), 4.09 (d, *J* = 7.9 Hz, 1 H), 4.80 (m, 2 H), 5.17 (s, 1 H), 5.25 (d, *J* = 10.0 Hz, 1 H), 5.35 (d, *J* = 5.3 Hz, 1 H), 5.76 (dd, *J* = 8.9, 19.8 Hz, 1 H), 5.90 (d, *J* = 9.1 Hz, 1 H), 6.14 (m, 1 H), 6.36 (m, 2 H); high resolution mass spectrum (negative ion FAB) *m/z* 957.2 [(M-H); calcd for $C_{53}H_{82}NO_{14}$: 957.5].

Anal. Calcd for $C_{53}H_{83}NO_{14}$·0.1$Et_2O$: C, 65.9 H, 8.66 N, 1.45.

Found: C, 65.9 H, 8.72 N, 1.34.

The following representative compounds or pharmaceutically acceptable salts thereof could be readily prepared based on the methodology described in this disclosure.

27-Hydroxyrapamycin-27-ester with benzoic acid
27-Hydroxyrapamycin-27-ester with phenylacetic acid
27-Hydroxyrapamycin-27-ester with pyridine-2-carboxylic acid
27-Hydroxyrapamycin-27-ester with trifluoroacetic acid
27-Hydroxyrapamycin-27-ester with 3,3,3-trifluoropropanoic acid
27-Hydroxyrapamycin-27-ester with difluoroacetic acid
27-Hydroxyrapamycin-27-ester with pentafluoropropionic acid
27-Hydroxyrapamycin-27-ester with 4-(dimethylamino)-4-oxobutanoic acid
27-Hydroxyrapamycin-27-ester with 4-oxo-4-[ [2-(2-pyridinyl)ethyl]amino] butanoic acid
27-Hydroxyrapamycin-27-ester with 2-[2-[(3-carboxy-1-oxopropyl)amino]ethyl]-1-methyl-pyridinium iodide
27-Hydroxyrapamycin-27-ester with (4-fluorophenyl)carbamic acid
27-Hydroxyrapamycin-27-ester with phenylcarbamic acid
27-Hydroxyrapamycin-27-ester with 4-[(trifluoromethyl)phenyl]carbamic acid
27-Hydroxyrapamycin-27-ester with (4-nitrophenyl)carbamic acid
27-Hydroxyrapamycin-27-ester with (4-methyl-phenyl)carbamic acid
27-Hydroxyrapamycin-27-ester with (2,4-difluorophenyl)carbamic acid
27-Hydroxyrapamycin-27-ester with N-[(1,1-dimethylethoxy)carbonyl]-glycylglycine
27-Hydroxyrapamycin-27-ester with N-[(1,1-dimethylethoxy)carbonyl]-N-methyl-glycine
27-Hydroxyrapamycin-27-ester with 5-(1,1-dimethylethoxy)-2-[[(1,1-dimethylethoxy)carbonyl]amino]-5-oxopentanoic acid
27-Hydroxyrapamycin-27-ester with 2-[[(1,1-dimethylethoxy)carbonyl]amino] -4-oxo-4-(phenylmethoxy)butanoic acid
27-Hydroxyrapamycin-27-ester with 3-[[(1,1-dimethylethoxy)carbonyl]amino]-4-oxo-4-(phenylmethoxy)butanoic acid
27-Hydroxyrapamycin-27-ester with 5-(1,1-dimethyl-oxy)-4-[[(1,1-dimethylethoxy)-carbonyl]amino]-5-oxopentanoic acid
27-Hydroxyrapamycin-27-ester with $N^{\alpha}$,$N^{\varepsilon}$-bis[(1,1-dimethylethoxy)carbonyl]-L-lysine
27-Hydroxyrapamycin-27-ether with (1-methoxy-1-methyl)ethanol
27-Hydroxyrapamycin-27-ether with (2-(trimethylsilyl)ethoxy)methanol
27-Hydroxyrapamycin-27-ester with N,N-dimethylglycine
27-Hydroxyrapamycin-27-ester with 3-(N,N-diethylamino)propionic acid
27-Hydroxyrapamycin-27-ester with 4'-(N-pyrrolidino)butyric acid
27-Hydroxyrapamycin-27-ester with phenylsulfonylcarbamic acid
27-Hydroxyrapamycin-27-ester with (4-chlorophenylsulfonyl)carbamic acid
27-Hydroxyrapamycin-27-ester with (3-methylphenylsulfonyl)carbamic acid
27-Hydroxyrapamycin-27-ester with 5-(dimethylamino)-1-naphthalensulfonic acid
27-Hydroxyrapamycin-27-ester with 4-methylbenzenesulfonic acid
27-Hydroxyrapamycin-27-ester with 2-thiophenesulfonic acid
27-Hydroxyrapamycin-27-ester with 4-[[4-(dimethylamino)phenyl]aza]benzene-sulfonic acid
27-Hydroxyrapamycin-27-ester with 1-naphthalenesulfonic acid
27-Hydroxyrapamycin-27-ester with 8-quinolinsulfonic acid
27-Hydroxyrapamycin-27-ester with methanesulfonic acid
27-Hydroxyrapamycin-27-ester with 2,2,2-trifluoroethanesulfonic acid
27-Hydroxyrapamycin-27-ester with [(methoxycarbonyl)amino]sulfonic acid

## Claims

1. A compound which is 27-hydroxyrapamycin.

**2.** A compound of the formula

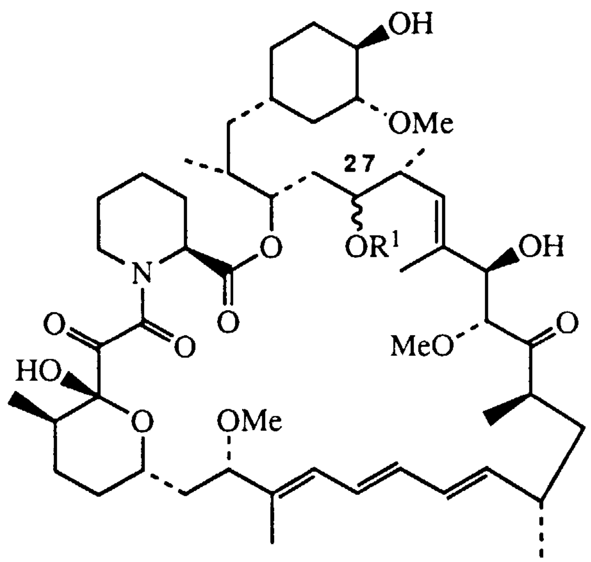

II

wherein

$R^1$ is

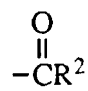

and

$R^2$ is alkyl of 1-10 carbon atoms, arylalkyl of 7-10 carbon atoms, or aryl wherein the aryl group may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof.

**3.** A compound of claim 2 which is 27-hydroxyrapamycin-27-ester with acetic acid.

**4.** A compound of the formula

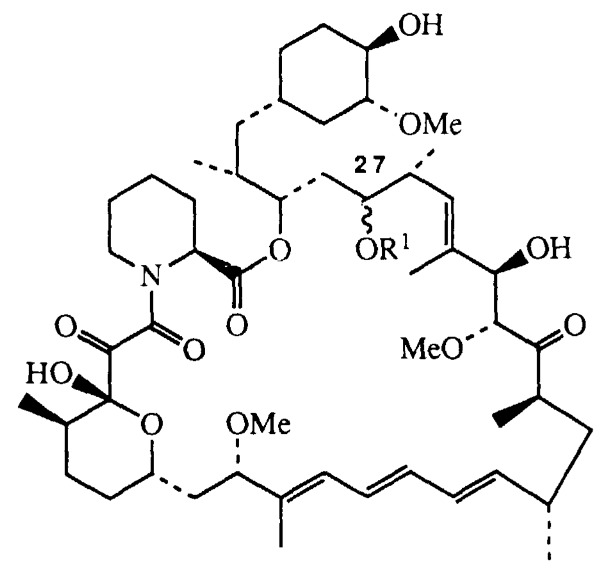

III

wherein

R¹ is

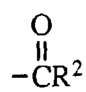

and

$R^2$ is a mono-, di-, poly-, or per-fluorinated alkyl group of 1-10 carbon atoms.

5. A compound of the formula

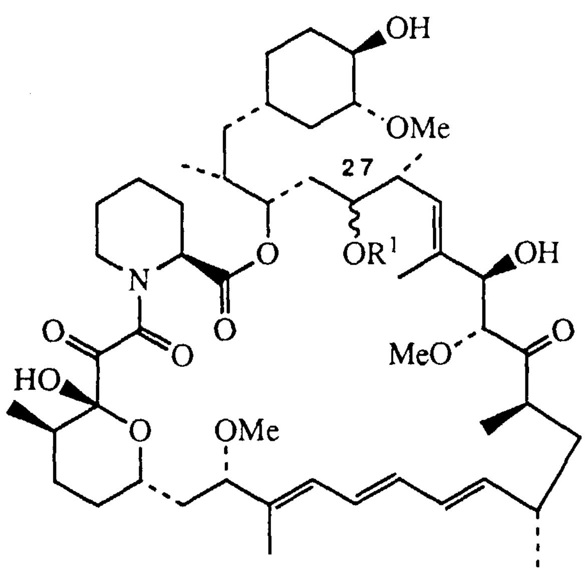

IV

wherein

$R^1$ is

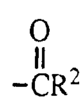 ;

$R^2$ is

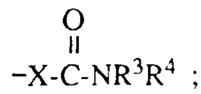

X is $-(CH_2)_m-$ or $-Ar^1-$; where $-AR^1-$ is an optionally mono- or di-substituted group selected from:

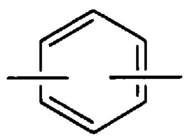 , 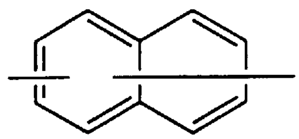 , 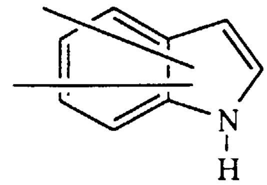 ,

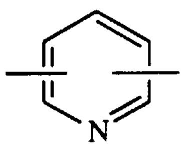 , 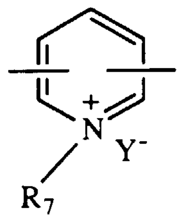 , 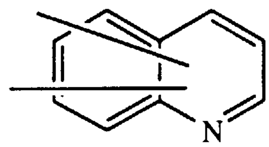 or 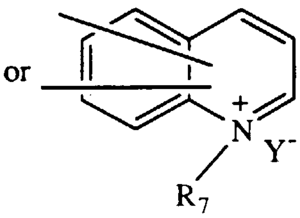

$R^3$ and $R^4$ are each, independently, hydrogen, alkyl of 1-12 carbon atoms, $-(CH_2)_n-Ar$, $-(CH_2)_p-NR^5R^6$ , or $-(CH_2)_p-N^+R^5R^6R^7Y^-$ ;

$R^5$ and $R^6$ are each, independently, hydrogen, alkyl of 1-12 carbon atoms, or $-(CH_2)_n-Ar$;

Ar is an optionally mono- or di- substituted group selected from

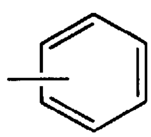 , 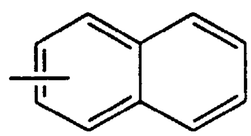 , 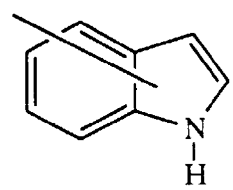 ,

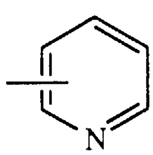 , 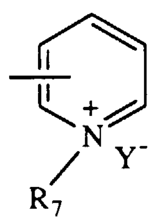 , 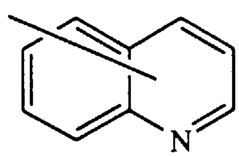 or 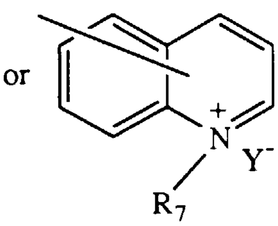

in which the optional substituents are selected from the group consisting of alkyl of 1-6 carbon atoms, arylalkyl of 7-10 carbon atoms, alkoxy of 1-6 carbon atoms, cyano, halo, nitro, carbalkoxy of 2-7 carbon atoms, or perfluoroalkyl of 1-6 carbon atoms;

$R^7$ is alkyl of 1-6 carbon atoms;

Y is a halide, sulfate, phosphate, or p-toluenesulfonate anion;

m = 1-6;

n = 1-6;

p = 1-6;

or a pharmaceutically acceptable salt thereof.

**6.** A compound of the formula

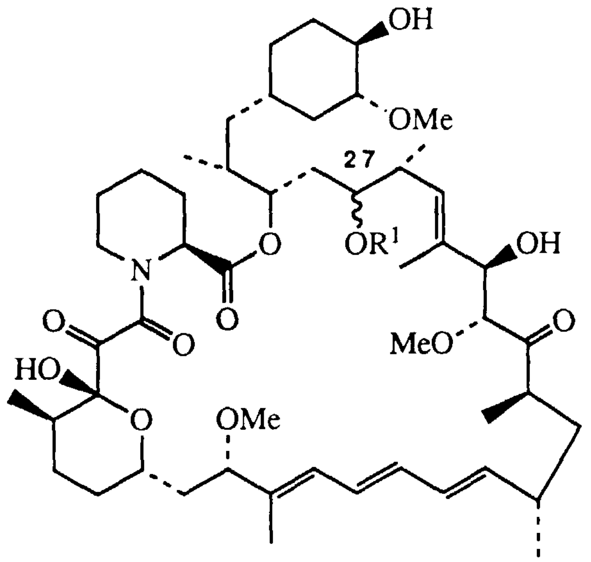

V

wherein

$R^1$ is

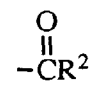 ;

| | |
|---|---|
| $R^2$ is | $-NH(CR^3R^4)_n-X$ ; |
| $R^3$ and $R^4$ are | each, independently, hydrogen, alkyl of 1-6 carbon atoms, arylalkyl of 7-10 carbon atoms, cycloalkyl of 3-8 carbon atoms, halogen, or trifluoromethyl; |
| X is | hydrogen, lower alkyl of 1-6 carbon atoms, cycloalkyl of 3-8 carbon atoms, trifluoromethyl, nitro, alkoxy of 1-6 carbon atoms, carboalkoxy of 2-7 carbon atoms, arylalkyl of 7-10 carbon atoms, halo, dialkylamino of 1-6 carbon atoms per alkyl group, thioalkyl of 1-6 carbon atoms, or Y; |
| Y is | a phenyl group which may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, dialkylamino of 1-6 carbon atoms per alkyl group, or alkylthio of 1-6 carbon atoms, $-SO_3H$, $-PO_3H$, and $-CO_2H$; |

n = 0-5;
with the proviso that when n = 0, X is lower alkyl of 1-6 carbon atoms, cycloalkyl of 3-8 carbon atoms, arylalkyl of 7-10 carbon atoms, or Y;
or a pharmaceutically acceptable salt thereof.

**7.** A compound of the formula

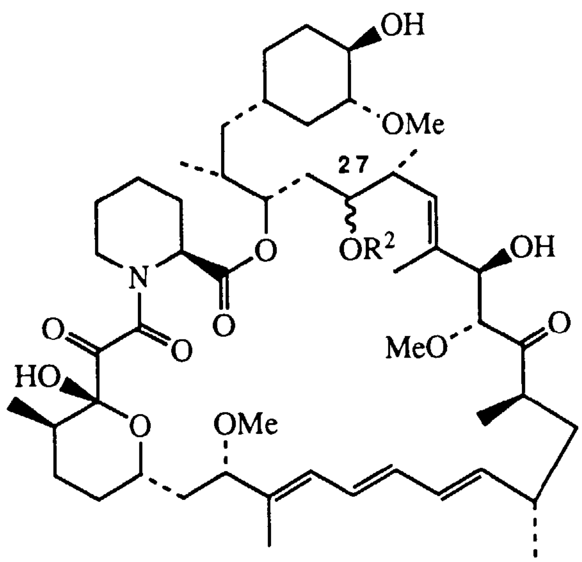

VI

wherein

$R^2$ is

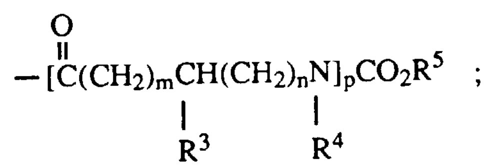

| | |
|---|---|
| $R^3$ is | hydrogen, alkyl of 1-6 carbon atoms, arylalkyl of 7-10 carbon atoms, $-(CH_2)_qCO_2R^6$, $-(CH_2)_rNR^7CO_2R^8$, carbamylalkyl of 2-3 carbon atoms, aminoalkyl of 1-4 carbon atoms, hydroxyalkyl of 1-4 carbon atoms, guanylalkyl of 2-4 carbon atoms, mercaptoalkyl of 1-4 carbon atoms, alkylthioalkyl of 2-6 carbon atoms, indolylmethyl, hydroxyphenylmethyl, imidazoylmethyl or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| $R^4$ and $R^7$ are | each, independently, hydrogen, alkyl of 1-6 carbon atoms, or arylalkyl of 7-10 carbon atoms; |
| $R^5$, $R^6$, and $R^8$ are | each, independently, alkyl of 1-6 carbon atoms, arylalkyl of 7-10 carbon atoms, fluorenylmethyl, or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| m is | 0-4; |
| n is | 0-4; |
| p is | 1-2; |
| q is | 0-4; |
| r is | 0-4; |

wherein $R^3$, $R^4$, m, and n are independent in each of the

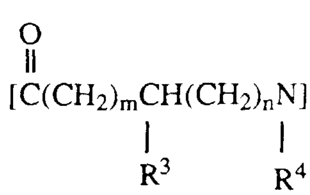

subunits when p = 2;

or a pharmaceutically acceptable salt thereof.

**8.** A compound of the formula

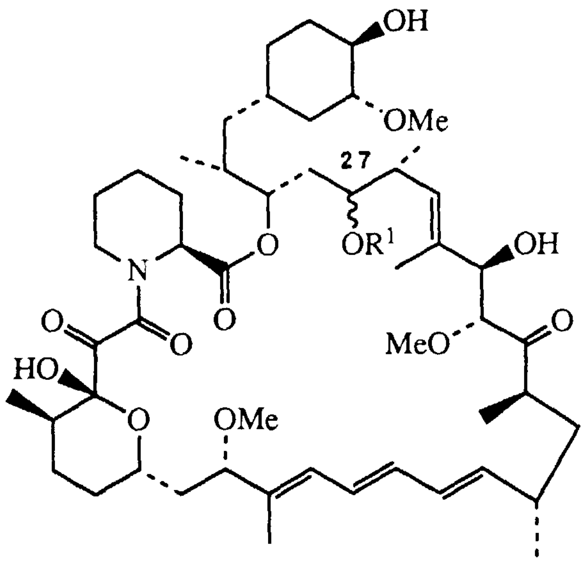

VII

wherein

R[1] is alkyl of 1-6 carbon atoms, arylalkyl of 7-10 carbon atoms, $-CH_2YX$, $-C(CH_3)_2YX$, $-CH(CH_3)YX$, or L ;
Y is O or S;
X is $-CH_3$, $-(CH_2)_nCH_3$, $-CH_2Ar$, $-(CH_2)_2OCH_3$, $-CH_2CCl_3$, $-CH(CH_3)_2$, or $-CH_2CH_2SiMe_3$ ;
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri-substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;
L is tetrahydrofuran-2-yl, tetrahydrothiophen-2-yl, tetrahydrothiopyran-2-yl, tetrahydropyran-2-yl, 4-methoxytetrahydropyran-2-yl, 4-methoxytetrahydrothiopyran-2-yl, or 4-methoxytetrahydrothiopyran-2-yl S,S dioxide; and

n = 1-5.

**9.** A compound of the formula

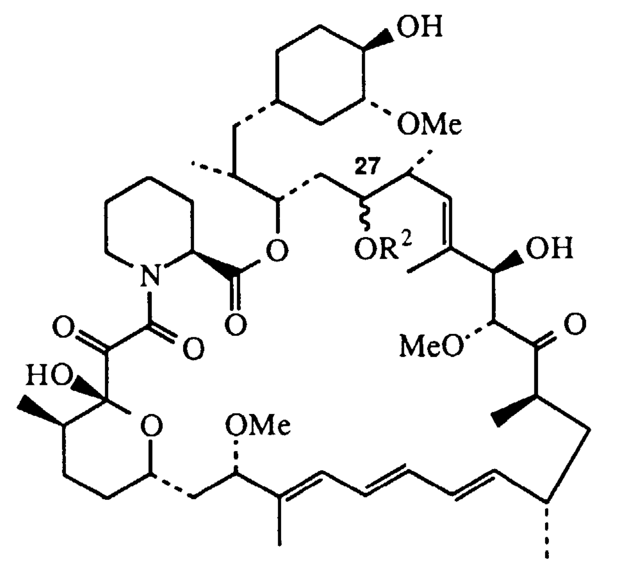

VIII

wherein

$R^2$ is

$$-\overset{O}{\overset{\|}{C}}(CH_2)_mNRR^1 \;;$$

R and $R^1$ are each hydrogen or alkyl of 1-3 carbon atoms or R and $R^1$ together with the nitrogen to which they are attached form a saturated heterocyclic ring having 4-5 carbon atoms; and

m = 1-3; or a pharmaceutically acceptable salt thereof.

**10.** A compound of the formula

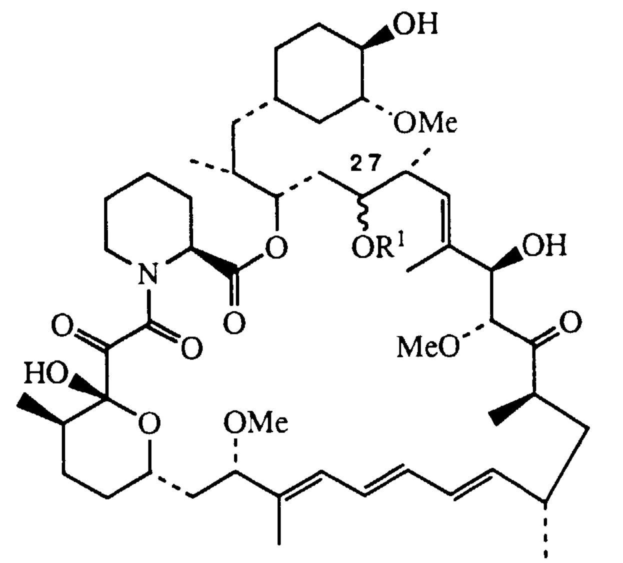

IX

wherein

$R^1$ is -$CONHSO_2$-Ar ; and
Ar is phenyl, naphthyl, pyridyl, quinolyl, isoquinolyl. quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof when the Ar group contains a basic nitrogen or when the Ar group is substituted by dialklyamino of 1-6 carbon atoms per alkyl group, $-SO_3H$, $-PO_3H$, or $-CO_2H$.

11. A compound of the formula

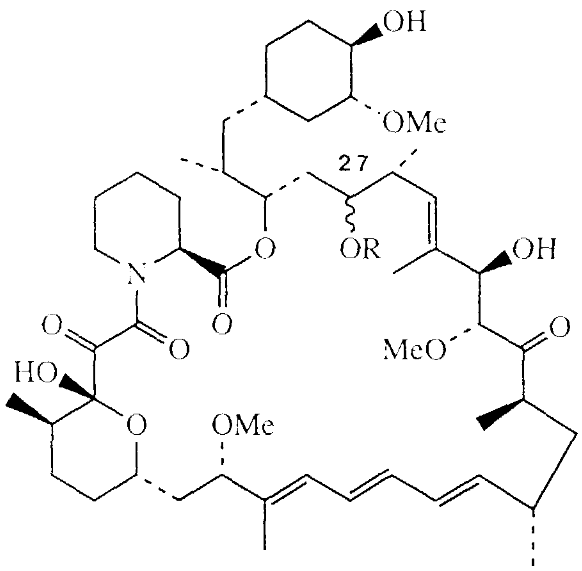

X

wherein

R is $-SO_2R^1$;
$R^1$ is alkyl, alkenyl, alkenyl containing 1 to 6 carbon atoms; or an aromatic moiety selected from the group consisting of phenyl and naphthyl or a heterocyclic moiety selected from the group consisting of thiophenyl and quinolinyl; or $-NHCOR^2$; and
$R^2$ is lower alkyl containing 1 to 6 carbon atoms;

or a pharmaceutically acceptable salt thereof.

12. Use of 27-hydroxyrapamycin in the preparation of a medicament for use in inducing immunosuppression.

13. A use according to claim 12 wherein the induced immunosuppression is used to prevent or treat transplantation rejection or host versus graft disease.

14. A use according to claim 12 wherein the induced immunosuppression is used to treat autoimmune diseases, diseases of inflammation, or hyperproliferative vascular disorders.

15. Use of a compound of formula II,

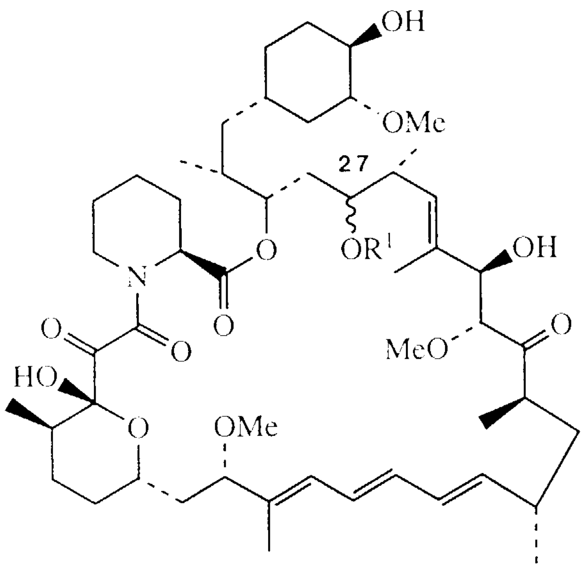

II

wherein

$R^1$ is

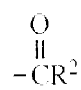

and

$R^2$ is alkyl of 1-10 carbon atoms, arylalkyl of 7-10 carbon atoms, or aryl wherein the aryl group may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable acceptable salt thereof,
or of formula III,

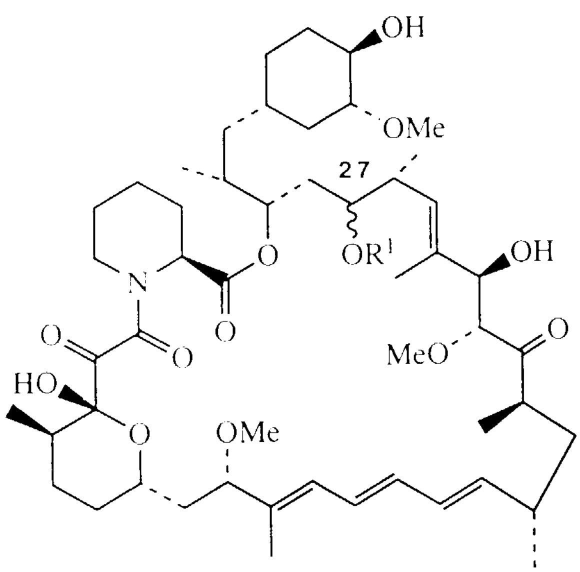

III

wherein

$R^1$ is

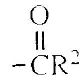

and

$R^2$ is a mono-, di-, poly-, or per-fluorinated alkyl group of 1-10 carbon atoms;

or of formula IV.

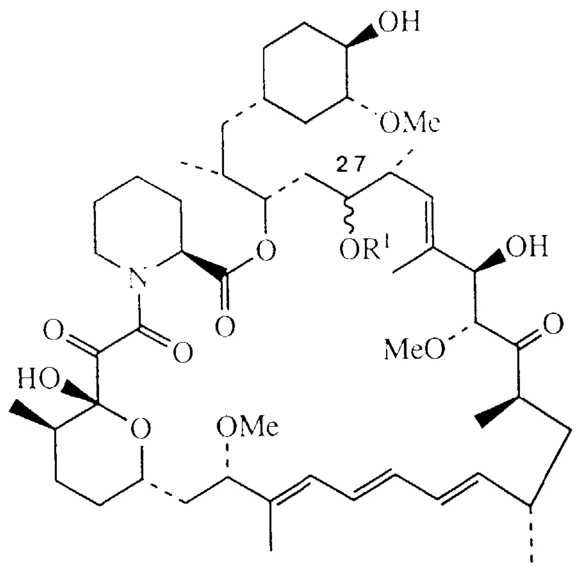

IV

wherein

$R^1$ is

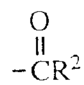 ;

$R^2$ is

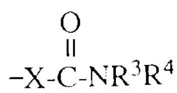 ;

X is $-(CH_2)_m-$ or $-Ar^1-$ ; where $-Ar^1-$ is an optionally mono- or di-substituted group selected from:

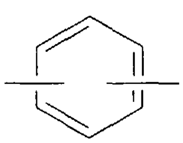 , 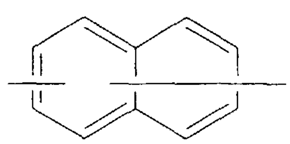 , 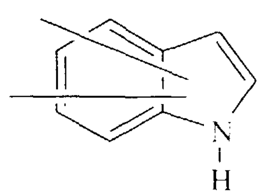 ,

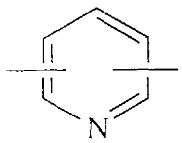 , 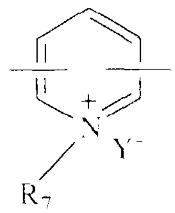 , 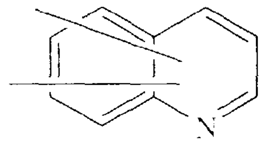 or 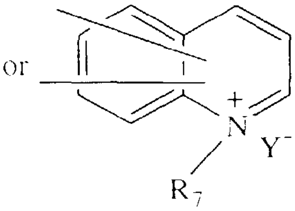

$R^3$ and $R^4$ are each, independently, hydrogen, alkyl of 1-12 carbon atoms, $-(CH_2)_n-Ar$, $-(CH_2)_p-NR^5R^6$, or $-(CH_2)_p-N^+R^5R^6R^7Y^-$ ;

$R^5$ and $R^6$ are each, independently, hydrogen, alkyl of 1-12 carbon atoms, or $-(CH_2)_n-Ar$;

Ar is an optionally moni- or di- substituted group selected from

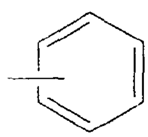 , 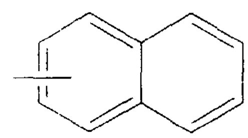 , 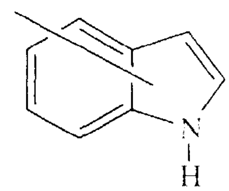 ,

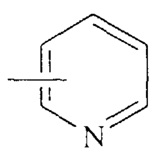 , 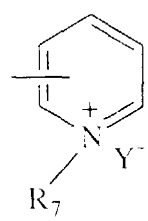 , 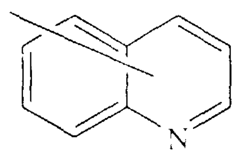 or 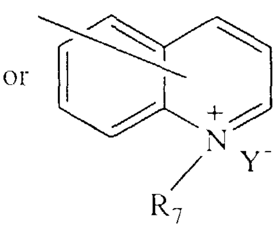

in which the optional substituents are selected from the group consisting of alkyl of 1-6 carbon atoms, arylalkyl of 7-10 carbon atoms, alkoxy of 1-6 carbon atoms, cyano, halo, nitro, carbalkoxy of 2-7 carbon atoms, or perfluoroalkyl of 1-6 carbon atoms;

$R^7$ is alkyl of 1-6 carbon atoms;

Y is a halide, sulfate, phosphate, or p-toluenesulfonate anion;

m = 1-6;
n = 1-6;
p = 1-6;
or a pharmaceutically acceptable salt thereof;
or of formula V,

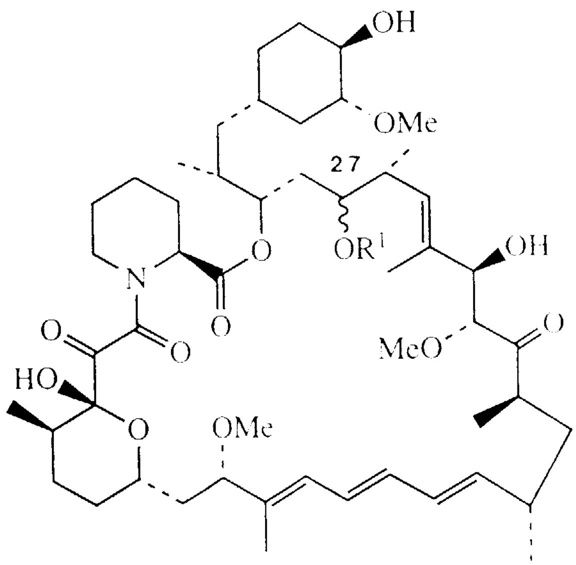

V

wherein

$R^1$ is

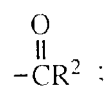

$R^2$ is $-NH(CR^3R^4)_n-X$ ;

$R^3$ and $R^4$ are each, independently, hydrogen, alkyl of 1-6 carbon atoms, arylalkyl of 7-10 carbon atoms, cycloalkyl of 3-8 carbon atoms, halogen, or trifluoromethyl;

X is hydrogen, lower alkyl of 1-6 carbon atoms, cycloalkyl of 3-8 carbon atoms, trifluoromethyl, nitro, alkoxy of 1-6 carbon atoms, carboalkoxy of 2-7 carbon atoms, arylalkyl of 7-10 carbon atoms, halo, dialkylamino of 1-6 carbon atoms per alkyl group, thioalkyl of 1-6 carbon atoms, or Y;

Y is a phenyl group which may be optionally mono-, di-, or tri- substituted with a group selected from alkyl of 1-6 carbon atoms. arylalkyl of 7-10 carbon atoms, alkoxy of 1-6 carbon atoms. cyano, halo, nitro, carbalkoxy of 2-7 carbon atoms, trifluoromethyl, dialkylamino of 1-6 carbon atoms per alkyl group, or alkylthio of 1-6 carbon atoms, $-SO_3H$, $-PO_3H$, and $-CO_2H$;

n = 0-5;
with the proviso that when n = 0, X is lower alkyl of 1-6 carbon atoms, cycloalkyl of 3-8 carbon atoms, arylalkyl of 7-10 carbon atoms, or Y;
or a pharmaceutically acceptable salt thereof:
or of formula VI,

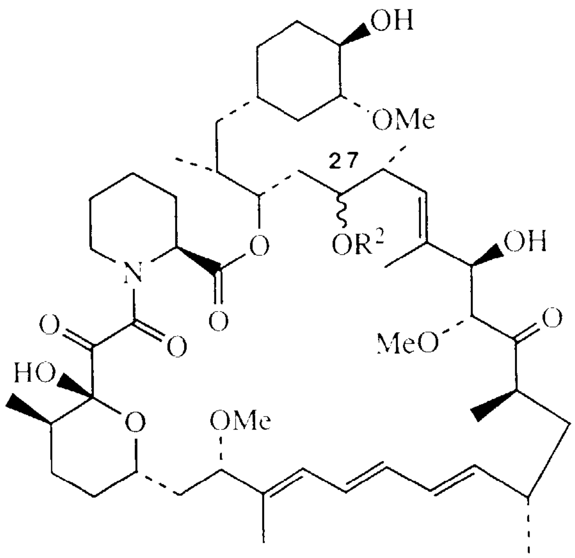

VI

wherein

$R^2$ is

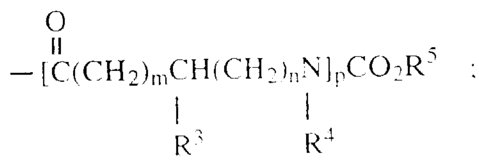

| | |
|---|---|
| $R^3$ is | hydrogen, alkyl of 1-6 carbon atoms, arylalkyl of 7-10 carbon atoms, $-(CH_2)_qCO_2R^6$, $-(CH_2)_rNR^7CO_2R^8$, carbamylalkyl of 2-3 carbon atoms, aminoalkyl of 1-4 carbon atoms, hydroxyalkyl of 1-4 carbon atoms, guanylalkyl of 2-4 carbon atoms, mercaptoalkyl of 1-4 carbon atoms, alkylthioalkyl of 2-3 carbon atoms, indolylmethyl, hydroxyphenylmethyl, imidazoylmethyl or phenyl which is optionally mono-, di-, or tri-substituted with a subtituent selected from alkyl of 1-6 carbon atoms, alkoxy of 1-6 carbon atoms, hydroxy cyano, halo, nitro, carbalkoxy of 2-7 carbon atoms, trifluoromethyl, amino, or $-CO_2H$: |
| $R^4$ and $R^7$ are | each, independently, hydrogen, alkyl of 1-6 carbon atoms, or arylalkyl of 7-10 carbon atoms; |
| $R^5$, $R^6$, and $R^8$ are | each, independently, alkyl of 1-6 carbon atoms, arylalkyl of 7-10 carbon atoms, fluorenylmethyl, or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| m is | 0-4; |
| n is | 0-4; |
| p is | 1-2; |
| q is | 0-4; |
| r is | 0-4; |

wherein $R^3$, $R^4$, m, and n are independent in each of the

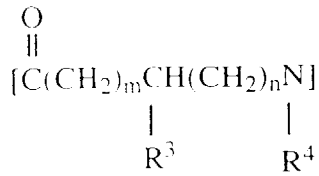

subunits when p = 2;

or a pharmaceutically acceptable salt thereof;
or of formula VII,

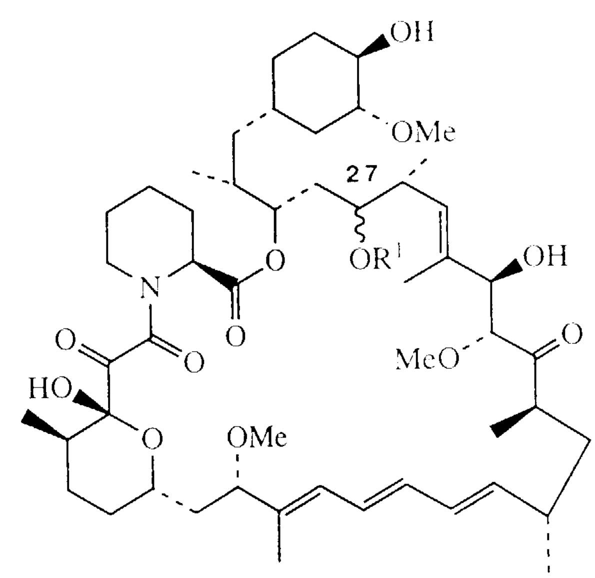

VII

wherein $R^1$ is alkyl of 1-6 carbon atoms, arylalkyl of 7-10 carbon atoms, $-CH_2YX$, $-C(CH_3)_2YX$, $-CH(CH_3)YX$, or L;

Y is O or S;
X is $-CH_3$, $-(CH_2)_nCH_3$, $-CH_2Ar$, $-(CH_2)_2OCH_3$, $-CH_2CCl_3$, $-CH(CH_3)_2$, or $-CH_2CH_2SiMe_3$;
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri-substituted with a group selected from alkyl of 1-6 carbon atoms, arylalkyl of 7-10 carbon atoms, alkoxy of 1-6 carbon atoms, cyano halo, nitro, carbalkoxy of 2-7 carbon atoms, trifluoromethyl. amino, dialkylamino of 1-6 carbon atoms per alkyl group, alkylthio of 1-6 carbon atoms, $-SO_3H$, $-PO_3H$, and $-CO_2H$;
L is tetrahydrofuran-2-yl, tetrahydrothiophen-2-yl, tetrahydrothiopyran-2-yl, tetrahydropyran-2-yl, 4-methoxytetrahydropyran-2-yl, 4-methoxytetrahydrothiopyran-2-yl, or 4-methoxytetrahydrothiopyran-2-yl S,S dioxide; and

n = 1-5; or of formula VIII,

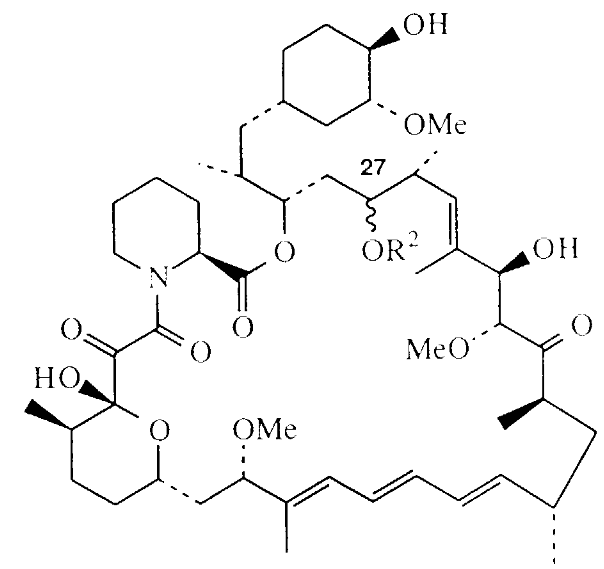

VIII

wherein

$R^2$ is

$$-\overset{O}{\overset{\|}{C}}(CH_2)_mNRR^1 ;$$

R and $R^1$ areeach hydrogen or alkyl of 1-3 carbon atoms or R and $R^1$ together with the nitrogen to which they are attached form a saturated heterocyclic ring having 4-5 carbon atoms; and

m= 1-3; or a pharmaceutically acceptable salt thereof;
or of formula IX,

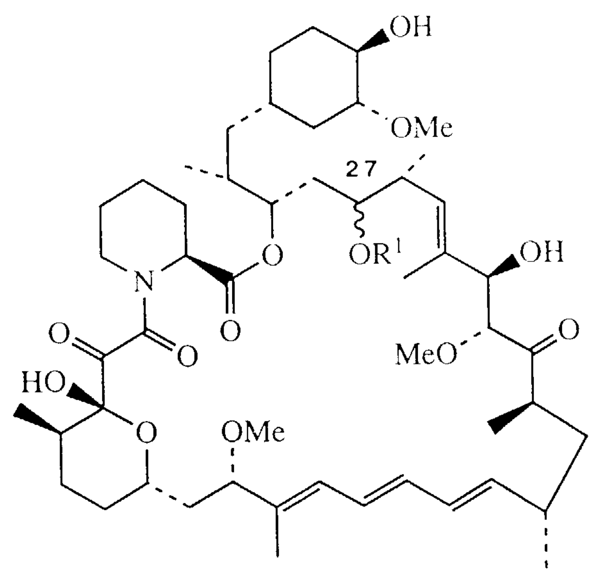

IX

wherein

$R^1$ is -$CONHSO_2$-Ar ; and
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-,

di-, or tri-substituted with a group selected from alkyl of 1-6 carbon atoms arylalkyl of 7-10 carbon atoms, alkoxy of 1-6 carbon atoms, cyano, halo, nitro, carbalkoxy of 2-7 carbon atoms, trifluromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof when the Ar group contains a basic nitrogen or when the Ar group is substituted by a dialklyamino of 1-6 carbon atoms per alkyl group, $-SO_3H$, $-PO_3H$, or $-CO_2H$;
or of formula X,

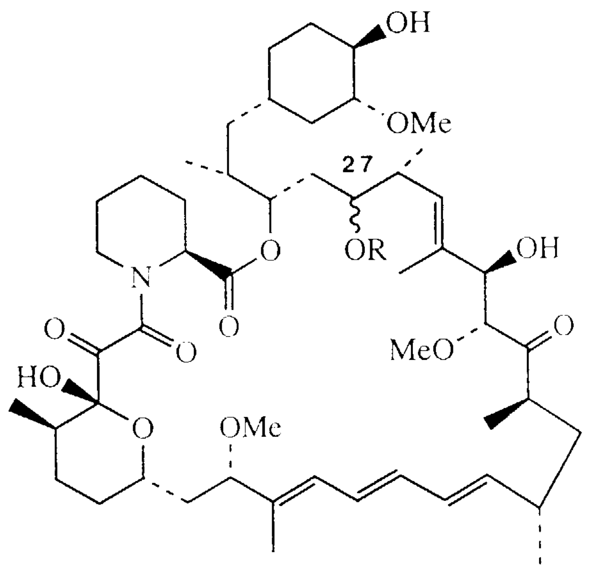

X

wherein

R is $-SO_2R^1$ ;
$R^1$ is alkyl, alkenyl, alkynyl containing I to 6 carbon atoms; or an aromatic moiety selected from the group consisting of phenyl and naphthyl or a heterocyclic moiety selected from the group consisting of thiophenyl and quinolinyl: or $-NHCOR^2$ ; and
$R^2$ is lower alkyl containing 1 to 6 carbon atoms;

or a pharmaceutically acceptable salt thereof, in the preparation of a medicament for inducing immunosuppression.

**16.** A pharmaceutical composition which comprises an effective amount of 27-hydroxyrapamycin and a pharmaceutically acceptable carrier.

**17.** A pharmaceutical composition which comprises an effective amount of a combination of 27-hydroxyrapamycin; an antirejection chemotherapeutic agent selected from the group consisting of azathioprine, corticosteroids, cyclophosphamide, rapamycin, cyclosporin A, FK-506, OKT-3, and ATG; and a pharmaceutically acceptable carrier.

**18.** A pharmaceutical composition which comprises an effective amount of a compound of formula II,

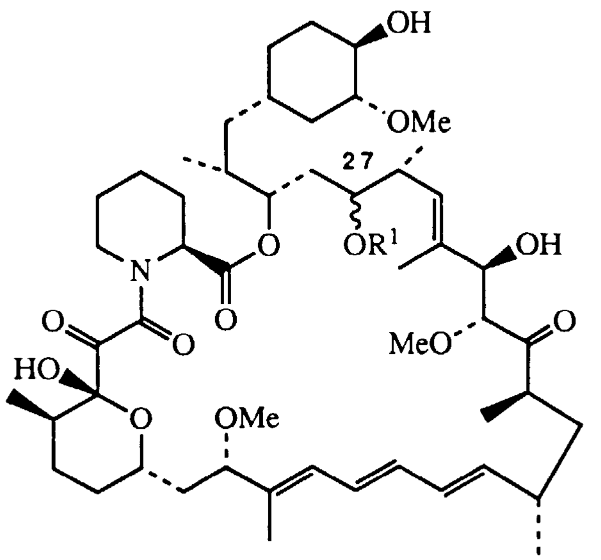

II

wherein

$R^1$ is

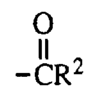

and

$R^2$ is alkyl of 1-10 carbon atoms, arylalkyl of 7-10 carbon atoms, or aryl wherein the aryl group may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof;
or of formula III,

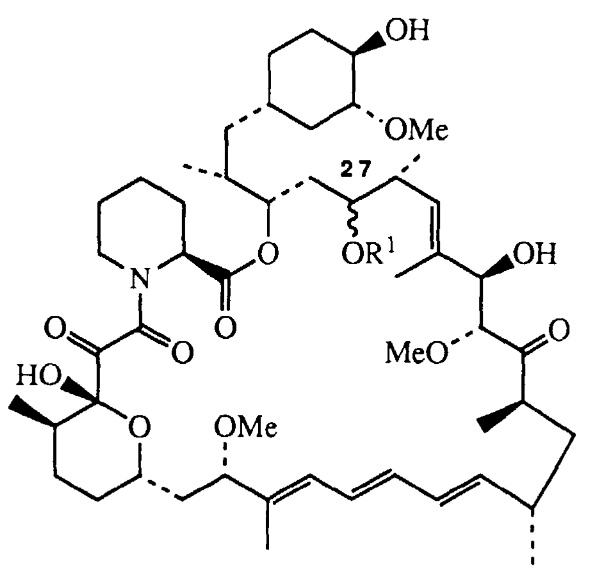

III

wherein

$R^1$ is

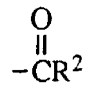

and

$R^2$ is a mono-, di-, poly-, or per-fluorinated alkyl group of 1-10 carbon atoms; or of formula IV,

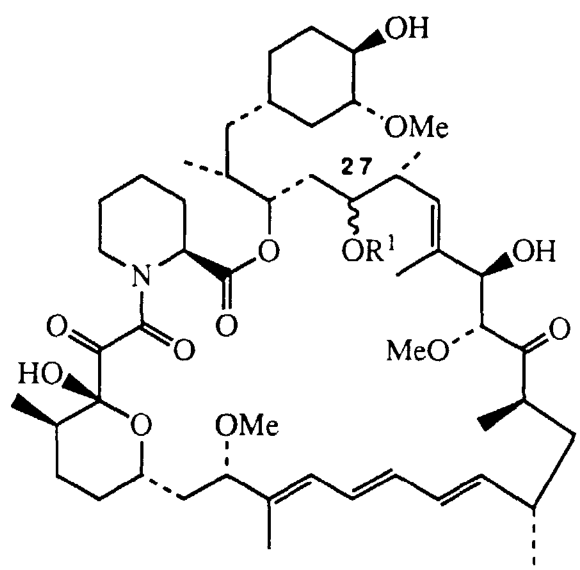

IV

wherein

$R^1$ is

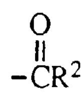 ;

$R^2$ is

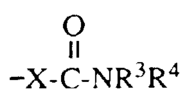 ;

X is $-(CH_2)_m-$ or $-Ar^1-$ ; where $-AR^1-$ is an optionally mono- or di-substituted group selected from:

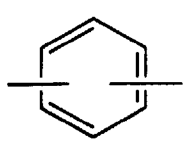 ,

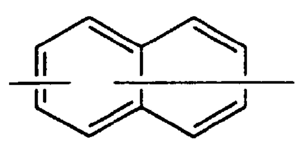 ,

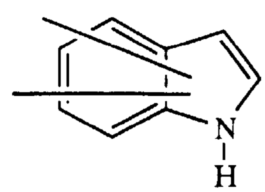 ,

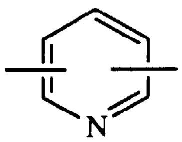 , 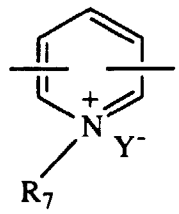 , 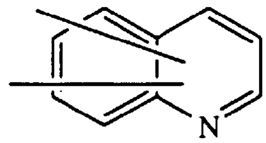 or 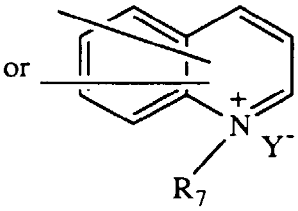

$R^3$ and $R^4$ areeach, independently, hydrogen, alkyl of 1-12 carbon atoms, $-(CH_2)_n-Ar$, $-(CH_2)_p-NR^5R^6$, or $-(CH_2)_p-N^+R^5R^6R^7Y^-$;

$R^5$ and $R^6$ areeach, independently, hydrogen, alkyl of 1-12 carbon atoms, or $-(CH_2)_n-Ar$;

Ar is an optionally mono- or di- substituted group selected from

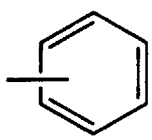 , 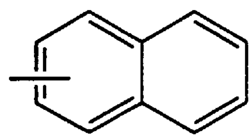 , 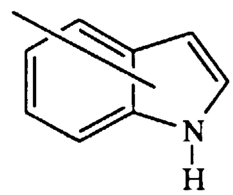 ,

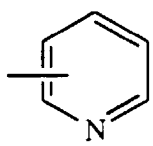 , 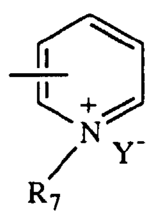 , 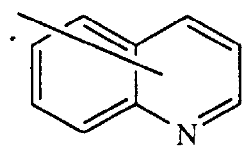 or 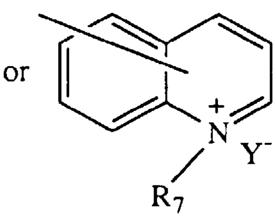

in which the optional substituents are selected from the group consisting of alkyl of 1-6 carbon atoms, arylalkyl of 7-10 carbon atoms, alkoxy of 1-6 carbon atoms, cyano, halo, nitro, carbalkoxy of 2-7 carbon atoms, or perfluoroalkyl of 1-6 carbon atoms;

$R^7$ is alkyl of 1-6 carbon atoms;

Y is a halide, sulfate, phosphate, or p-toluenesulfonate anion;

m = 1-6;
n = 1-6;
p = 1-6;
or a pharmaceutically acceptable salt thereof;
or of formula V,

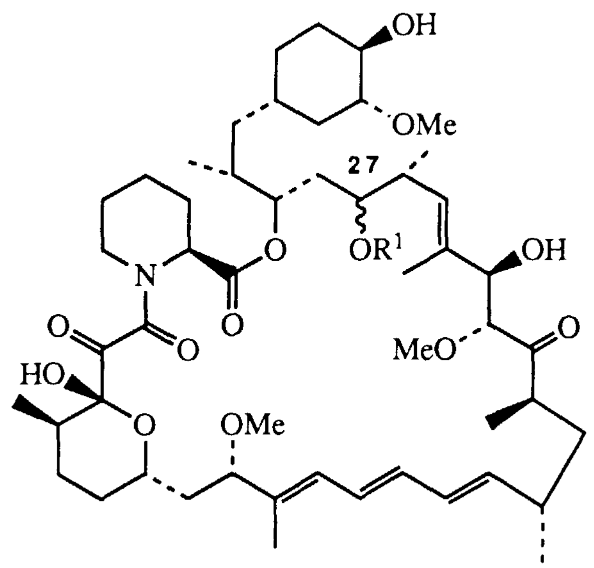

V

wherein

$R^1$ is

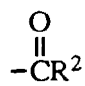 ;

| | |
|---|---|
| $R^2$ is | $-NH(CR^3R^4)_n-X$ ; |
| $R^3$ and $R^4$ are | each, independently, hydrogen, alkyl of 1-6 carbon atoms, arylalkyl of 7-10 carbon atoms, cycloalkyl of 3-8 carbon atoms, halogen, or trifluoromethyl; |
| X is | hydrogen, lower alkyl of 1-6 carbon atoms, cycloalkyl of 3-8 carbon atoms, trifluoromethyl, nitro, alkoxy of 1-6 carbon atoms, carboalkoxy of 2-7 carbon atoms, arylalkyl of 7-10 carbon atoms, halo, dialkylamino of 1-6 carbon atoms per alkyl group, thioalkyl of 1-6 carbon atoms, or Y; |
| Y is | a phenyl group which may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, dialkylamino of 1-6 carbon atoms per alkyl group, or alkylthio of 1-6 carbon atoms, $-SO_3H$, $-PO_3H$, and $-CO_2H$; |

n = 0-5;
with the proviso that when n = 0, X is lower alkyl of 1-6 carbon atoms, cycloalkyl of 3-8 carbon atoms, arylalkyl of 7-10 carbon atoms, or Y;
or a pharmaceutically acceptable salt thereof;
or of formula VI,

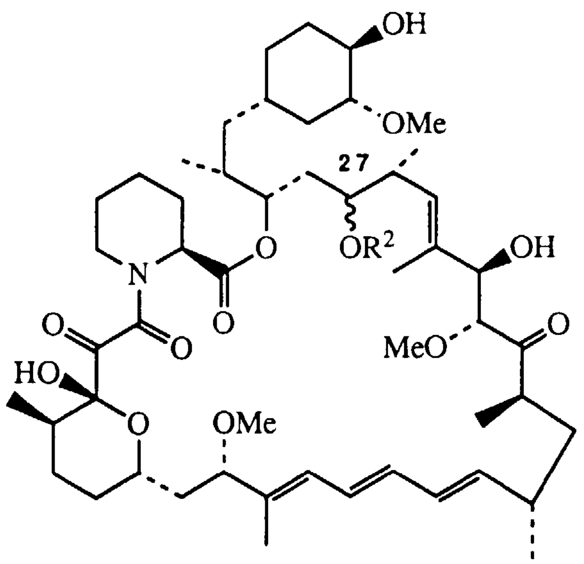

VI

wherein

$R^2$ is

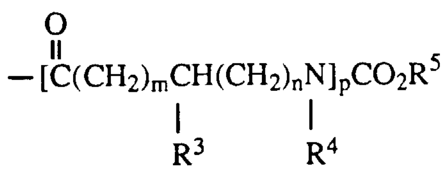

;

| | |
|---|---|
| R3 is | hydrogen, alkyl of 1-6 carbon atoms, arylalkyl of 7-10 carbon atoms, $-(CH_2)_qCO_2R^6$, $-(CH_2)_rNR^7CO_2R^8$, carbamylalkyl of 2-3 carbon atoms, aminoalkyl of 1-4 carbon atoms, hydroxyalkyl of 1-4 carbon atoms, guanylalkyl of 2-4 carbon atoms, mercaptoalkyl of 1-4 carbon atoms, alkylthioalkyl of 2-6 carbon atoms, indolylmethyl, hydroxyphenylmethyl, imidazoylmethyl or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| $R^4$ and $R^7$ are | each, independently, hydrogen, alkyl of 1-6 carbon atoms, or arylalkyl of 7-10 carbon atoms; |
| $R^5$, $R^6$, and $R^8$ are | each, independently, alkyl of 1-6 carbon atoms, arylalkyl of 7-10 carbon atoms, fluorenylmethyl, or phenyl which is optionally mono-, di-, or tri-substituted with a substituent selected from alkyl of 1-6 carbon atoms, alkoxy of 1-6 carbon atoms, hydroxy, cyano, halo, nitro, carbalkoxy of 2-7 carbon atoms, trifluoromethyl, amino, or $-CO_2H$; |
| m is | 0-4; |
| n is | 0-4; |
| p is | 1-2; |
| q is | 0-4; |
| r is | 0-4; |

wherein $R^3$, $R^4$, m, and n are independent in each of the

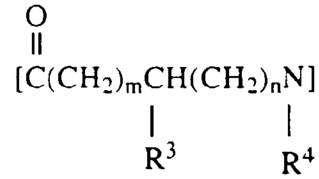

subunits when p = 2;

or a pharmaceutically acceptable salt thereof;
or of formula VII,

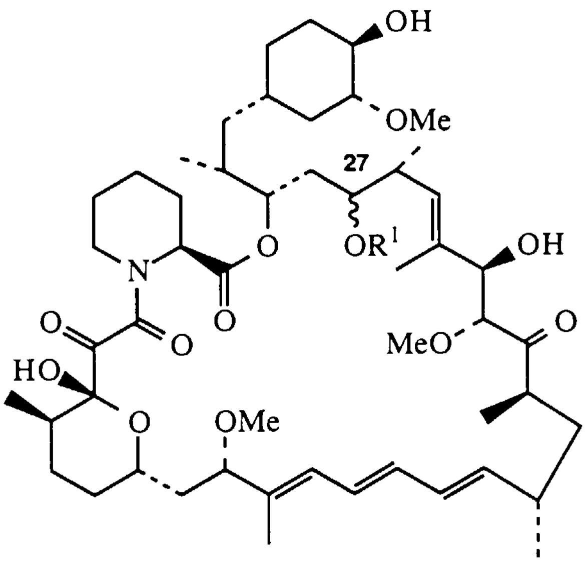

VII

wherein

R[1] is alkyl of 1-6 carbon atoms, arylalkyl of 7-10 carbon atoms, $-CH_2YX$, $-C(CH_3)_2YX$ , $-CH(CH_3)YX$ , or L ;
Y is O or S;
X is $-CH_3$, $-(CH_2)_nCH_3$, $-CH_2Ar$ , $-(CH_2)_2OCH_3$, $-CH_2CCl_3$, $-CH(CH_3)_2$, or $-CH_2CH_2SiMe_3$ ;
Ar is phenyl, naphthyl. pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-, di-, or tri- substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl. amino, dialkylamino of 1-6 carbon atoms per alkyl group, alkylthio of 1-6 carbon atoms, $-SO_3H$, $-PO_3H$, and $-CO_2H$;
L is tetrahydrofuran-2-yl, tetrahydrothiophen-2-yl, tetrahydrothiopyran-2-yl, tetrahydropyran-2-yl, 4-methoxytetrahydropyran-2-yl, 4-methoxytetrahydrothiopyran-2-yl, or 4-methoxytetrahydrothiopyran-2-yl S,S dioxide; and

n = 1-5;
or of formula VIII,

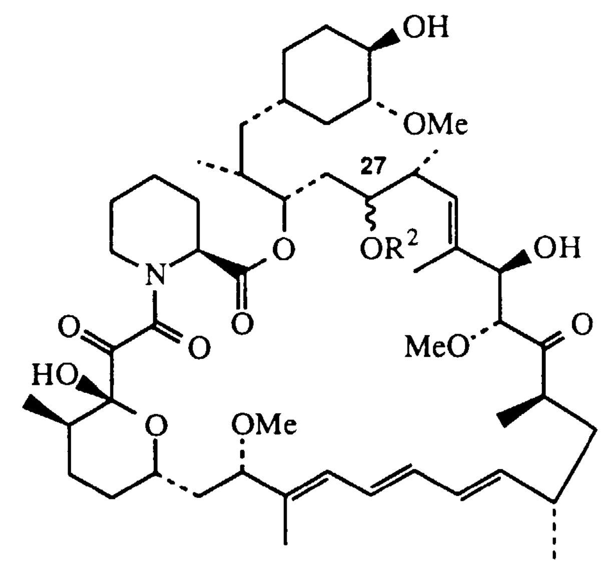

VIII

wherein

$R^2$ is

$$-\overset{O}{\overset{\|}{C}}(CH_2)_mNRR^1\ ;$$

R and $R^1$ are each hydrogen or alkyl of 1-3 carbon atoms or R and $R^1$ together with the the nitrogen to which they are attached form a saturated heterocyclic ring having 4-5 carbon atoms; and

m = 1-3 or a pharmaceutically acceptable salt thereof;
or of formula IX,

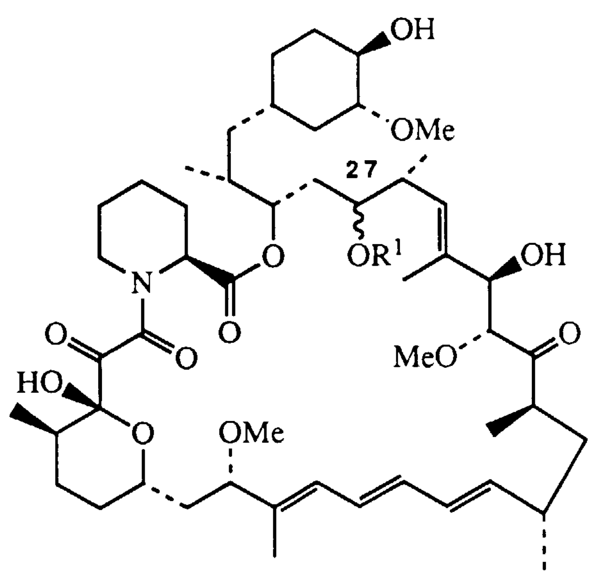

IX

wherein

$R^1$ is -$CONHSO_2$-Ar ; and
Ar is phenyl, naphthyl, pyridyl, quinolyl, isoquinolyl, quinoxalyl, thienyl, thionaphthyl, furyl, benzofuryl, benzodioxyl, benzoxazolyl, benzoisoxazolyl, or benzodioxolyl; wherein the Ar group may be optionally mono-,

di-, or tri-substituted with a group selected from alkyl of 1-6 carbon atoms, arylalkyl of 7-10 carbon atoms, alkoxy of 1-6 carbon atoms, cyano, halo, nitro, carbalkoxy of 2-7 carbon atoms, trifluoromethyl, amino, dialkylamino of 1-6 carbon atoms per alkyl group, alkylthio of 1-6 carbon atoms, $-SO_3H$, $-PO_3H$, and $-CO_2H$;

or a pharmaceutically acceptable salt thereof when the Ar group contains a basic nitrogen or when the Ar group is substituted by dialklyamino of 1-6 carbon atoms per alkyl group, $-SO_3H$, $-PO_3H$, or $-CO_2H$;
or of formula X,

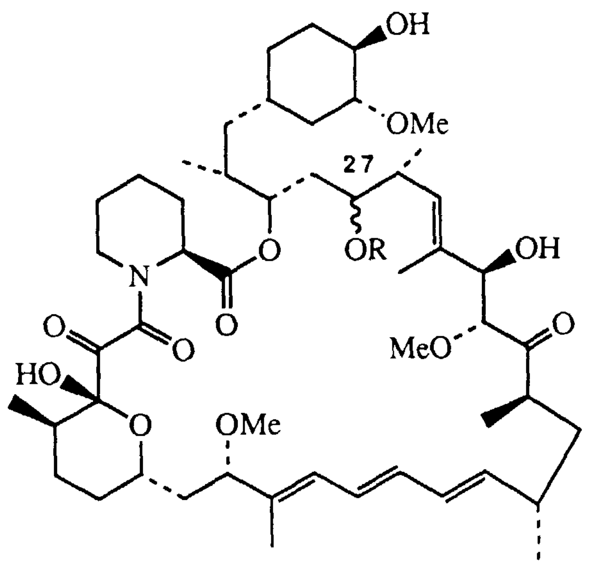

X

wherein

R is $-SO_2R^1$ ;
$R^1$ is alkyl, alkenyl, alkynyl containing 1 to 6 carbon atoms; or an aromatic moiety selected from the group consisting of phenyl and naphthyl or a heterocyclic moiety selected from the group consisting of thiophenyl and quinolinyl; or $-NHCOR^2$ ; and
$R^2$ is lower alkyl containing 1 to 6 carbon atoms;

or a pharmaceutically acceptable salt thereof,
and a pharmaceutically acceptable carrier,

**19.** A compound which is 31,42-bis-triethylsilyl ether of 27-hydroxyrapamycin.

**20.** A compound which is 31,42-bis-triethylsilyl ether of 27-hydroxyrapamycin-27-ester with acetic acid.

**Patentansprüche**

**1.** Verbindung, welche 27-Hydroxyrapamycin ist.

**2.** Verbindung der Formel

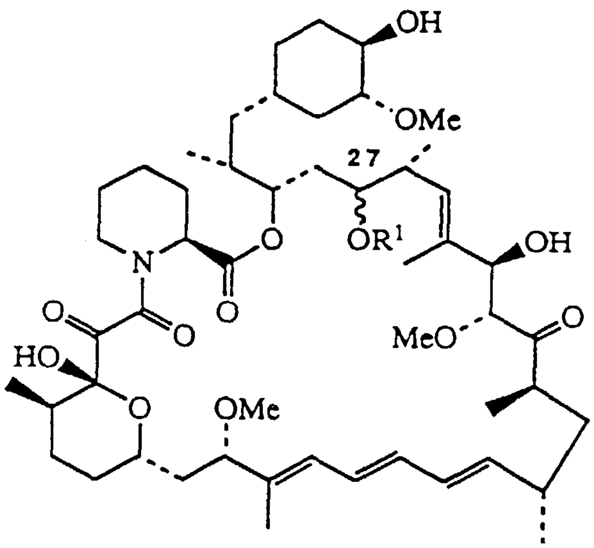

(II),

worin $R^1$ die Bedeutung

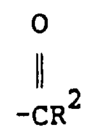

hat; und $R^2$ darstellt: Alkyl mit 1 bis 10 Kohlenstoffatomen, Arylalkyl mit 7 bis 10 Kohlenstoffatomen oder Aryl, wobei die Aryl-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; oder ein pharmazeutisch annehmbares Salz hievon.

**3.** Verbindung nach Anspruch 2, welche 27-Hydroxyrapamycin-27-ester mit Essigsäure ist.

**4.** Verbindung der Formel

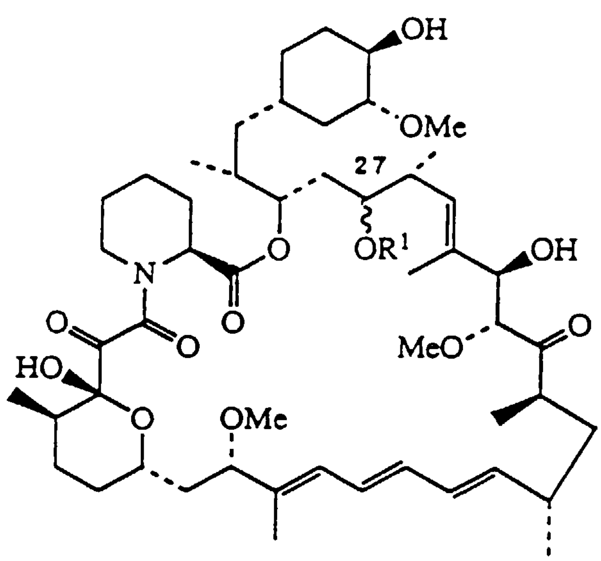

(III),

worin $R^1$ die Bedeutung

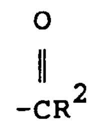

hat; und $R^2$ eine mono-, di-, poly- oder perfluorierte Alkyl-Gruppe mit 1 bis 10 Kohlenstoffatomen darstellt.

**5.** Verbindung der Formel

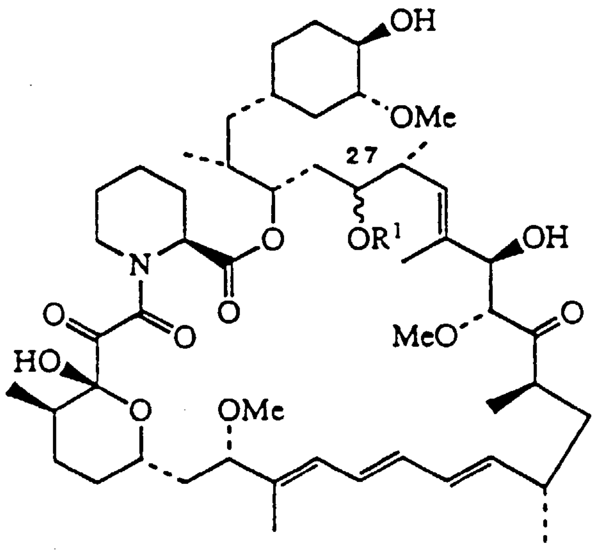

(IV),

worin $R^1$ die Bedeutung

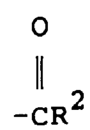

hat; $R^2$

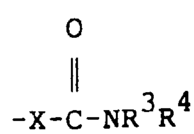

darstellt; X $-(CH_2)_m-$ oder $-Ar^1-$ ist, wobei $-Ar^1-$ eine gegebenenfalls mono-oder disubstituierte Gruppe bedeutet, ausgewählt aus:

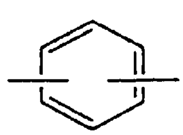

,

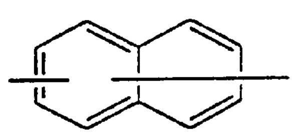

,

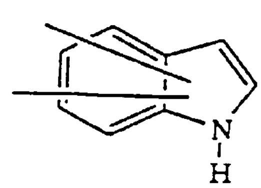

,

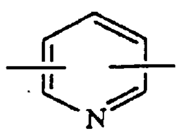 , 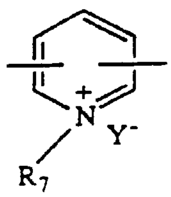 , 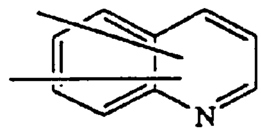 oder 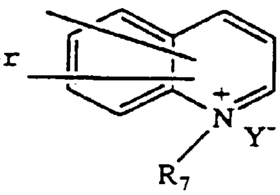 ;

$R^3$ und $R^4$ jeweils unabhängig Wasserstoff, Alkyl mit 1 bis 12 Kohlenstoffatomen, $-(CH_2)_n$-Ar, $-(CH_2)_p-NR^5R^6$ oder $-(CH_2)_p-N^+R^5R^6R^7Y^-$ sind; $R^5$ und $R^6$ jeweils unabhängig Wasserstoff, Alkyl mit 1 bis 12 Kohlenstoffatomen oder $-(CH_2)_n$-Ar darstellen; Ar eine gegebenenfalls mono- oder disubstituierte Gruppe ist, ausgewählt aus

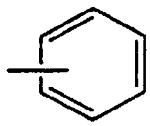 , 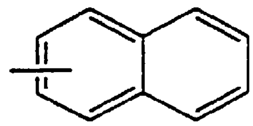 , 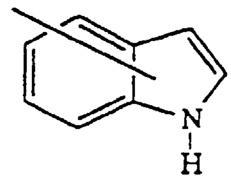 ,

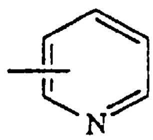 , 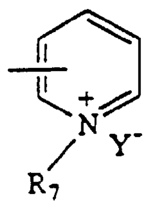 , 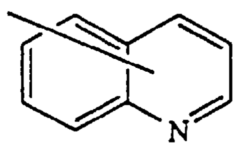 oder 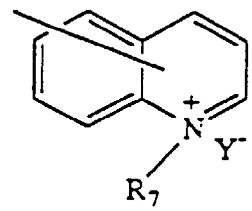 ,

wobei die gegebenenfalls vorliegenden Substituenten ausgewählt sind aus der Gruppe bestehend aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen oder Perfluoralkyl mit 1 bis 6 Kohlenstoffatomen; $R^7$ Alkyl mit 1 bis 6 Kohlenstoffatomen bedeutet; Y ein Halogenid-, Sulfat-, Phosphat- oder p-Toluolsulfonatanion darstellt; m = 1 bis 6; n = 1 bis 6; p = 1 bis 6; oder ein pharmazeutisch annehmbares Salz hievon.

**6.** Verbindung der Formel

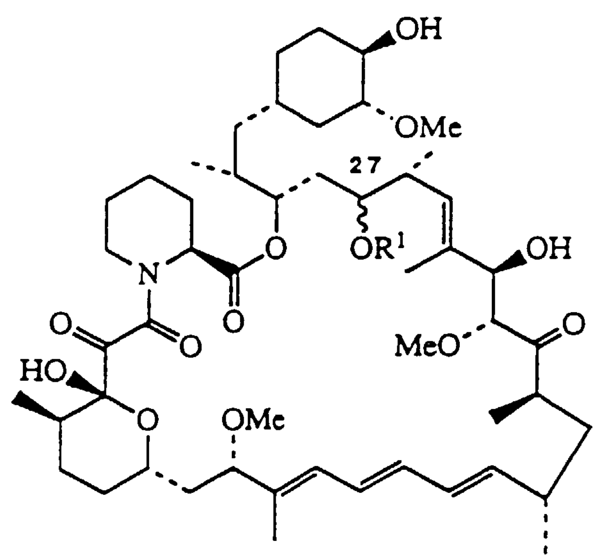

(V),

worin $R^1$ die Bedeutung

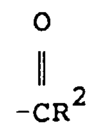

hat; $R^2$ -NH($CR^3R^4$)$_n$-X darstellt; $R^3$ und $R^4$ jeweils unabhänging Wasserstoff, Alkyl mit 1 bis 6 Kohlenstoffatomen, Arylalkyl mit 7 bis 10 Kohlenstoffatomen, Cycloalkyl mit 3 bis 8 Kohlenstoffatomen, Halogen oder Trifluormethyl sind; X Wasserstoff, nied.Alkyl mit 1 bis 6 Kohlenstoffatomen, Cycloalkyl mit 3 bis 8 Kohlenstoffatomen, Trifluormethyl, Nitro, Alkoxy mit 1 bis 6 Kohlenstoffatomen, Carbalkoxy mit 2 bis 7 Kohlenstoffatomen, Arylalkyl mit 7 bis 10 Kohlenstoffatomen, Halogen, Dialkylamino mit 1 bis 6 Kohlenstoffatomen pro Alkyl-Gruppe, Thioalkyl mit 1 bis 6 Kohlenstoffatomen oder Y bedeutet; Y darstellt: eine Phenyl-Gruppe, die gegebenenfalls mono-, di-oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Dialkylamino mit 1 bis 6 Kohlenstoffatomen pro Alkyl-Gruppe oder Alkylthio mit 1 bis 6 Kohlenstoffatomen, -$SO_3H$, -$PO_3H$ und -$CO_2H$; n = Null bis 5; mit der Maßgabe, daß, wenn n = Null, X nied.Alkyl mit 1 bis 6 Kohlenstoffatomen, Cycloalkyl mit 3 bis 8 Kohlenstoffatomen, Arylalkyl mit 7 bis 10 Kohlenstoffatomen oder Y bedeutet; oder ein pharmazeutisch annehmbares Salz hievon.

**7.** Verbindung der Formel

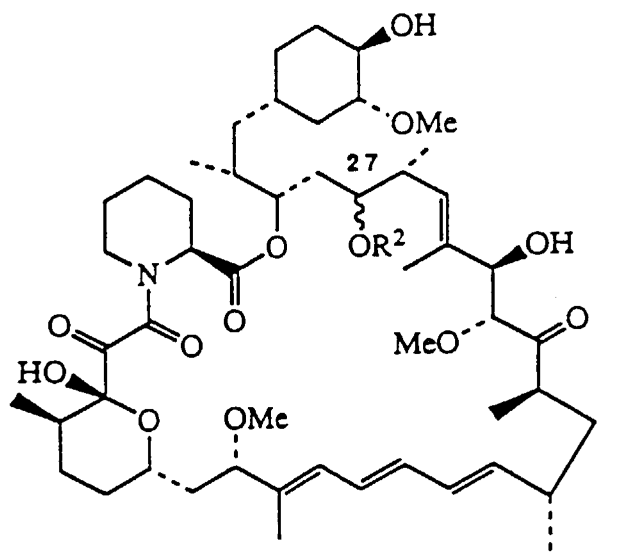

(VI),

worin $R^2$ die Bedeutung

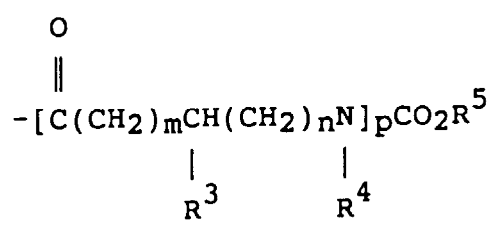

hat; $R^3$ darstellt: Wasserstoff, Alkyl mit 1 bis 6 Kohlenstoffatomen, Arylalkyl mit 7 bis 10 Kohlenstoffatomen, -($CH_2$)$_q$$CO_2R^6$,
-($CH_2$)$_r$$NR^7CO_2R^8$, Carbamoylalkyl mit 2 bis 3 Kohlenstoffatomen, Aminoalkyl mit 1 bis 4 Kohlenstoffatomen, Hydroxyalkyl mit 1 bis 4 Kohlenstoffatomen, Guanylalkyl mit 2 bis 4 Kohlenstoffatomen, Mercaptoalkyl mit 1 bis 4 Kohlenstoffatomen, Alkylthioalkyl mit 2 bis 6 Kohlenstoffatomen, Indolylmethyl, Hydroxyphenylmethyl, Imidazoylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro,

Carbalkoxy mit 2 bis 7 Kohlenstoffatomen, Trifluormethyl, Amino oder $-CO_2H$; $R^4$ und $R^7$ jeweils unabhängig Wasserstoff, Alkyl mit 1 bis 6 Kohlenstoffatomen oder Arylalkyl mit 7 bis 10 Kohlenstoffatomen sind; $R^5$, $R^6$ und $R^8$ jeweils unabhängig bedeuten: Alkyl mit 1 bis 6 Kohlenstoffatomen, Arylalkyl mit 7 bis 10 Kohlenstoffatomen, Fluorenylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro, Carbalkoxy mit 2 bis 7 Kohlenstoffatomen, Trifluormethyl, Amino oder $-CO_2H$; m Null bis 4 ist; n Null bis 4 ist; p 1 bis 2 ist; q Null bis 4 ist; r Null bis 4 ist; wobei $R^3$, $R^4$, m und n in jeder der Untereinheiten

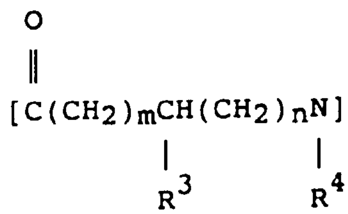

unabhängig sind, wenn p = 2; oder ein pharmazeutisch annehmbares Salz hievon.

**8.** Verbindung der Formel

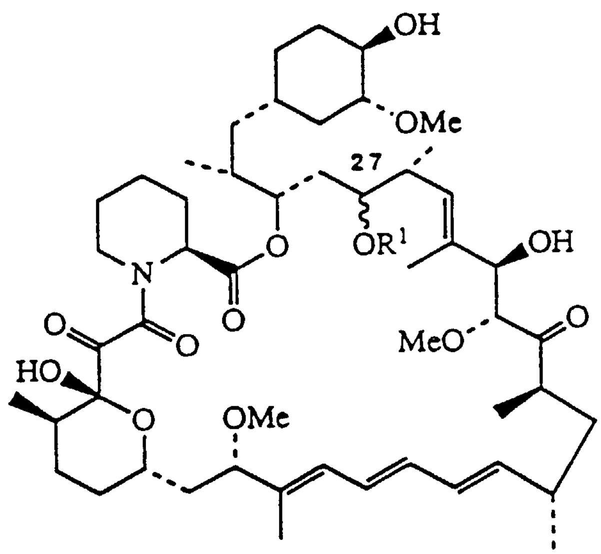

(VII),

worin $R^1$ Alkyl mit 1 bis 6 Kohlenstoffatomen, Arylalkyl mit 7 bis 10 Kohlenstoffatomen, $-CH_2YX$, $-C(CH_3)_2YX$, $-CH(CH_3)YX$ oder L bedeutet; Y 0 oder S darstellt; X $-CH_3$, $-(CH_2)_nCH_3$, $-CH_2Ar$, $-(CH_2)_2OCH_3$, $CH_2CCl_3$, $-CH(CH_3)_2$ oder $-CH_2CH_2SiMe_3$ ist; Ar bedeutet: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; L Tetrahydrofuran-2-yl, Tetrahydrothiophen-2-yl, Tetrahydrothiopyran-2-yl, Tetrahydropyran-2-yl, 4-Methoxytetrahydropyran-2-yl, 4-Methoxytetrahydrothiopyran-2-yl oder 4-Methoxytetrahydrothiopyran-2-yl-S,S-dioxid darstellt; und n = 1 bis 5.

**9.** Verbindung der Formel

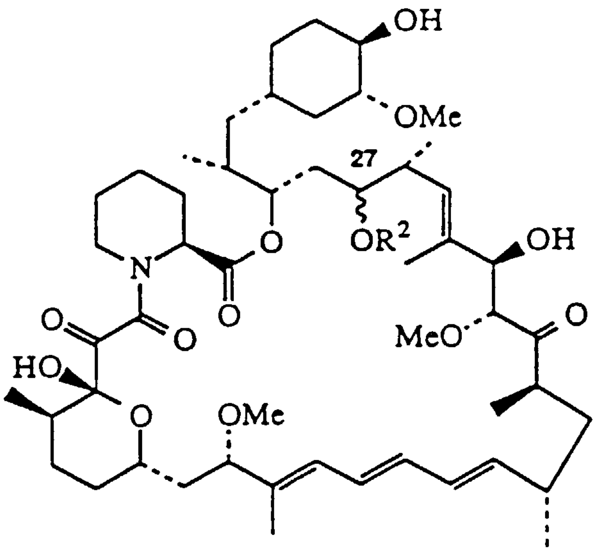

(VIII),

worin $R^2$ die Bedeutung

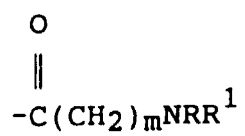

hat; R und $R^1$ jeweils Wasserstoff oder Alkyl mit 1 bis 3 Kohlenstoffatomen bedeuten, oder R und $R^1$ zusammen mit dem Stickstoff, an den sie gebunden sind, einen gesättigten heterocyclischen Ring mit 4 bis 5 Kohlenstoffatomen bilden; und m = 1 bis 3; oder ein pharmazeutisch annehmbares Salz hievon.

**10.** Verbindung der Formel

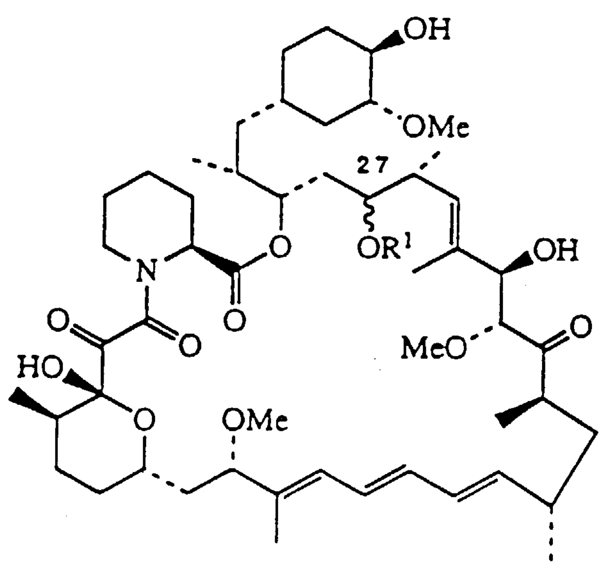

(IX),

worin $R^1$ die Bedeutung $-CONHSO_2-Ar$ hat; und Ar darstellt: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino

mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; oder ein pharmazeutisch annehmbares Salz hievon, wenn die Ar-Gruppe einen basischen Stickstoff enthält, oder wenn die Ar-Gruppe substituiert ist durch Dialkylamino mit 1 bis 6 Kohlenstoffatomen pro Alkyl-Gruppe, $-SO_3H$, $-PO_3H$ oder $-CO_2H$.

**11.** Verbindung der Formel

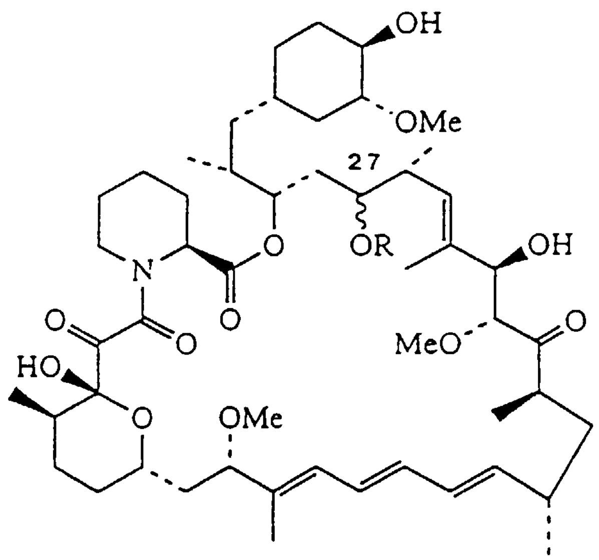

(X),

worin R die Bedeutung $-SO_2R^1$ hat; $R^1$ darstellt: Alkyl, Alkenyl, Alkinyl mit 1 bis 6 Kohlenstoffatomen; oder eine aromatische Gruppe, ausgewählt aus der Gruppe bestehend aus Phenyl und Naphthyl, oder eine heterocyclische Gruppe, ausgewählt aus der Gruppe bestehend aus Thiophenyl und Chinolinyl; oder $-NHCOR^2$; und $R^2$ nied. Alkyl mit 1 bis 6 Kohlenstoffatomen ist; oder ein pharmazeutisch annehmbares Salz hievon.

**12.** Verwendung von 27-Hydroxyrapamycin bei der Herstellung eines Medikaments zur Verwendung bei der Induktion einer Immunsuppression.

**13.** Verwendung nach Anspruch 12, wobei die induzierte Immunsuppression zur Prävention oder Behandlung von einer Transplantationsabstoßung oder Abstoßungsreaktionen verwendet wird.

**14.** Verwendung nach Anspruch 12, wobei die induzierte Immunsuppression zur Behandlung von Autoimmunkrankheiten, entzündlichen Erkrankungen oder hyperproliferativen Gefäßstörungen verwendet wird.

**15.** Verwendung einer Verbindung der Formel (II)

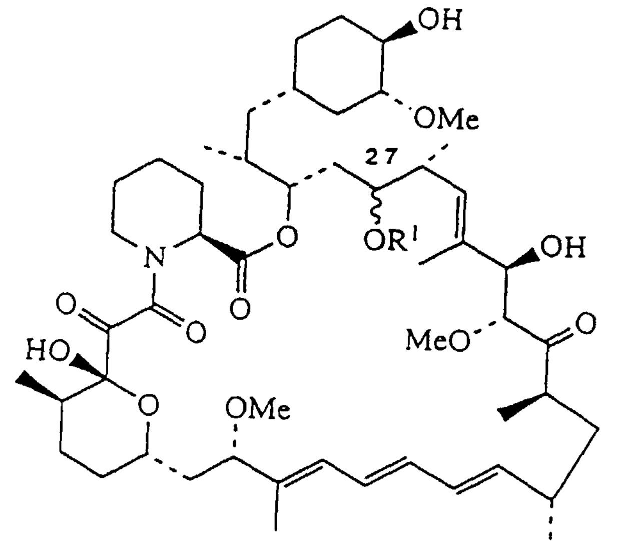

(II),

worin $R^1$ die Bedeutung

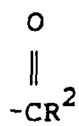

hat; und $R^2$ darstellt: Alkyl mit 1 bis 10 Kohlenstoffatomen, Arylalkyl mit 7 bis 10 Kohlenstoffatomen oder Aryl, wobei die Aryl-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; oder eines pharmazeutisch annehmbaren Salzes hievon; oder der Formel (III)

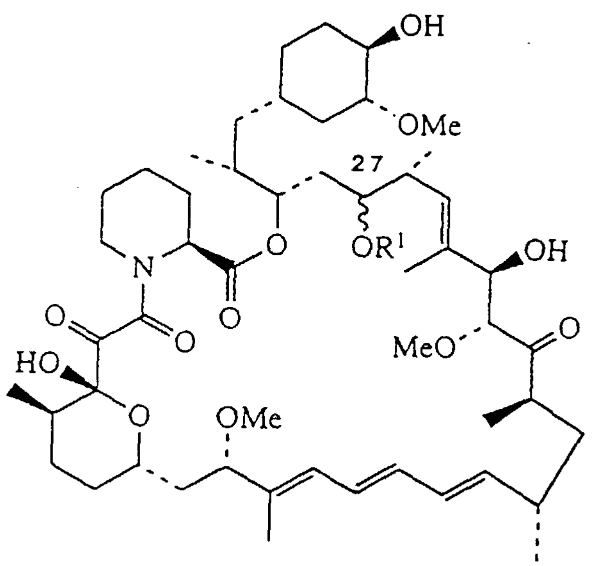

(III),

worin $R^1$ die Bedeutung

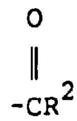

hat; und $R^2$ eine mono-, di-, poly- oder perfluorierte Alkyl-Gruppe mit 1 bis 10 Kohlenstoffatomen darstellt; oder der Formel (IV)

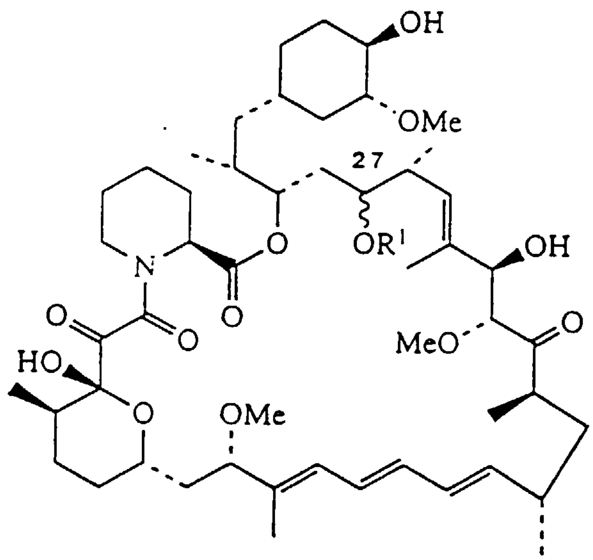

(IV),

worin $R^1$ die Bedeutung

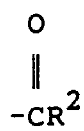

hat; $R^2$

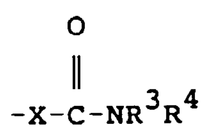

darstellt; X $-(CH_2)_m-$ oder $-Ar^1-$ ist, wobei $-Ar^1-$ eine gegebenenfalls mono-oder disubstituierte Gruppe bedeutet, ausgewählt aus:

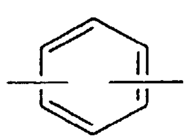 , 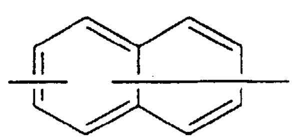 , 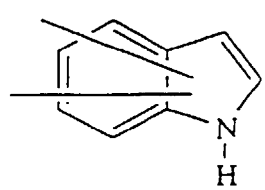 ,

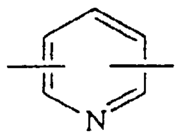 , 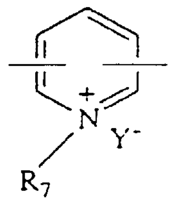 , 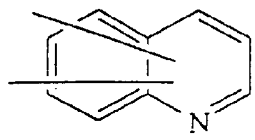 oder 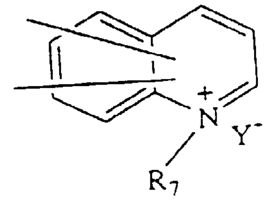 ;

$R^3$ und $R^4$ jeweils unabhängig Wasserstoff, Alkyl mit 1 bis 12 Kohlenstoffatomen, $-(CH_2)_n$-Ar, $-(CH_2)_p-NR^5R^6$ oder $-(CH_2)_p-N^+R^5R^6R^7Y^-$ sind; $R^5$ und $R^6$ jeweils unabhängig Wasserstoff, Alkyl mit 1 bis 12 Kohlenstoffatomen oder $-(CH_2)_n$-Ar darstellen; Ar eine gegebenenfalls mono- oder disubstituierte Gruppe ist, ausgewählt aus

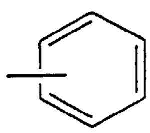 , 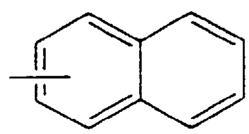 , 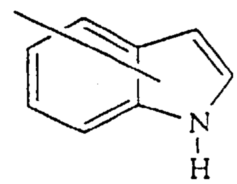 ,

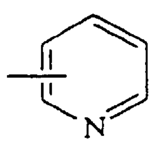 , 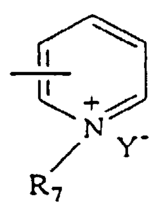 , 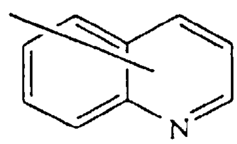 oder 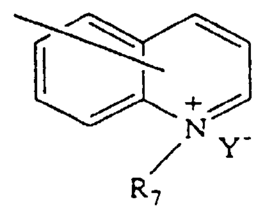 ,

wobei die gegebenenfalls vorliegenden Substituenten ausgewählt sind aus der Gruppe bestehend aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen oder Perfluoralkyl mit 1 bis 6 Kohlenstoffatomen; $R^7$ Alkyl mit 1 bis 6 Kohlenstoffatomen bedeutet; Y ein Halogenid-, Sulfat-, Phosphat- oder p-Toluolsulfonatanion darstellt; m = 1 bis 6; n = 1 bis 6; p = 1 bis 6; oder eines pharmazeutisch annehmbaren Salzes hievon; oder der Formel (V)

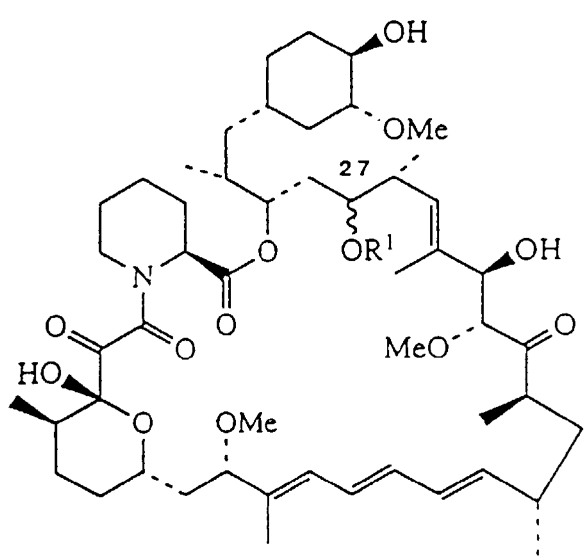

(V),

worin $R^1$ die Bedeutung

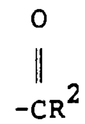

hat; $R^2$ -NH$(CR^3R^4)_n$-X darstellt; $R^3$ und $R^4$ jeweils unabhänging Wasserstoff, Alkyl mit 1 bis 6 Kohlenstoffatomen, Arylalkyl mit 7 bis 10 Kohlenstoffatomen, Cycloalkyl mit 3 bis 8 Kohlenstoffatomen, Halogen oder Trifluormethyl sind; X Wasserstoff, nied.Alkyl mit 1 bis 6 Kohlenstoffatomen, Cycloalkyl mit 3 bis 8 Kohlenstoffatomen, Trifluormethyl, Nitro, Alkoxy mit 1 bis 6 Kohlenstoffatomen, Carbalkoxy mit 2 bis 7 Kohlenstoffatomen, Arylalkyl mit 7 bis 10 Kohlenstoffatomen, Halogen, Dialkylamino mit 1 bis 6 Kohlenstoffatomen pro Alkyl-Gruppe, Thioalkyl mit 1 bis 6 Kohlenstoffatomen oder Y bedeutet; Y darstellt: eine Phenyl-Gruppe, die gegebenenfalls mono-, di-oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Dialkylamino mit 1 bis 6 Kohlenstoffatomen pro Alkyl-Gruppe oder Alkylthio mit 1 bis 6 Kohlenstoffatomen, $-SO_3H$, $-PO_3H$ und $-CO_2H$; n = Null bis 5; mit der Maßgabe, daß, wenn n = Null, X nied.Alkyl mit 1 bis 6 Kohlenstoffatomen, Cycloalkyl mit 3 bis 8 Kohlenstoffatomen, Arylalkyl mit 7 bis 10 Kohlenstoffatomen oder Y bedeutet; oder eines pharmazeutisch annehmbaren Salzes hievon; oder der Formel (VI)

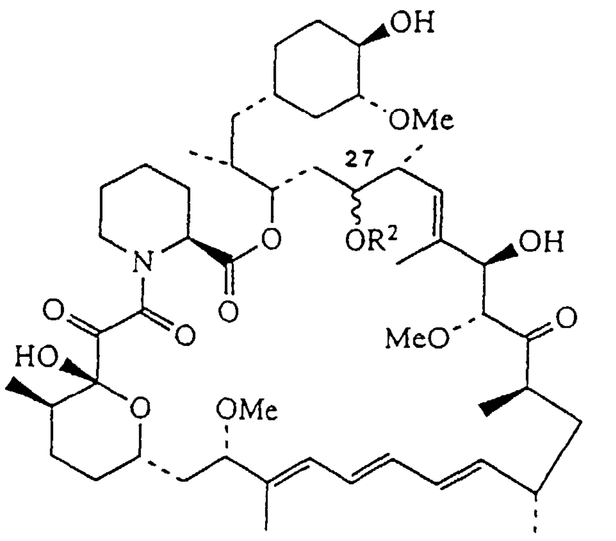

(VI),

worin $R^2$ die Bedeutung

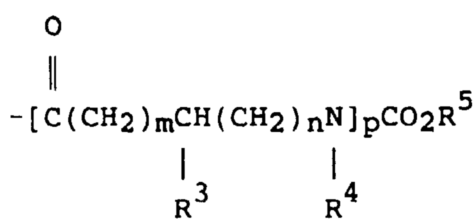

hat; $R^3$ darstellt: Wasserstoff, Alkyl mit 1 bis 6 Kohlenstoffatomen, Arylalkyl mit 7 bis 10 Kohlenstoffatomen, -$(CH_2)_qCO_2R^6$,
-$(CH_2)_rNR^7CO_2R^8$, Carbamoylalkyl mit 2 bis 3 Kohlenstoffatomen, Aminoalkyl mit 1 bis 4 Kohlenstoffatomen, Hydroxyalkyl mit 1 bis 4 Kohlenstoffatomen, Guanylalkyl mit 2 bis 4 Kohlenstoffatomen, Mercaptoalkyl mit 1 bis 4 Kohlenstoffatomen, Alkylthioalkyl mit 2 bis 6 Kohlenstoffatomen, Indolylmethyl, Hydroxyphenylmethyl, Imidazoylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro, Carbalkoxy mit 2 bis 7 Kohlenstoffatomen, Trifluormethyl, Amino oder $-CO_2H$; $R^4$ und $R^7$ jeweils unabhängig Wasserstoff, Alkyl mit 1 bis 6 Kohlenstoffatomen oder Arylalkyl mit 7 bis 10 Kohlenstoffatomen sind; $R^5$, $R^6$ und $R^8$ jeweils unabhängig bedeuten: Alkyl mit 1 bis 6 Kohlenstoffatomen, Arylalkyl mit 7 bis 10 Kohlenstoffatomen, Fluorenylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro, Carbalkoxy mit 2 bis 7 Kohlenstoffatomen, Trifluormethyl, Amino oder $-CO_2H$; m Null bis 4 ist; n Null bis 4 ist; p 1 bis 2 ist; q Null bis 4 ist; r Null bis 4 ist; wobei $R^3$, $R^4$, m und n in jeder der Untereinheiten

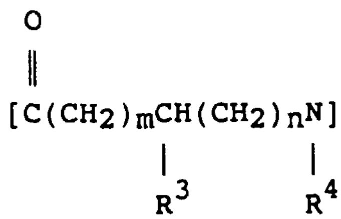

unabhängig sind, wenn p = 2; oder eines pharmazeutisch annehmbaren Salzes hievon; oder der Formel (VII)

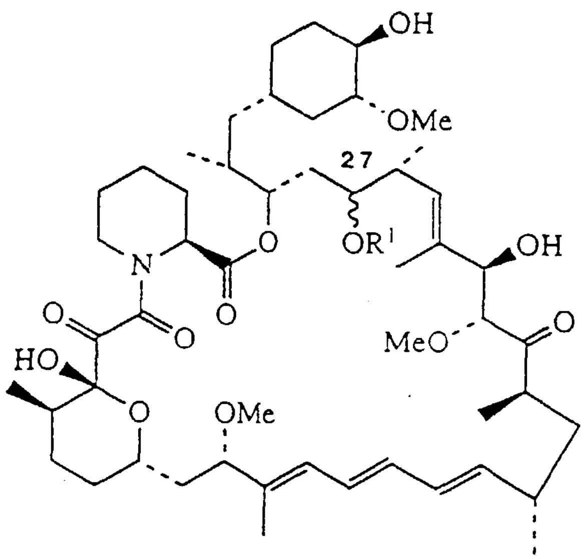

(VII),

worin $R^1$ Alkyl mit 1 bis 6 Kohlenstoffatomen, Arylalkyl mit 7 bis 10 Kohlenstoffatomen, $-CH_2YX$, $-C(CH_3)_2YX$, $-CH(CH_3)YX$ oder L bedeutet; Y 0 oder S darstellt; X $-CH_3$, $-(CH_2)_nCH_3$, $-CH_2Ar$, $-(CH_2)_2OCH_3$, $CH_2CCl_3$, $-CH(CH_3)_2$ oder $-CH_2CH_2SiMe_3$ ist; Ar bedeutet: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; L Tetrahydrofuran-2-yl, Tetrahydrothiophen-2-yl, Tetrahydrothiopyran-2-yl, Tetrahydropyran-2-yl, 4-Methoxytetrahydropyran-2-yl, 4-Methoxytetrahydrothiopyran-2-yl oder 4-Methoxytetrahydrothiopyran-2-yl-S,S-dioxid darstellt; und n = 1 bis 5; oder der Formel (VIII)

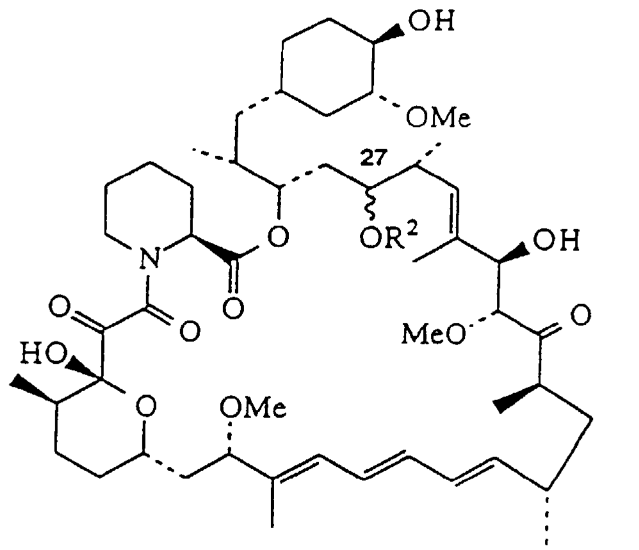

(VIII),

worin $R^1$ die Bedeutung

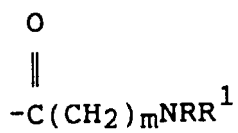

hat; R und $R^1$ jeweils Wasserstoff oder Alkyl mit 1 bis 3 Kohlenstoffatomen bedeuten, oder R und $R^1$ zusammen mit dem Stickstoff, an den sie gebunden sind, einen gesättigten heterocyclischen Ring mit 4 bis 5 Kohlenstoffatomen bilden; und m = 1 bis 3; oder eines pharmazeutisch annehmbaren Salzes hievon; oder der Formel (IX)

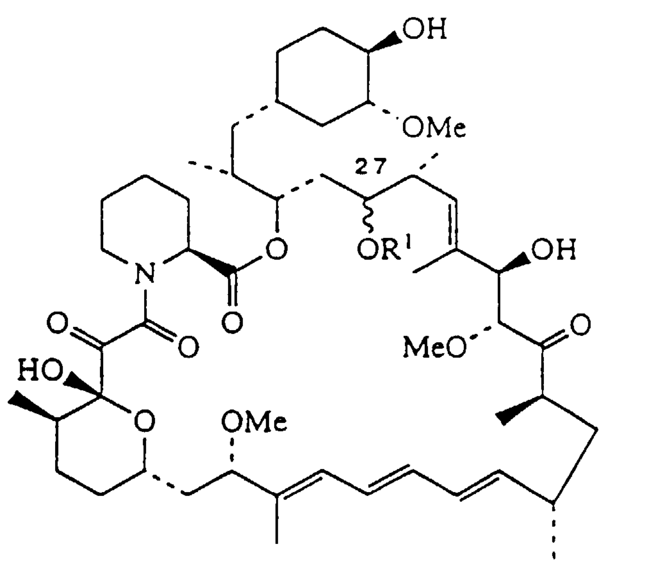

(IX),

worin $R^1$ die Bedeutung $-CONHSO_2$-Ar hat; und Ar darstellt: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$;

oder eines pharmazeutisch annehmbaren Salzes hievon, wenn die Ar-Gruppe einen basischen Stickstoff enthält, oder wenn die Ar-Gruppe substituiert ist durch Dialkylamino mit 1 bis 6 Kohlenstoffatomen pro Alkyl-Gruppe, $-SO_3H$, $-PO_3H$ oder $-CO_2H$; oder der Formel (X)

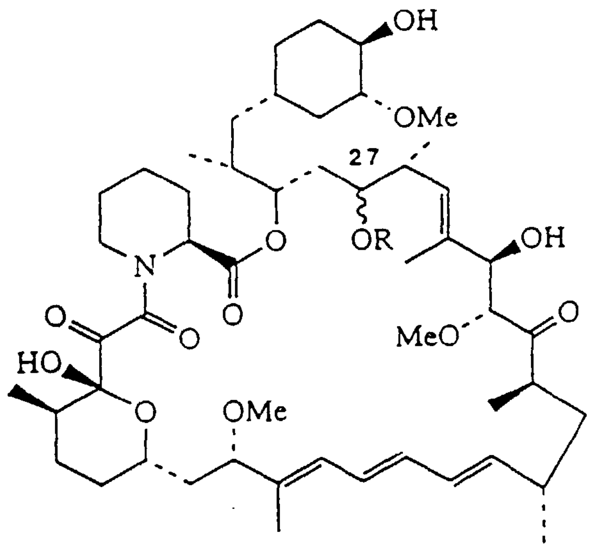

(X),

worin R die Bedeutung $-SO_2R^1$ hat; $R^1$ darstellt: Alkyl, Alkenyl, Alkinyl mit 1 bis 6 Kohlenstoffatomen; oder eine aromatische Gruppe, ausgewählt aus der Gruppe bestehend aus Phenyl und Naphthyl, oder eine heterocyclische Gruppe, ausgewählt aus der Gruppe bestehend aus Thiophenyl und Chinolinyl; oder $-NHCOR^2$; und $R^2$ nied. Alkyl mit 1 bis 6 Kohlenstoffatomen ist; oder eines pharmazeutisch annehmbaren Salzes hievon, bei der Herstellung eines Medikaments zur Induktion einer Immunsuppression.

**16.** Pharmazeutische Zusammensetzung, welche eine wirksame Menge von 27-Hydroxyrapamycin und einen pharmazeutisch annehmbaren Träger umfaßt.

**17.** Pharmazeutische Zusammensetzung, welche eine wirksame Menge einer Kombination von 27-Hydroxyrapamycin und einem Anti-Abstoßungs-Chemotherapeutikum, ausgewählt aus der Gruppe bestehend aus Azathioprin, Corticosteroiden, Cyclophosphamid, Rapamycin, Cyclosporin A, FK-506, OKT-3 und ATG, und einen pharmazeutisch annehmbaren Träger umfaßt.

**18.** Pharmazeutische Zusammensetzung, welche umfaßt: eine wirksame Menge einer Verbindung der Formel (II)

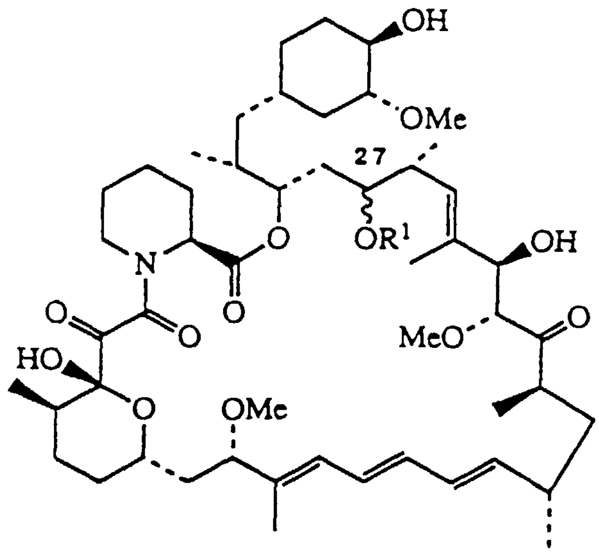

(II),

worin $R^1$ die Bedeutung

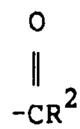

hat; und $R^2$ darstellt: Alkyl mit 1 bis 10 Kohlenstoffatomen, Arylalkyl mit 7 bis 10 Kohlenstoffatomen oder Aryl, wobei die Aryl-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; oder eines pharmazeutisch annehmbaren Salzes hievon; oder der Formel (III)

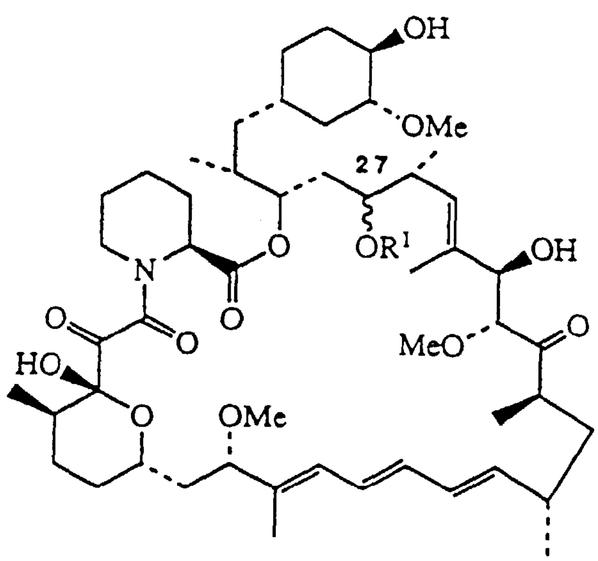

(III),

worin $R^1$ die Bedeutung

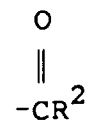

hat; und $R^2$ eine mono-, di-, poly- oder perfluorierte Alkyl-Gruppe mit 1 bis 10 Kohlenstoffatomen darstellt; oder der Formel (IV)

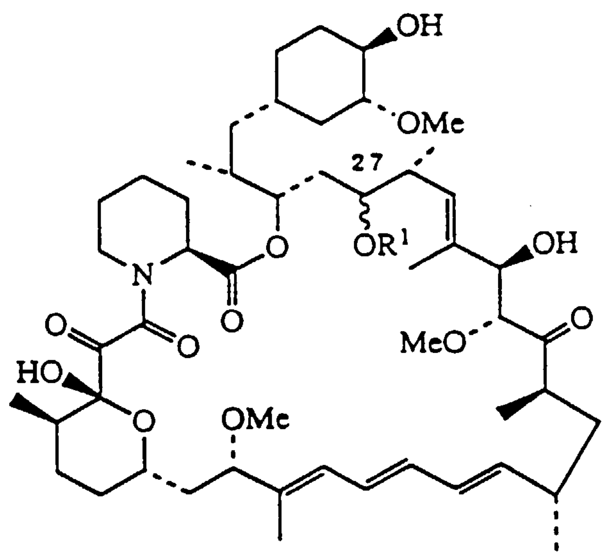

(IV),

worin $R^1$ die Bedeutung

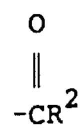

hat; $R^2$

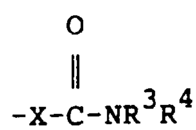

darstellt; X $-(CH_2)_m-$ oder $-Ar^1-$ ist, wobei $-Ar^1-$ eine gegebenenfalls mono-oder disubstituierte Gruppe bedeutet, ausgewählt aus:

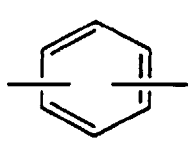

,

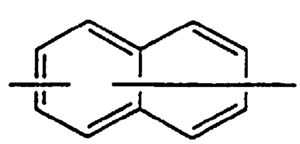

,

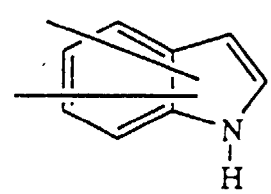

,

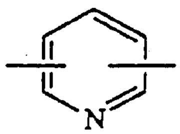 , 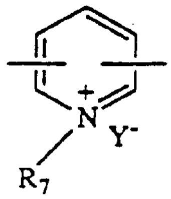 , 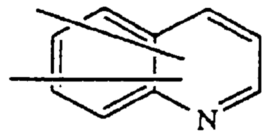 oder 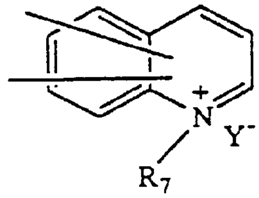 ;

$R^3$ und $R^4$ jeweils unabhängig Wasserstoff, Alkyl mit 1 bis 12 Kohlenstoffatomen, $-(CH_2)_n$-Ar, $-(CH_2)_p-NR^5R^6$ oder $-(CH_2)_p-N^+R^5R^6R^7Y^-$ sind; $R^5$ und $R^6$ jeweils unabhängig Wasserstoff, Alkyl mit 1 bis 12 Kohlenstoffatomen oder $-(CH_2)_n$-Ar darstellen; Ar eine gegebenenfalls mono- oder disubstituierte Gruppe ist, ausgewählt aus

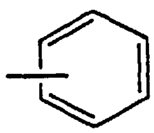 , 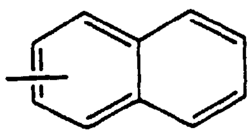 , 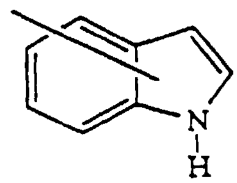 ,

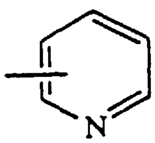 , 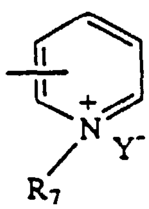 , 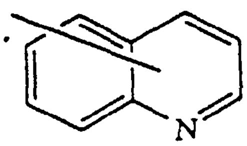 oder 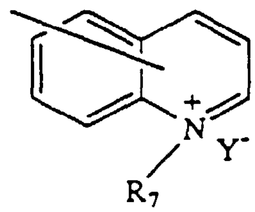 ,

wobei die gegebenenfalls vorliegenden Substituenten ausgewählt sind aus der Gruppe bestehend aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen oder Perfluoralkyl mit 1 bis 6 Kohlenstoffatomen; $R^7$ Alkyl mit 1 bis 6 Kohlenstoffatomen bedeutet; Y ein Halogenid-, Sulfat-, Phosphat- oder p-Toluolsulfonatanion darstellt; m = 1 bis 6; n = 1 bis 6; p = 1 bis 6; oder eines pharmazeutisch annehmbaren Salzes hievon; oder der Formel (V)

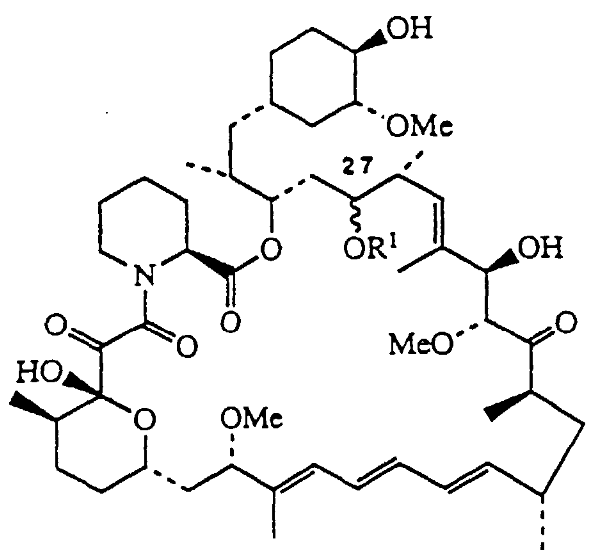

(V),

worin $R^1$ die Bedeutung

$$-\overset{O}{\overset{\|}{C}}R^2$$

hat; $R^2$ $-NH(CR^3R^4)_n-X$ darstellt; $R^3$ und $R^4$ jeweils unabhänging Wasserstoff, Alkyl mit 1 bis 6 Kohlenstoffatomen, Arylalkyl mit 7 bis 10 Kohlenstoffatomen, Cycloalkyl mit 3 bis 8 Kohlenstoffatomen, Halogen oder Trifluormethyl sind; X Wasserstoff, nied.Alkyl mit 1 bis 6 Kohlenstoffatomen, Cycloalkyl mit 3 bis 8 Kohlenstoffatomen, Trifluormethyl, Nitro, Alkoxy mit 1 bis 6 Kohlenstoffatomen, Carbalkoxy mit 2 bis 7 Kohlenstoffatomen, Arylalkyl mit 7 bis 10 Kohlenstoffatomen, Halogen, Dialkylamino mit 1 bis 6 Kohlenstoffatomen pro Alkyl-Gruppe, Thioalkyl mit 1 bis 6 Kohlenstoffatomen oder Y bedeutet; Y darstellt: eine Phenyl-Gruppe, die gegebenenfalls mono-, di-oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Dialkylamino mit 1 bis 6 Kohlenstoffatomen pro Alkyl-Gruppe oder Alkylthio mit 1 bis 6 Kohlenstoffatomen, $-SO_3H$, $-PO_3H$ und $-CO_2H$; n = Null bis 5; mit der Maßgabe, daß, wenn n = Null, X nied.Alkyl mit 1 bis 6 Kohlenstoffatomen, Cycloalkyl mit 3 bis 8 Kohlenstoffatomen, Arylalkyl mit 7 bis 10 Kohlenstoffatomen oder Y bedeutet; oder eines pharmazeutisch annehmbaren Salzes hievon; oder der Formel (VI)

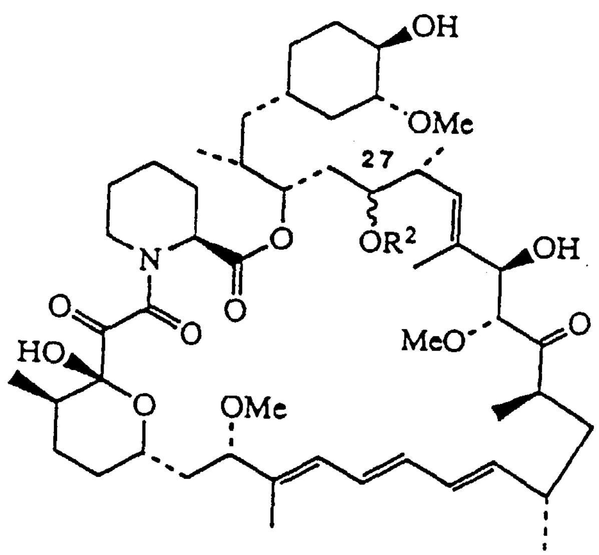

(VI),

worin $R^2$ die Bedeutung

$$-[\overset{O}{\overset{\|}{C}}(CH_2)_m\underset{R^3}{\underset{|}{CH}}(CH_2)_n\underset{R^4}{\underset{|}{N}}]_pCO_2R^5$$

hat; $R^3$ darstellt: Wasserstoff, Alkyl mit 1 bis 6 Kohlenstoffatomen, Arylalkyl mit 7 bis 10 Kohlenstoffatomen, $-(CH_2)_qCO_2R^6$, $-(CH_2)_rNR^7CO_2R^8$, Carbamoylalkyl mit 2 bis 3 Kohlenstoffatomen, Aminoalkyl mit 1 bis 4 Kohlenstoffatomen, Hydroxyalkyl mit 1 bis 4 Kohlenstoffatomen, Guanylalkyl mit 2 bis 4 Kohlenstoffatomen, Mercaptoalkyl mit 1 bis 4 Kohlenstoffatomen, Alkylthioalkyl mit 2 bis 6 Kohlenstoffatomen, Indolylmethyl, Hydroxyphenylmethyl, Imidazoylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro, Carbalkoxy mit 2 bis 7 Kohlenstoffatomen, Trifluormethyl, Amino oder $-CO_2H$; $R^4$ und $R^7$ jeweils unabhängig Wasserstoff, Alkyl mit 1 bis 6 Kohlenstoffatomen oder Arylalkyl mit 7 bis 10 Kohlenstoffatomen sind; $R^5$, $R^6$ und

$R^8$ jeweils unabhängig bedeuten: Alkyl mit 1 bis 6 Kohlenstoffatomen, Arylalkyl mit 7 bis 10 Kohlenstoffatomen, Fluorenylmethyl oder Phenyl, das gegebenenfalls mono-, di- oder trisubstituiert ist mit einem Substituenten, ausgewählt aus Alkyl mit 1 bis 6 Kohlenstoffatomen, Alkoxy mit 1 bis 6 Kohlenstoffatomen, Hydroxy, Cyano, Halogen, Nitro, Carbalkoxy mit 2 bis 7 Kohlenstoffatomen, Trifluormethyl, Amino oder $-CO_2H$; m Null bis 4 ist; n Null bis 4 ist; p 1 bis 2 ist; q Null bis 4 ist; r Null bis 4 ist; wobei $R^3$, $R^4$, m und n in jeder der Untereinheiten

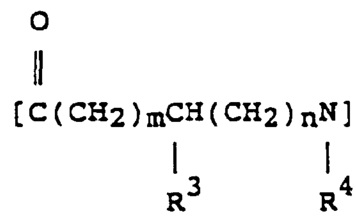

unabhängig sind, wenn p = 2; oder eines pharmazeutisch annehmbaren Salzes hievon; oder der Formel (VII)

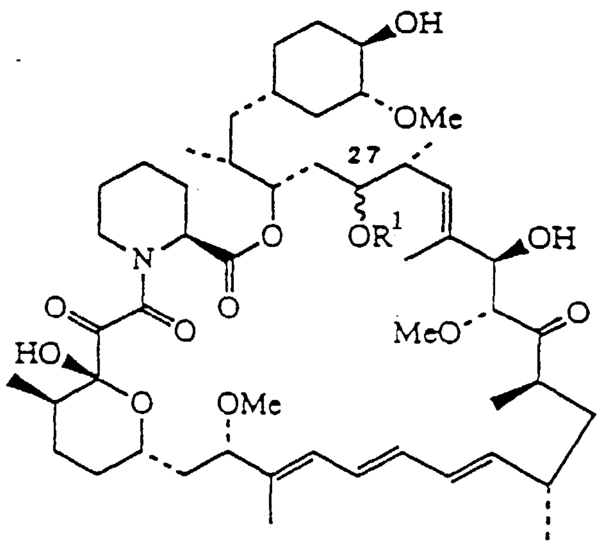

(VII),

worin $R^1$ Alkyl mit 1 bis 6 Kohlenstoffatomen, Arylalkyl mit 7 bis 10 Kohlenstoffatomen, $-CH_2YX$, $-C(CH_3)_2YX$, $-CH(CH_3)YX$ oder L bedeutet; Y O oder S darstellt; X $-CH_3$, $-(CH_2)_nCH_3$, $-CH_2Ar$, $-(CH_2)_2OCH_3$, $CH_2CCl_3$, $-CH(CH_3)_2$ oder $-CH_2CH_2SiMe_3$ ist; Ar bedeutet: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$; L Tetrahydrofuran-2-yl, Tetrahydrothiophen-2-yl, Tetrahydrothiopyran-2-yl, Tetrahydropyran-2-yl, 4-Methoxytetrahydropyran-2-yl, 4-Methoxytetrahydrothiopyran-2-yl oder 4-Methoxytetrahydrothiopyran-2-yl-S,S-dioxid darstellt; und n = 1 bis 5; oder der Formel (VIII)

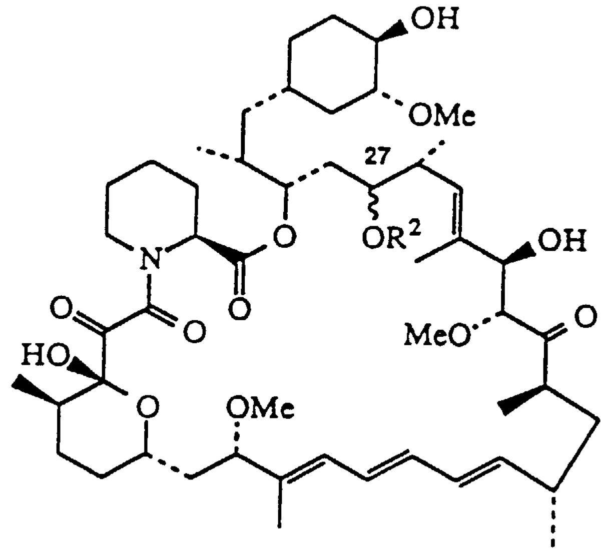

(VIII),

worin $R^1$ die Bedeutung

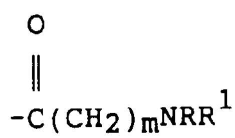

hat; R und $R^1$ jeweils Wasserstoff oder Alkyl mit 1 bis 3 Kohlenstoffatomen bedeuten, oder R und $R^1$ zusammen mit dem Stickstoff, an den sie gebunden sind, einen gesättigten heterocyclischen Ring mit 4 bis 5 Kohlenstoffatomen bilden; und m = 1 bis 3; oder eines pharmazeutisch annehmbaren Salzes hievon; oder der Formel (IX)

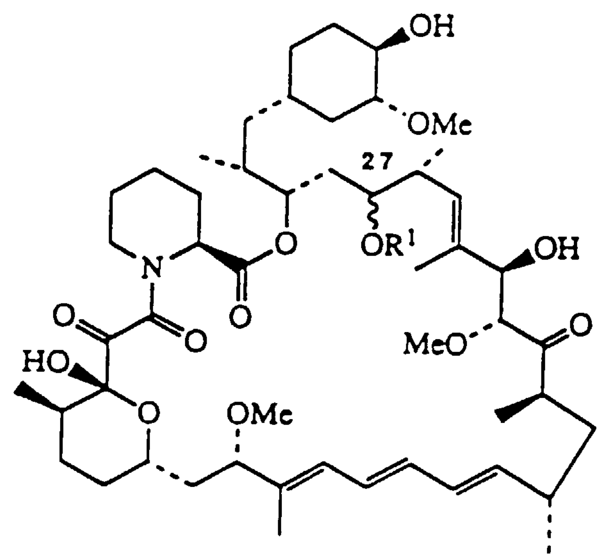

(IX),

worin $R^1$ die Bedeutung $-CONHSO_2$-Ar hat; und Ar darstellt: Phenyl, Naphthyl, Pyridyl, Chinolyl, Isochinolyl, Chinoxalyl, Thienyl, Thionaphthyl, Furyl, Benzofuryl, Benzodioxyl, Benzoxazolyl, Benzoisoxazolyl oder Benzodioxolyl, wobei die Ar-Gruppe gegebenenfalls mono-, di- oder trisubstituiert sein kann mit einer Gruppe, ausgewählt aus Alkyl mit 1 bis 6 Kohlenstoffatomen, Arylalkyl mit 7 bis 10 Kohlenstoffatomen, Alkoxy mit 1 bis 6 Kohlenstoffatomen, Cyano, Halogen, Nitro, Carbalkoxy mit 2 bis 7 Kohlenstoffatomen, Trifluormethyl, Amino, Dialkylamino mit 1 bis 6 Kohlenstoffatomen pro Alkyl-Gruppe, Alkylthio mit 1 bis 6 Kohlenstoffatomen, $-SO_3H$, $-PO_3H$ und $-CO_2H$;

oder eines pharmazeutisch annehmbaren Salzes hievon, wenn die Ar-Gruppe einen basischen Stickstoff enthält, oder wenn die Ar-Gruppe substituiert ist durch Dialkylamino mit 1 bis 6 Kohlenstoffatomen pro Alkyl-Gruppe, $-SO_3H$, $-PO_3H$ oder $-CO_2H$; oder der Formel (X)

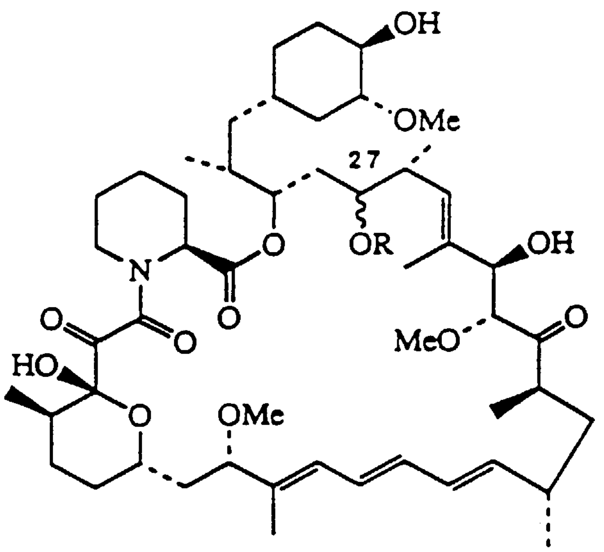

(X),

worin R die Bedeutung $-SO_2R^1$ hat; $R^1$ darstellt: Alkyl, Alkenyl, Alkinyl mit 1 bis 6 Kohlenstoffatomen; oder eine aromatische Gruppe, ausgewählt aus der Gruppe bestehend aus Phenyl und Naphthyl, oder eine heterocyclische Gruppe, ausgewählt aus der Gruppe bestehend aus Thiophenyl und Chinolinyl; oder $-NHCOR^2$; und $R^2$ nied. Alkyl mit 1 bis 6 Kohlenstoffatomen ist; oder eines pharmazeutisch annehmbaren Salzes hievon, und einen pharmazeutisch annehmbaren Träger.

**19.** Verbindung, welche 31,42-Bistriethylsilylether von 27-Hydroxyrapamycin ist.

**20.** Verbindung, welche 31,42-Bistriethylsilylether von 27-Hydroxyrapamycin-27-ester mit Essigsäure ist.

**Revendications**

**1.** Composé qui est la 27-hydroxyrapamycine.

**2.** Composé de formule :

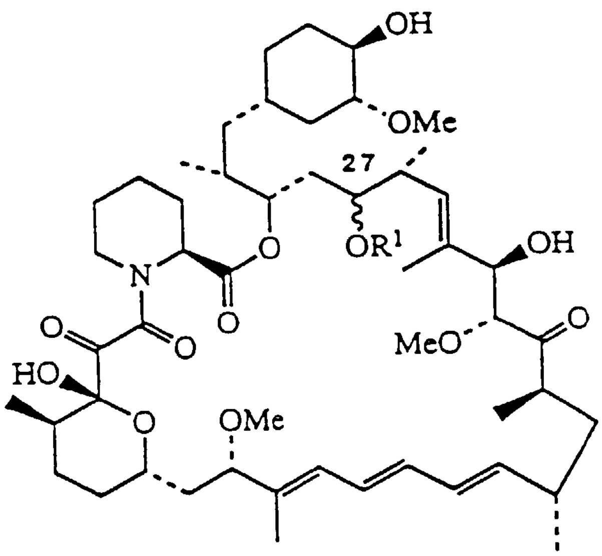

(II)

$R^1$ est

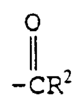

et

$R^2$ est alcoyle avec 1 à 10 atomes de carbone, arylalcoyle avec 7 à 10 atomes de carbone ou aryle dans lequel le radical aryle peut être facultativement mono-, di-ou trisubstitué au moyen d'un groupe choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;

ou un sel pharmaceutiquement acceptable de ceux-ci.

**3.** Composé suivant la revendication 2, qui est l'ester en 27 avec l'acide acétique de la 27-hydroxyrapamycine.

**4.** Composé de formule :

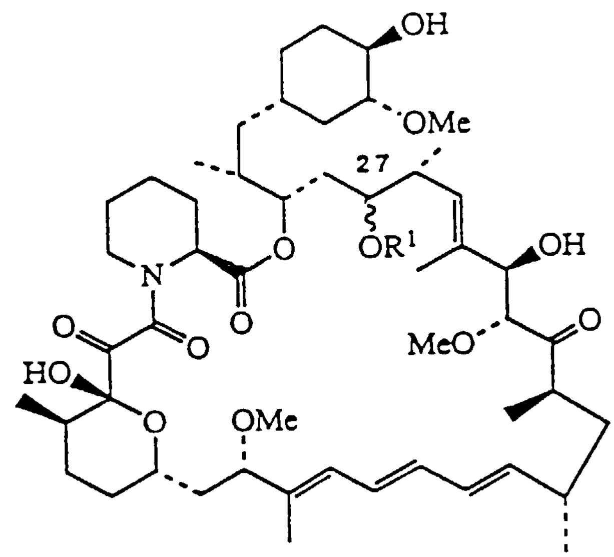

(III)

où

$R^1$ est

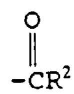

et

$R^2$ est un radical alcoyle mono-, di-, poly- ou perfluoré avec 1 à 10 atomes de carbone.

**5.** Composé de formule :

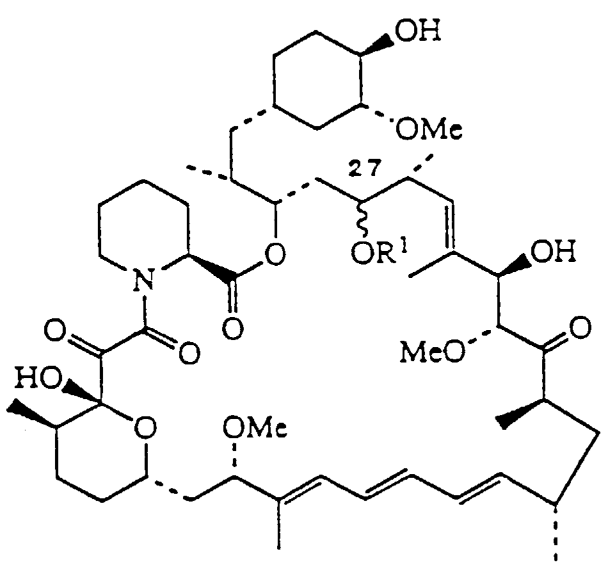

(IV)

où

$R^1$ est

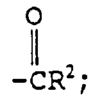

$R^2$ est

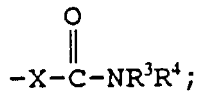

X est $-(CH_2)_m-$ ou $-Ar^1-$; où $-Ar^1-$ est un radical facultativement mono- ou disubstitué choisi parmi :

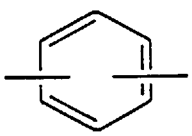 , 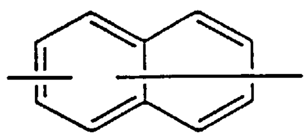 , 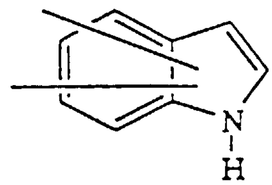 ,

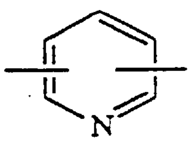 , 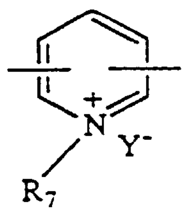 , 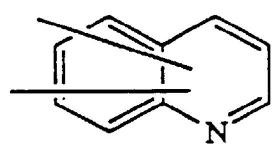 ou 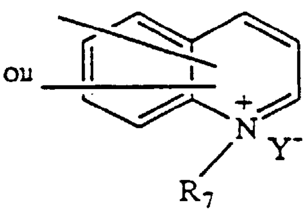

$R^3$ et $R^4$ sont chacun indépendamment, hydrogène, alcoyle avec 1 à 12 atomes de carbone, $-(CH_2)_n-Ar$, $-(CH_2)_p-NR^5R^6$ ou $-(CH_2)_p-N^+R^5R^6R^7Y^-$;
$R^5$ et $R^6$ sont chacun indépendamment, hydrogène, alcoyle avec 1 à 12 atomes de carbone ou $-(CH_2)_n-Ar$;
Ar est un radical facultativement mono- ou disubstitué, choisi parmi :

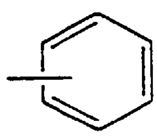 , 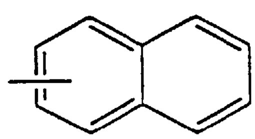 , 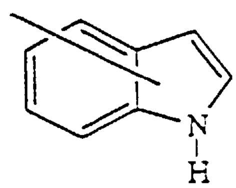 ,

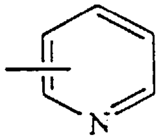 , 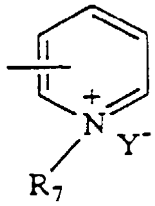 , 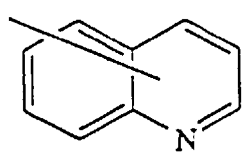 ou 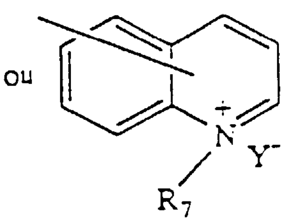

où les substituants facultatifs sont choisis parmi le groupe consistant en alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, ou perfluoroalcoyle avec 1 à 6 atomes de carbone;
$R^7$ est alcoyle avec 1 à 6 atomes de carbone;
Y est un anion halogénure, sulfate, phosphate ou p-toluènesulfonate;

m = 1-6;
n = 1-6;
p = 1-6;
ou un sel pharmaceutiquement acceptable de ceux-ci.

**6.** Composé de formule :

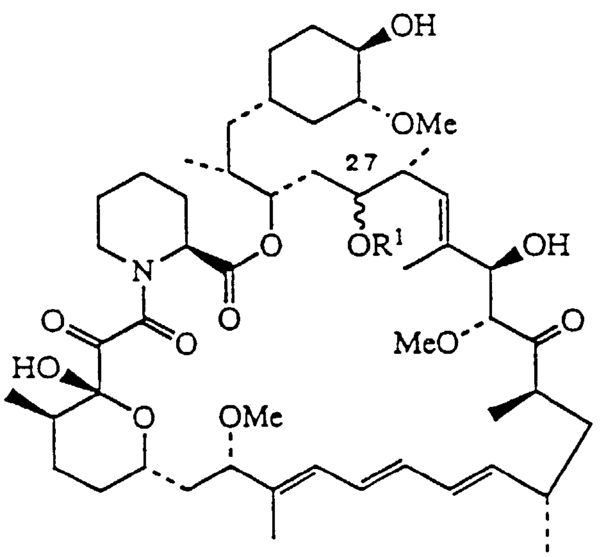

(V)

$R^1$ est

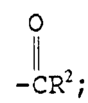

$R^2$ est $-NH(CR^3R^4)_n-X$;
$R^3$ et $R^4$ sont chacun indépendamment, hydrogène, alcoyle avec 1 à 6 atomes de carbone, aralcoyle avec 7 à 10 atomes de carbone, cycloalcoyle avec 3 à 8 atomes de carbone, halogène ou trifluorométhyle;
X est hydrogène, alcoyle inférieur avec 1 à 6 atomes de carbone, cycloalcoyle avec 3 à 8 atomes de carbone, trifluorométhyle, nitro, alcoxy avec 1 à 6 atomes de carbone, carboalcoxy avec 2 à 7 atomes de carbone, arylalcoyle avec 7 à 10 atomes de carbone, halo, dialcoylamino avec 1 à 6 atomes de carbone par radical alcoyle, thioalcoyle avec 1 à 6 atomes de carbone, ou Y;
Y est un radical phényle qui peut être facultativement mono-, di- ou trisubstitué par un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, dialcoylamino avec 1 à 6 atomes de carbone par radical alcoyle, ou alcoylthio avec 1 à 6 atomes de carbone, $-SO_3H$, $-PO_3H$ et $-CO_2H$;
n = 0-5;
avec la condition que lorsque n=0, X est alcoyle inférieur avec 1 à 6 atomes de carbone, cycloalcoyle avec 3 à 8 atomes de carbone, arylalcoyle avec 7 à 10 atomes de carbone, ou Y;
ou un sel pharmaceutiquement acceptable de ceux-ci.

**7.** Composé de formule :

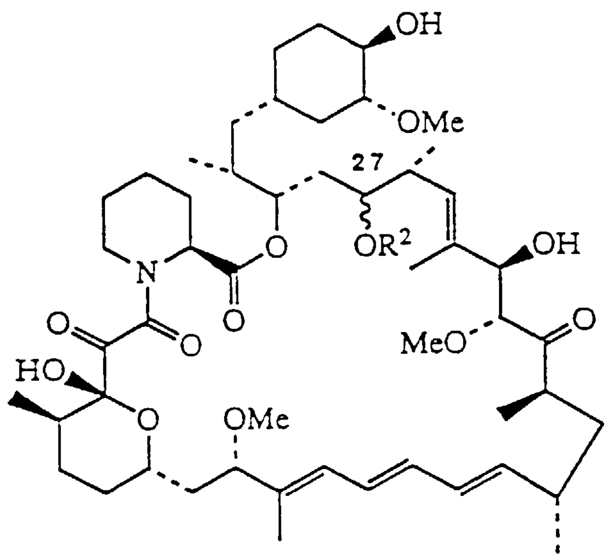

(VI)

où

$R^2$ est

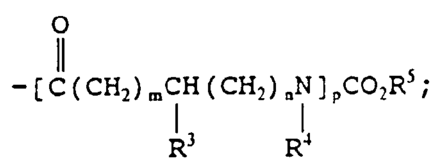

$R^3$ est hydrogène, alcoyle avec 1 à 6 atomes de carbone, arylalcoyle avec 7 à 10 atomes de carbone, -$(CH_2)_qCO_2R^6$, -$(CH_2)_rNR^7CO_2R^8$, carbamoylalcoyle avec 2 à 3 atomes de carbone, aminoalcoyle avec 1 à 4 atomes de carbone, hydroxyalcoyle avec 1 à 4 atomes de carbone, guanylalcoyle avec 2 à 4 atomes de carbone, mercaptoalcoyle avec 1 à 4 atomes de carbone, alcoylthioalcoyle avec 2 à 6 atomes de carbone, indolylméthyle, hydroxyphénylméthyle, imidazoylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

$R^4$ et $R^7$ sont chacun indépendamment, hydrogène, alcoyle avec 1 à 6 atomes de carbone ou arylalcoyle avec 7 à 10 atomes de carbone;

$R^5$, $R^6$ et $R^8$ sont chacun indépendamment, alcoyle avec 1 à 6 atomes de carbone, arylalcoyle avec 7 à 10 atomes de carbone, fluorénylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

m est 0-4;

n est 0-4;

p est 1-2;

q est 0-4;

r est 0-4;

où

$R^3$, $R^4$, m et n sont indépendants dans chacune des sous-unités :

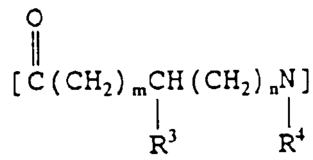

lorsque p = 2;
ou un sel pharmaceutiquement acceptable de ceux-ci.

8. Composé de formule :

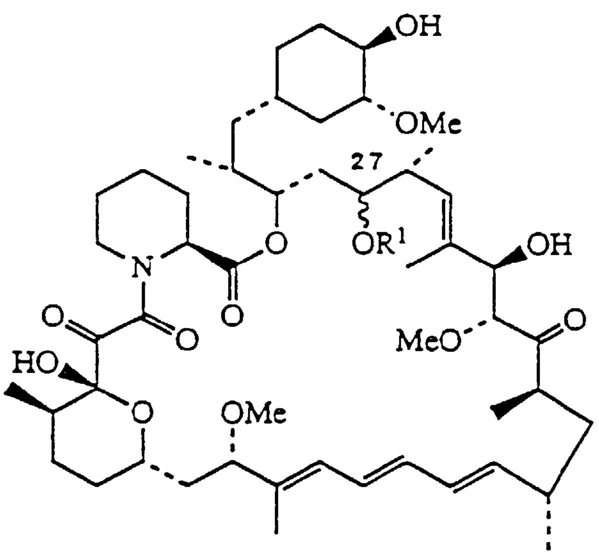

(VII)

où

$R^1$ est alcoyle avec 1 à 6 atomes de carbone, arylalcoyle avec 7 à 10 atomes de carbone, $-CH_2YX$, $-C(CH_3)_2YX$, $-CH(CH_3)YX$ ou L;
Y est O ou S;
X est $-CH_3$, $-(CH_2)_nCH_3$, $-CH_2Ar$, $-(CH_2)_2OCH_3$, $-CH_2CCl_3$, $-CH(CH_3)_2$ ou $-CH_2CH_2SiMe_3$;
Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué avec un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;
L est tétrahydrofurann-2-yle, tétrahydrothiophèn-2-yle, tétrahydrothiopyrann-2-yle, tétrahydropyrann-2-yle, 4-méthoxytétrahydropyrann-2-yle, 4-méthoxytétrahydrothiopyrann-2-yle ou 4-méthoxytétrahydrothiopyrann-2-yl-S,S-dioxyde, et
n = 1-5.

9. Composé de formule :

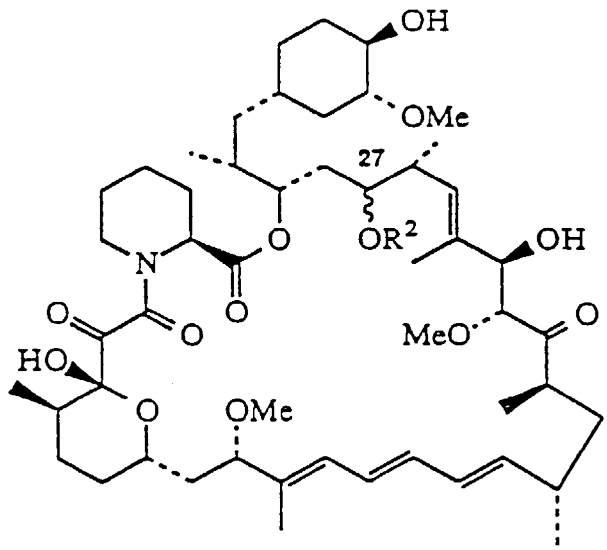

(VIII)

où

R2 est

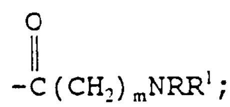

R et $R^1$ sont chacun hydrogène ou alcoyle avec 1 à 3 atomes de carbone ou R et $R^1$ forment ensemble avec l'azote auquel ils sont attachés, un hétérocycle saturé ayant 4 à 5 atomes de carbone, et
m = 1-3;
ou un sel pharmaceutiquement acceptable de ceux-ci.

**10.** Composé de formule :

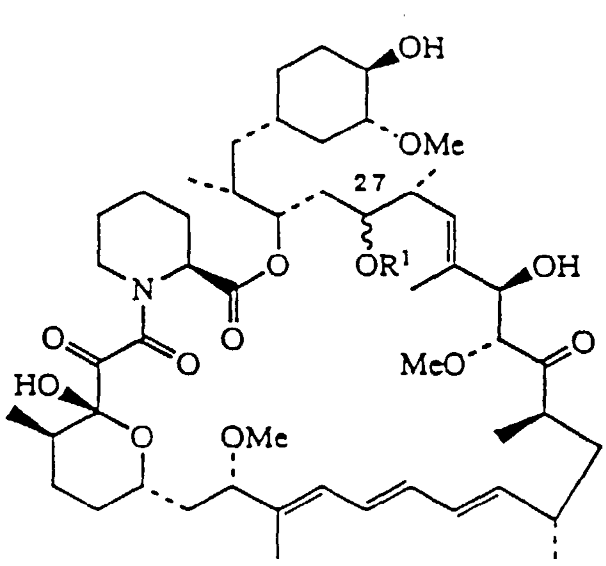

(IX)

où

$R^1$ est $-CONHSO_2$-Ar, et

Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué au moyen d'un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3HF$ $-PO_3H$ et $-CO_2H$; ou un sel pharmaceutiquement acceptable de ceux-ci, lorsque le radical Ar contient un azote basique ou lorsque le radical Ar est substitué par un dialcoylamino avec 1 à 6 atomes de carbone par radical alcoyle, $-SO_3H$,$-PO_3H$ ou $-CO_2H$.

**11.** Composé de formule :

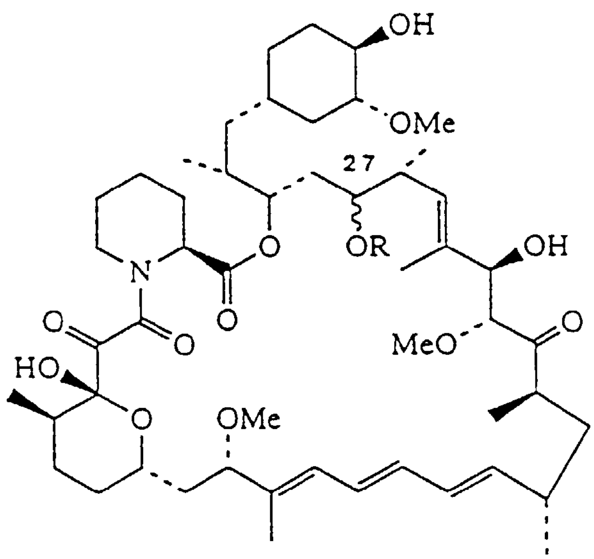

(X)

où

R est $-SO_2R^1$;

$R^1$ est alcoyle, alcényle, alcynyle contenant 1 à 6 atomes de carbone; ou une fraction aromatique choisie parmi le groupe consistant en phényle et naphtyle ou une fraction hétérocyclique choisie parmi le groupe consistant en thiophényle et quinoléinyle, ou $-NHCOR^2$, et

$R^2$ est alcoyle inférieur contenant 1 à 6 atomes de carbone;

ou un sel pharmaceutiquement acceptable de ceux-ci.

**12.** Utilisation de la 27-hydroxyrapamycine dans la préparation d'un médicament à utiliser pour induire l'immunosuppression.

**13.** Utilisation suivant la revendication 12, où l'immunosuppression induite est utilisée pour induire ou traiter le rejet de transplantation ou la réaction du greffon contre l'hôte.

**14.** Utilisation suivant la revendication 12, où l'immunosuppression induite est utilisée pour traiter des maladies autoimmunitaires, des maladies inflammatoires ou des troubles vasculaires hyperprolifératifs.

**15.** Utilisation d'un composé de formule (II) :

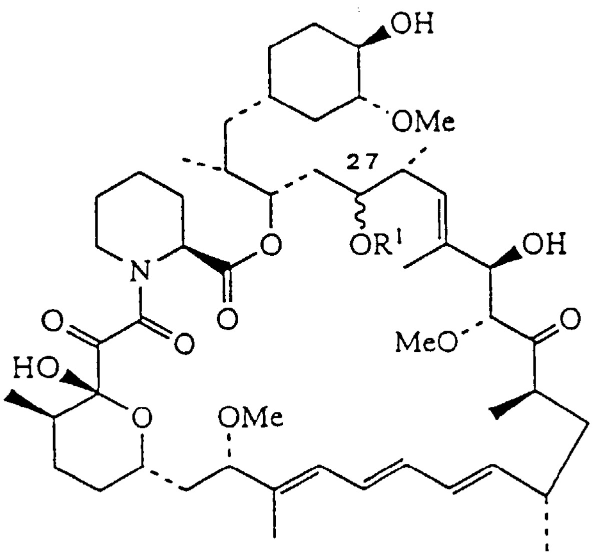

(II)

où

$R^1$ est

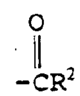

et

$R^2$ est alcoyle avec 1 à 10 atomes de carbone, arylalcoyle avec 7 à 10 atomes de carbone ou aryle dans lequel le radical aryle peut être facultativement mono-, di-ou trisubstitué au moyen d'un groupe choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3HF$ $-PO_3H$ et $-CO_2H$;

ou un sel pharmaceutiquement acceptable de ceux-ci;

ou ayant la formule (III) :

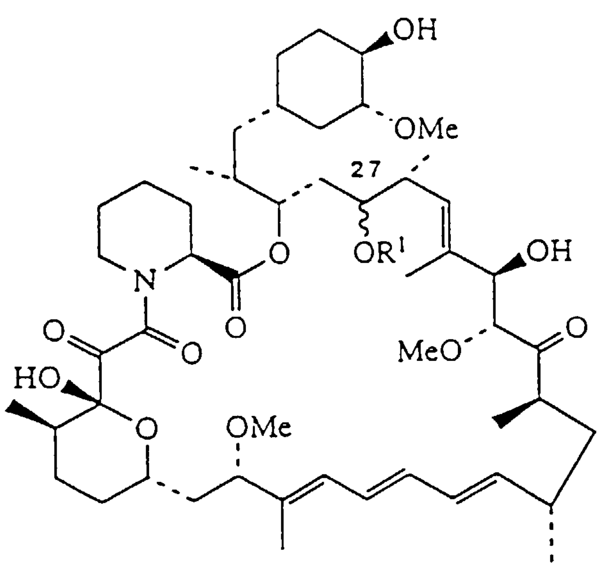

(III)

où
$R^1$ est

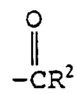

et
$R^2$ est un radical alcoyle mono-, di-, poly- ou perfluoré avec 1 à 10 atomes de carbone;
ou ayant la formule (IV) :

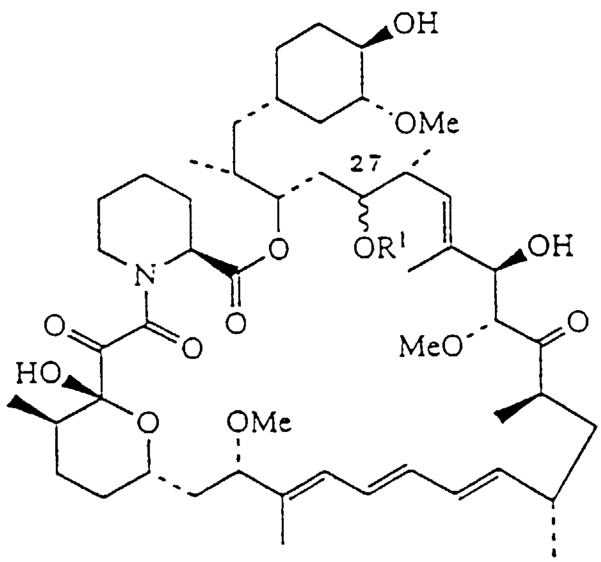

(IV)

où
$R^1$ est

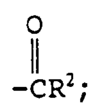

$R^2$ est

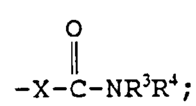

X est $-(CH_2)_m-$ ou $-Ar^1-$; où $-Ar^1-$ est un radical facultativement mono- ou disubstitué choisi parmi :

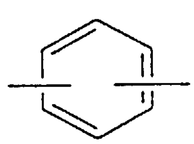 , 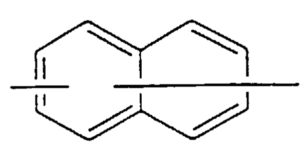 , 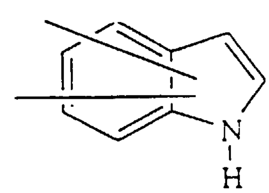 ,

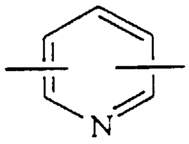 , 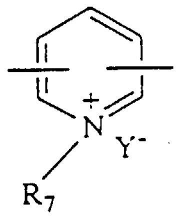 , 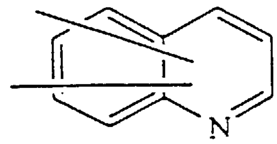 ou 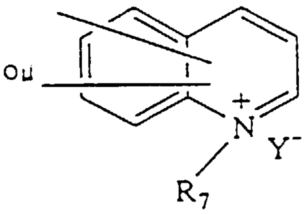

$R^3$ et $R^4$ sont chacun indépendamment, hydrogène, alcoyle avec 1 à 12 atomes de carbone, $-(CH_2)_n$-Ar, $-(CH_2)_p-NR^5R^6$ ou - $(CH_2)_p-N^+R^5R^6R^7Y^-$;
$R^5$ et $R^6$ sont chacun indépendamment, hydrogène, alcoyle avec 1 à 12 atomes de carbone ou $-(CH_2)_n$-Ar;
Ar est un radical facultativement mono- ou disubstitué, choisi parmi :

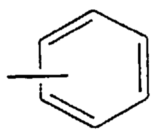 . 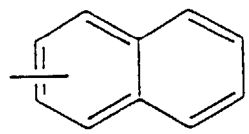 . 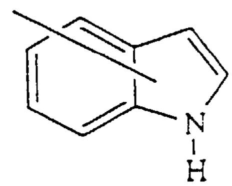 ,

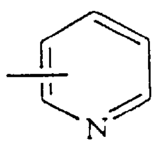 , 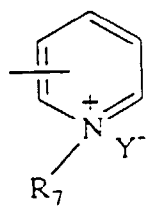 , 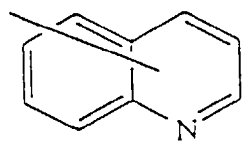 ou 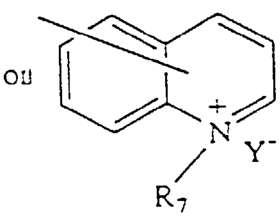

où les substituants facultatifs sont choisis parmi le groupe consistant en alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, ou perfluoroalcoyle avec 1 à 6 atomes de carbone;
$R^7$ est alcoyle avec 1 à 6 atomes de carbone;
Y est un anion halogénure, sulfate, phosphate ou p-toluènesulfonate;
m = 1-6;
n = 1-6;
p = 1-6;
ou un sel pharmaceutiquement acceptable de ceux-ci;
ou avant la formule (V) :

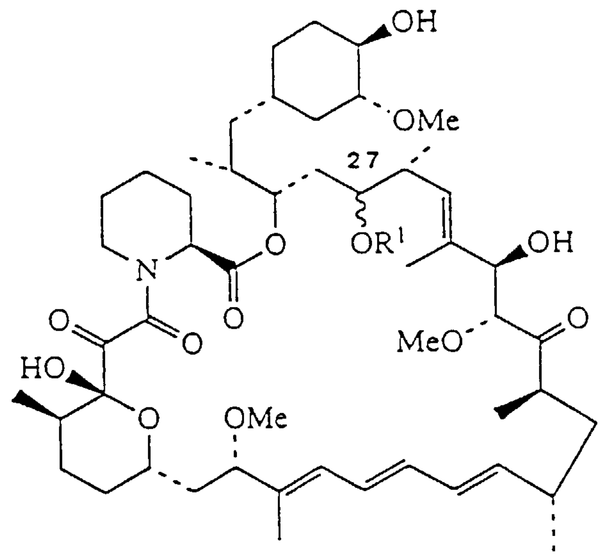

(V)

$R^1$ est

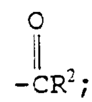

$R^2$ est $-NH(CR^3R^4)_n-X$;

$R^3$ et $R^4$ sont chacun indépendamment, hydrogène, alcoyle avec 1 à 6 atomes de carbone, aralcoyle avec 7 à 10 atomes de carbone, cycloalcoyle avec 3 à 8 atomes de carbone, halogène ou trifluorométhyle;

X est hydrogène, alcoyle inférieur avec 1 à 6 atomes de carbone, cycloalcoyle avec 3 à 8 atomes de carbone, trifluorométhyle, nitro, alcoxy avec 1 à 6 atomes de carbone, carboalcoxy avec 2 à 7 atomes de carbone, arylalcoyle avec 7 à 10 atomes de carbone, halo, dialcoylamino avec 1 à 6 atomes de carbone par radical alcoyle, thioalcoyle avec 1 à 6 atomes de carbone, ou Y;

Y est un radical phényle qui peut être facultativement mono-, di- ou trisubstitué par un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, dialcoylamino avec 1 à 6 atomes de carbone par radical alcoyle, ou alcoylthio avec 1 à 6 atomes de carbone, $-SO_3HF$ $-PO_3H$ et $-CO_2H$;

n = 0-5;

avec la condition que lorsque n=o, X est alcoyle inférieur avec 1 à 6 atomes de carbone, cycloalcoyle avec 3 à 8 atones de carbone, arylalcoyle avec 7 à 10 atomes de carbone, ou Y;

ou un sel pharmaceutiquement acceptable de ceux-ci;

ou ayant la formule (VI) :

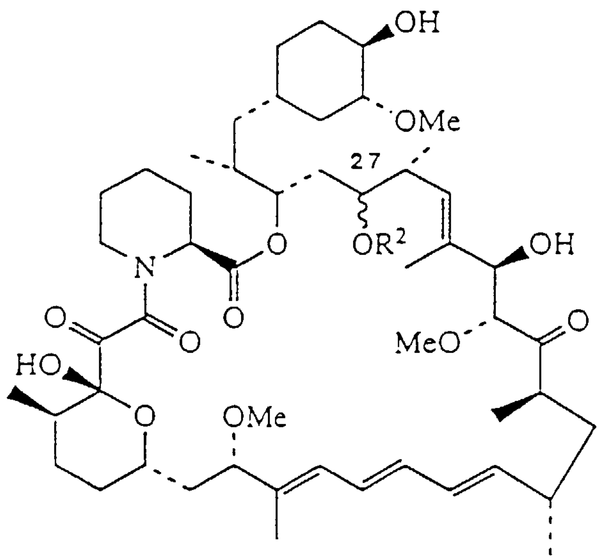

(VI)

où

$R^2$ est

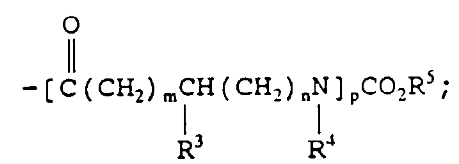

$R^3$ est hydrogène, alcoyle avec 1 à 6 atomes de carbone, arylalcoyle avec 7 à 10 atomes de carbone, -$(CH_2)_qCO_2R^6$, -$(CH_2)_rNR^7CO_2R^8$, carbamoylalcoyle avec 2 à 3 atomes de carbone, aminoalcoyle avec 1 à 4 atomes de carbone, hydroxyalcoyle avec 1 à 4 atomes de carbone, guanylalcoyle avec 2 à 4 atomes de carbone, mercaptoalcoyle avec 1 à 4 atomes de carbone, alcoylthioalcoyle avec 2 à 6 atomes de carbone, indolylméthyle, hydroxyphénylméthyle, imidazoylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

$R^4$ et $R^7$ sont chacun indépendamment, hydrogène, alcoyle avec 1 à 6 atomes de carbone ou arylalcoyle avec 7 à 10 atomes de carbone;

$R^5$, $R^6$ et $R^8$ sont chacun indépendamment, alcoyle avec 1 à 6 atomes de carbone, arylalcoyle avec 7 à 10 atomes de carbone, fluorénylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

m est 0-4;

n est 0-4;

p est 1-2;

q est 0-4;

r est 0-4;

où

$R^3$, $R^4$, m et n sont indépendants dans chacune des sous-unités :

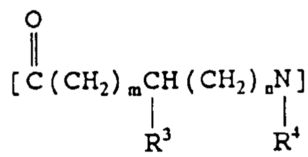

lorsque p = 2;
ou un sel pharmaceutiquement acceptable de ceux-ci;
ou ayant la formule (VII) :

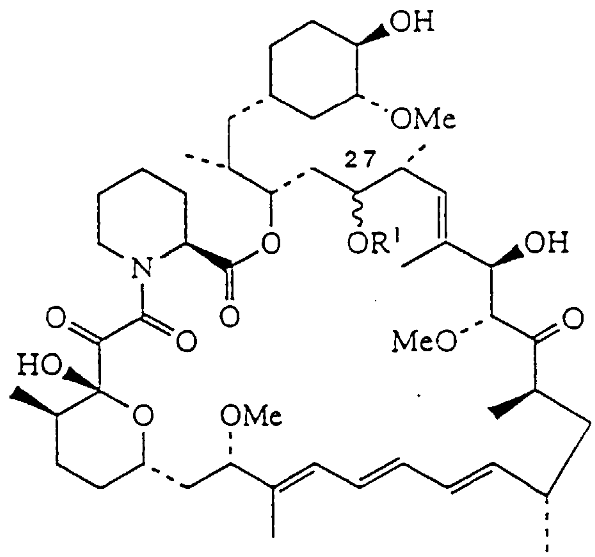

(VII)

où

$R^1$ est alcoyle avec 1 à 6 atomes de carbone, arylalcoyle avec 7 à 10 atomes de carbone, $-CH_2YX$, $-C(CH_3)_2YX$, $-CH(CH_3)YX$ ou L;

Y est O ou S;

X est $-CH_3$, $-(CH_2)_nCH_3$, $-CH_2Ar$, $-(CH_2)_2OCH_3$, $-CH_2CCl_3$, $-CH(CH_3)_2$ ou $-CH_2CH_2SiMe_3$;

Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué avec un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;

L est tétrahydrofurann-2-yle, tétrahydrothiophèn-2-yle, tétrahydrothiopyrann-2-yle, tétrahydropyrann-2-yle, 4-méthoxytétrahydropyrann-2-yle, 4-méthoxytétrahydrothiophyrann-2-yle ou 4-méthoxytétrahydrothiopyrann-2-yl-S,S-dioxyde, et

n = 1-5;

ou ayant la formule (VIII) :

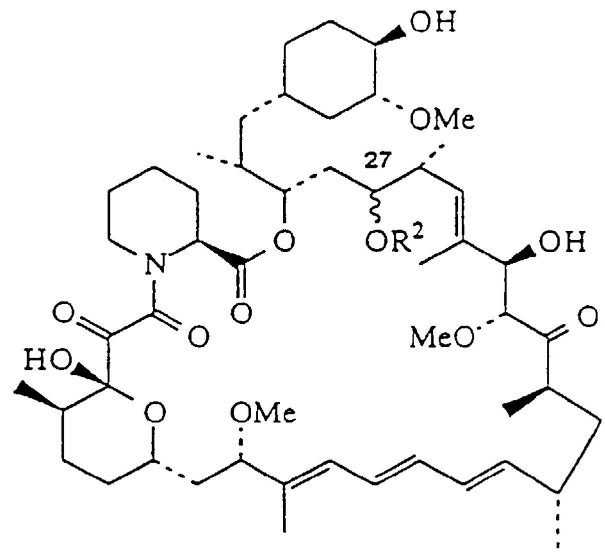

(VIII)

où
$R^2$ est

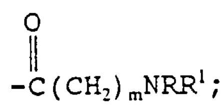

R et $R^1$ sont chacun hydrogène ou alcoyle avec 1 à 3 atomes de carbone ou R et $R^1$ forment ensemble avec l'azote auquel ils sont attachés, un hétérocycle saturé ayant 4 à 5 atomes de carbone, et
m = 1-3;
ou un sel pharmaceutiquement acceptable de ceux-ci;
ou ayant la formule (IX) :

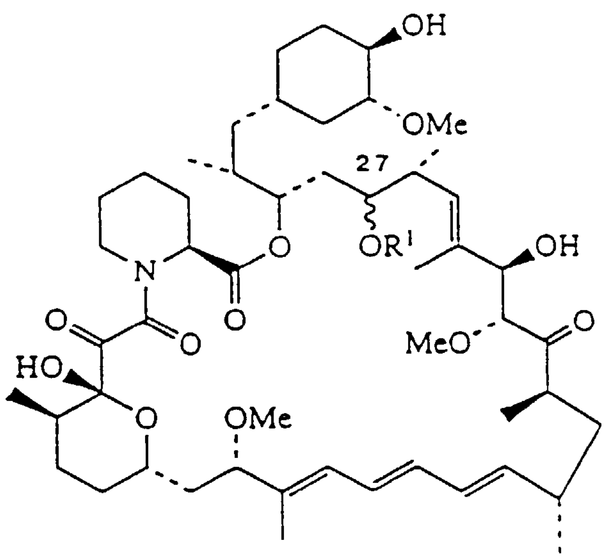

(IX)

où
$R^1$ est $-CONHSO_2$-Ar, et
Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué au moyen d'un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;
ou un sel pharmaceutiquement acceptable de ceux-ci, lorsque le radical Ar contient un azote basique ou lorsque le radical Ar est substitué par un dialcoylamino avec 1 à 6 atomes de carbone par radical alcoyle, $-SO_3H$, $-PO_3H$ ou $-CO_2H$;
ou ayant la formule (X) :

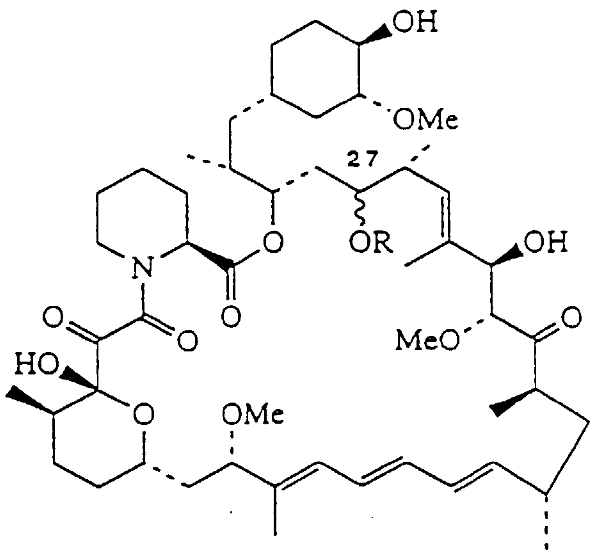

(X)

où
R est $-SO_2R^1$;
$R^1$ est alcoyle, alcényle, alcynyle contenant 1 à 6 atomes de carbone; ou une fraction aromatique choisie parmi le groupe consistant en phényle et naphtyle ou une fraction hétérocyclique choisie parmi le groupe consistant en thiophényle et quinoléinyle, ou $-NHCOR^2$, et
$R^2$ est alcoyle inférieur contenant 1 à 6 atomes de carbone;
ou un sel pharmaceutiquement acceptable de ceux-ci, dans la préparation d'un médicament pour induire l'immunosuppression.

**16.** Composition pharmaceutique qui comprend une quantité efficace de 27-hydroxyrapamycine et un excipient pharmaceutiquement acceptable.

**17.** Composition pharmaceutique qui comprend une quantité efficace d'une association de 27-hydroxyrapamycine; d'un agent chimiothérapeutique antirejet choisi parmi le groupe consistant en l'azathioprine, les corticostéroïdes, le cyclophosphamide, la rapamycine, la cyclosporine A, le FK-506, le OKT-3 et l'ATG; et un excipient pharmaceutiquement acceptable.

**18.** Composition pharmaceutique qui comprend une quantité efficace d'un composé de formule (II) :

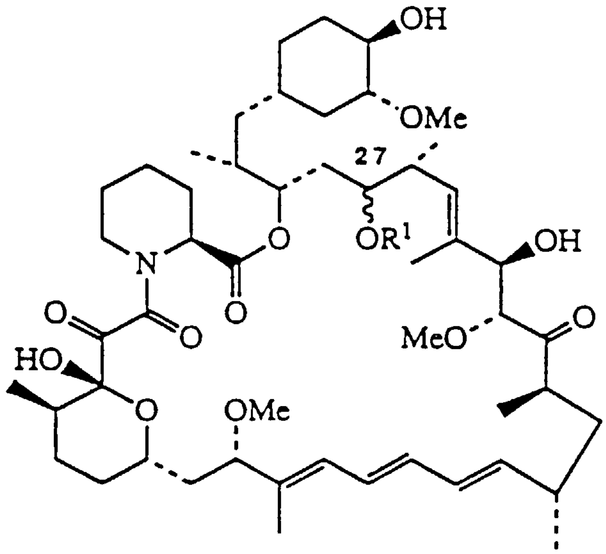

(II)

où

$R^1$ est

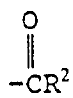

et

$R^2$ est alcoyle avec 1 à 10 atomes de carbone, arylalcoyle avec 7 à 10 atomes de carbone ou aryle dans lequel le radical aryle peut être facultativement mono-, di-ou trisubstitué au moyen d'un groupe choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;
ou un sel pharmaceutiquement acceptable de ceux-ci;
ou ayant la formule (III) :

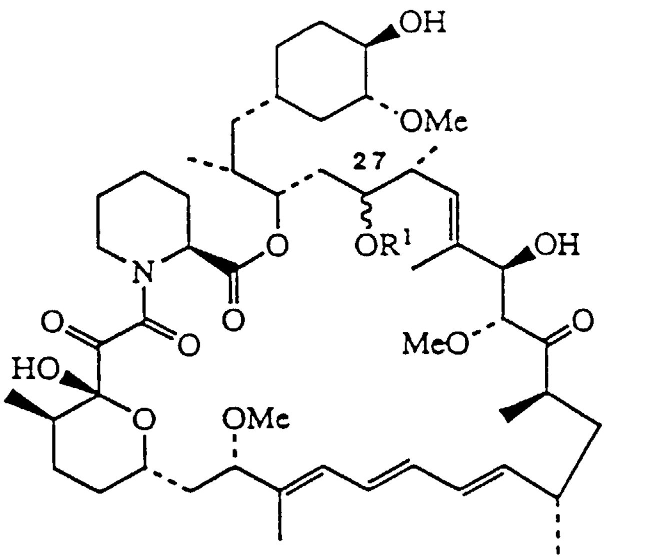

(III)

où
$R^1$ est

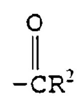

et
$R^2$ est un radical alcoyle mono-, di-, poly- ou perfluoré avec 1 à 10 atomes de carbone;
ou ayant la formule (IV) :

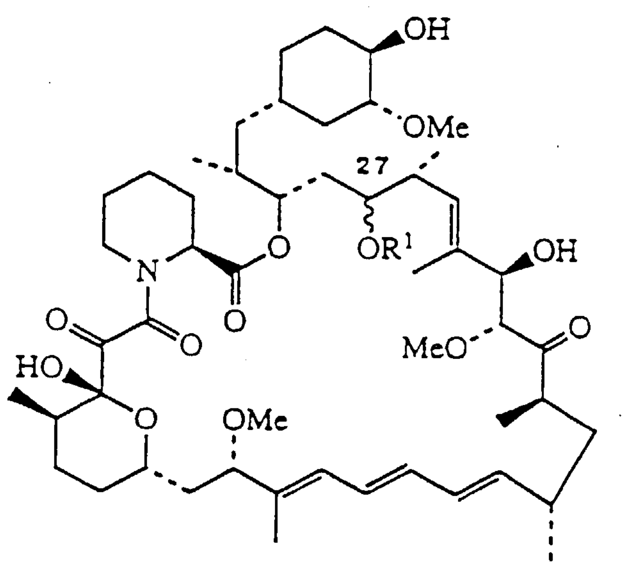

(IV)

où
$R^1$ est

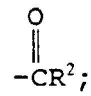

$R^2$ est

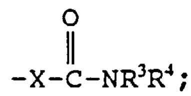

X est $-(CH_2)_m-$ ou $-Ar^1-$; où $-Ar^1-$ est un radical facultativement mono- ou disubstitué choisi parmi :

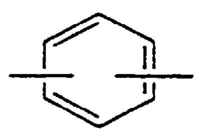 , 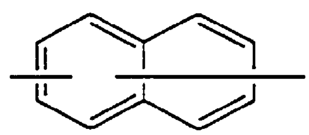 , 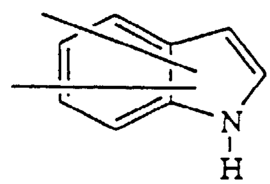 ,

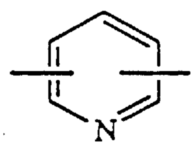 , 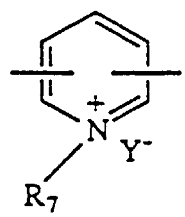 , 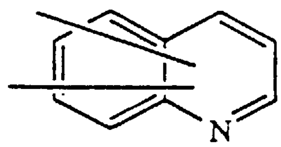 or 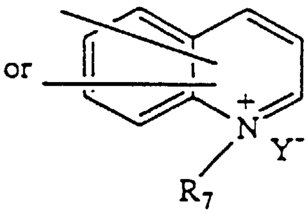

$R^3$ et $R^4$ sont chacun indépendamment, hydrogène, alcoyle avec 1 à 12 atomes de carbone, $-(CH_2)_n-Ar$, $-(CH_2)_p-NR^5R^6$ ou $-(CH_2)_p-N^+R^5R^6R^7Y^-$;
$R^5$ et $R^6$ sont chacun indépendamment, hydrogène, alcoyle avec 1 à 12 atomes de carbone ou $-(CH_2)_n-Ar$;
Ar est un radical facultativement mono- ou disubstitué, choisi parmi :

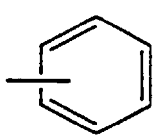 , 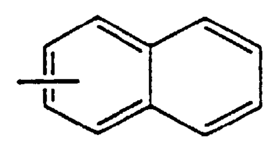 , 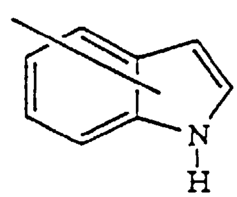 ,

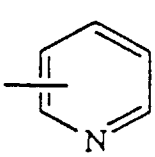 , 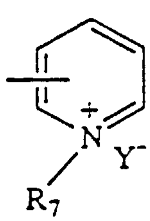 , 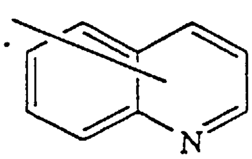 or 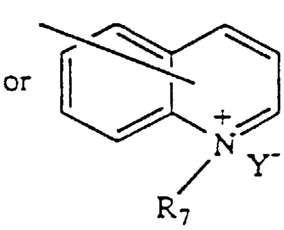

où les substituants facultatifs sont choisis parmi le groupe consistant en alcoyle avec 1 à 6 atomes de carbone,

arylalcoyle avec 7 à 10 atomes de carbone, alcoxy avec 1 à 6 atomes de carbone, cyano, halo, nitro, carbalcoxy avec 2 à 7 atomes de carbone, ou perfluoroalcoyle avec 1 à 6 atomes de carbone;
$R^7$ est alcoyle avec 1 à 6 atomes de carbone;
Y est un anion halogénure, sulfate, phosphate ou p-toluènesulfonate;
m = 1-6;
n = 1-6;
p = 1-6;
ou un sel pharmaceutiquement acceptable de ceux-ci;

ou ayant la formule (V) :

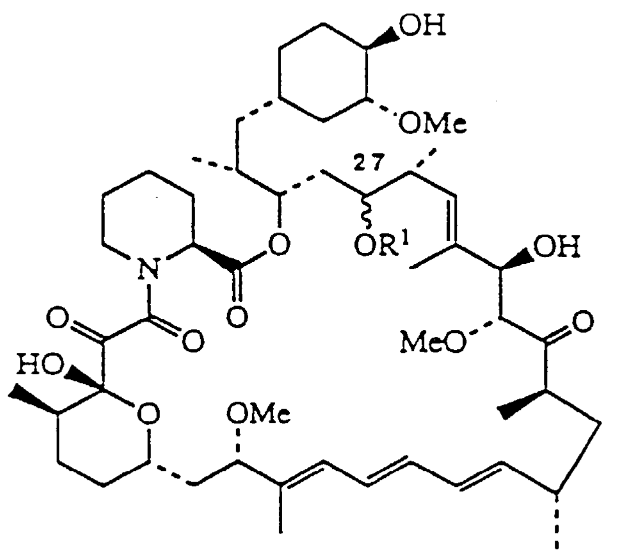

(V)

où

$R^1$ est

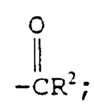

$R^2$ est $-NH(CR^3R^4)_n-X$;
$R^3$ et $R^4$ sont chacun indépendamment, hydrogène, alcoyle avec 1 à 6 atomes de carbone, aralcoyle avec 7 à 10 atomes de carbone, cycloalcoyle avec 3 à 8 atomes de carbone, halogène ou trifluorométhyle;
X est hydrogène, alcoyle inférieur avec 1 à 6 atomes de carbone, cycloalcoyle avec 3 à 8 atomes de carbone, trifluorométhyle, nitro, alcoxy avec 1 à 6 atomes de carbone, carboalcoxy avec 2 à 7 atomes de carbone, arylalcoyle avec 7 à 10 atomes de carbone, halo, dialcoylamino avec 1 à 6 atomes de carbone par radical alcoyle, thioalcoyle avec 1 à 6 atomes de carbone, ou Y;
Y est un radical phényle qui peut être facultativement mono-, di- ou trisubstitué par un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, dialcoylamino avec 1 à 6 atomes de carbone par radical alcoyle, ou alcoylthio avec 1 à 6 atomes de carbone, $-SO_3H$, $-PO_3H$ et $-CO_2H$;
n = 0-5;
avec la condition que lorsque n=0, X est alcoyle inférieur avec 1 à 6 atomes de carbone, cycloalcoyle avec 3 à 8 atomes de carbone, arylalcoyle avec 7 à 10 atomes de carbone, ou Y;
ou un sel pharmaceutiquement acceptable de ceux-ci;
ou ayant la formule (VI) :

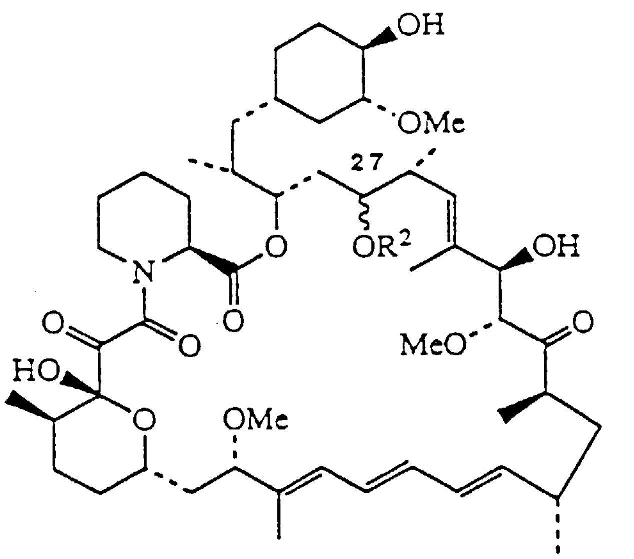

(VI)

où

$R^2$ est

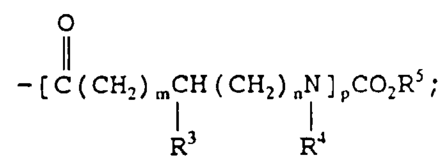

$R^3$ est hydrogène, alcoyle avec 1 à 6 atomes de carbone, arylalcoyle avec 7 à 10 atomes de carbone, -$(CH_2)_qCO_2R^6$, -$(CH_2)_rNR^7CO_2R^8$, carbamoylalcoyle avec 2 à 3 atomes de carbone, aminoalcoyle avec 1 à 4 atomes de carbone, hydroxyalcoyle avec 1 à 4 atomes de carbone, guanylalcoyle avec 2 à 4 atomes de carbone, mercaptoalcoyle avec 1 à 4 atomes de carbone, alcoylthioalcoyle avec 2 à 6 atomes de carbone, indolylméthyle, hydroxyphénylméthyle, imidazoylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

$R^4$ et $R^7$ sont chacun indépendamment, hydrogène, alcoyle avec 1 à 6 atomes de carbone ou arylalcoyle avec 7 à 10 atomes de carbone;

$R^5$, $R^6$ et $R^8$ sont chacun indépendamment, alcoyle avec 1 à 6 atomes de carbone, arylalcoyle avec 7 à 10 atomes de carbone, fluorénylméthyle ou phényle qui est facultativement mono-, di- ou trisubstitué avec un substituant choisi parmi alcoyle avec 1 à 6 atomes de carbone, alcoxy avec 1 à 6 atomes de carbone, hydroxyle, cyano, halo, nitro, carbalcoxy avec 2 à 7 atomes de carbone, trifluorométhyle, amino ou -$CO_2H$;

m est 0-4;

n est 0-4;

p est 1-2;

q est 0-4;

r est 0-4;

où

$R^3$, $R^4$, m et n sont indépendants dans chacune des sous-unités :

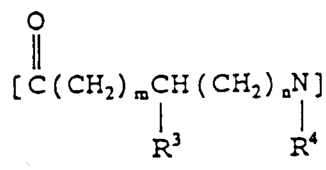

lorsque p = 2;
ou un sel pharmaceutiquement acceptable de ceux-ci;
ou ayant la formule (VII) :

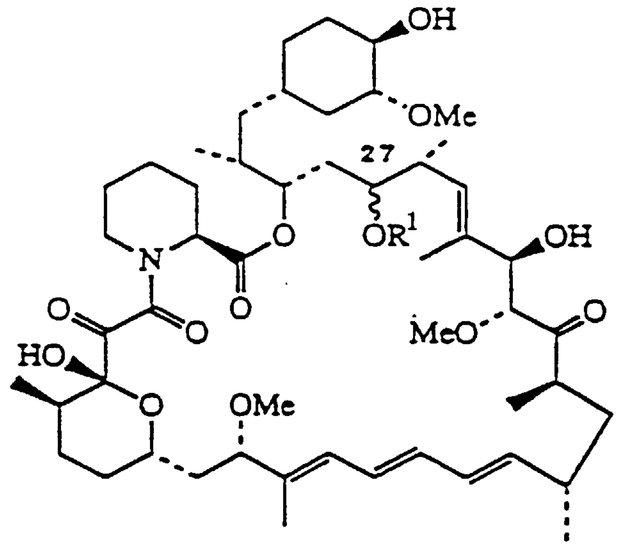

(VII)

où

$R^1$ est alcoyle en 1 à 6 atomes de carbone, arylalcoyle avec 7 à 10 atomes de carbone, $-CH_2YX$, $-C(CH_3)_2YX$, $-CH(CH_3)YX$ ou L;
Y est O ou S;
X est $-CH_3$, $-(CH_2)_nCH_3$, $-CH_2Ar$, $-(CH_2)_2OCH_3$, $-CH_2CCl_3$, $-CH(CH_3)_2$ ou $-CH_2CH_2SiMe_3$;
Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué avec un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;
L est tétrahydrofurann-2-yle, tétrahydrothiophèn-2-yle, tétrahydrothiopyrann-2-yle, tétrahydropyrann-2-yle, 4-méthoxytétrahydropyrann-2-yle, 4-méthoxytétrahydrothiophyrann-2-yle ou 4-méthoxytétrahydrothiopyrann-2-yl-S,S-dioxyde, et n =1-5;
ou ayant la formule (VIII) :

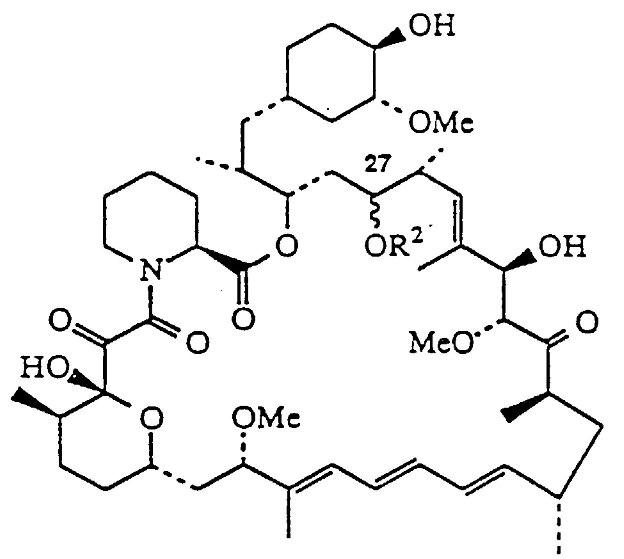

(VIII)

où
$R^2$ est

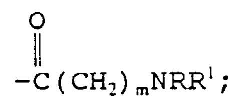

R et $R^1$ sont chacun hydrogène ou alcoyle avec 1 à 3 atomes de carbone ou R et $R^1$ forment ensemble avec l'azote auquel ils sont attachés, un hétérocycle saturé ayant 4 à 5 atomes de carbone, et
m = 1-3;
ou un sel pharmaceutiquement acceptable de ceux-ci;

ou ayant la formule (IX) :

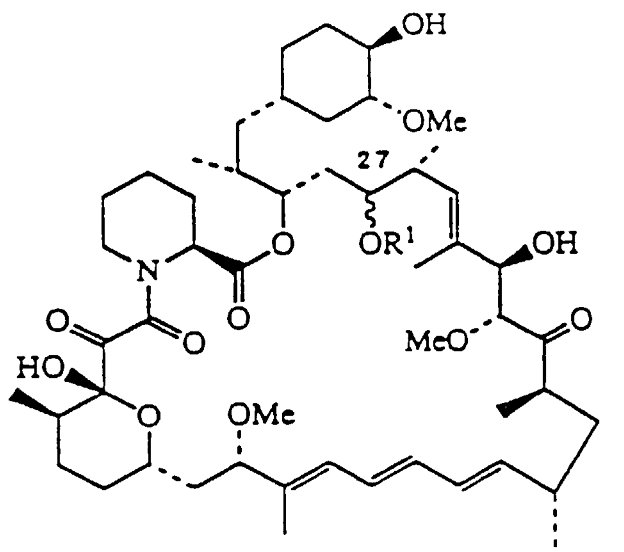

(IX)

où

$R^1$ est $-CONHSO_2$-Ar, et

Ar est phényle, naphtyle, pyridyle, quinoléyle, isoquinoléyle, quinoxalyle, thiényle, thionaphtyle, furyle, benzofuryle, benzodioxyle, benzoxazolyle, benzoisoxazolyle ou benzodioxolyle; où le radical Ar peut être facultativement mono-, di- ou trisubstitué au moyen d'un radical choisi parmi alcoyle avec 1 à 6 atomes de carbone, arylalcoyle avec 7 à 10 atomes de carbone, alcoxy avec 1 à 6 atomes de carbone, cyano, halo, nitro, carbalcoxy avec 2 à 7 atomes de carbone, trifluorométhyle, amino, dialcoylamino avec 1 à 6 atomes de carbone par radical alcoyle, alcoylthio avec 1 à 6 atomes de carbone, $-SO_3H$, $-PO_3H$ et $-CO_2H$;
ou un sel pharmaceutiquement acceptable de ceux-ci, lorsque le radical Ar contient un azote basique ou lorsque le radical Ar est substitué par un dialcoylamino avec 1 à 6 atomes de carbone par radical alcoyle, $-SO_3H$, $-PO_3H$ ou $-CO_2H$;
ou ayant la formule (X) :

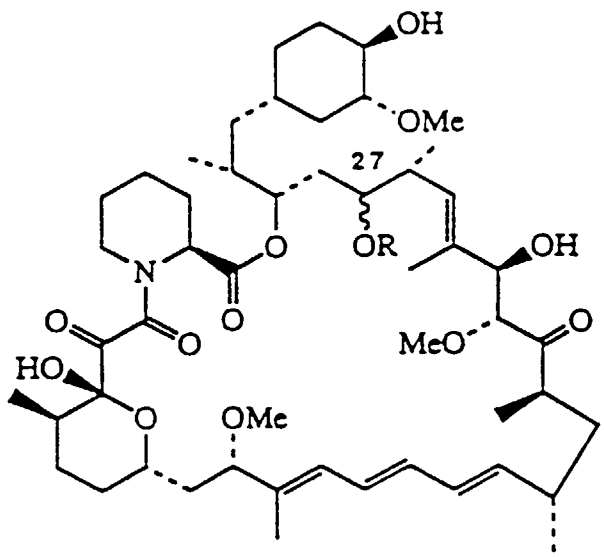

(X)

où
R est $-SO_2R^1$;
$R^1$ est alcoyle, alcényle, alcynyle contenant 1 à 6 atomes de carbone; ou une fraction aromatique choisie parmi le groupe consistant en phényle et naphtyle ou une fraction hétérocyclique choisie parmi le groupe consistant en thiophényle et quinoléinyle, ou $-NHCOR^2$, et
$R^2$ est alcoyle inférieur contenant 1 à 6 atomes de carbone;
ou un sel pharmaceutiquement acceptable de ceux-ci, et
d'un excipient pharmaceutiquement acceptable.

**19.** Composé qui est le 31,42-bistriéthylsilyl-éther de la 27-hydroxyrapamycine.

**20.** Composé qui est l'ester en 27 avec l'acide acétique du 31,42-bistriéthylsilyléther de la 27-hydroxyrapamycine.